# EUROPEAN PATENT APPLICATION

(11) **EP 4 393 949 A1**
(43) Date of publication of application: **03.07.2024**
(21) Application number: 22860595.2
(22) Date of filing: 25.08.2022
(51) Int. Cl.: C07K 16/24, C07K 14/55, A61K 39/00

(54) **PHARMACEUTICAL COMPOSITION CONTAINING FUSION PROTEIN**

(30) Priority: 25.08.2021 CN 202110985497
(71) Applicant: Jiangsu Hengrui Pharmaceuticals Co., Ltd., Lianyungang, Jiangsu 222047 (CN); Shanghai Shengdi Pharmaceutical Co., Ltd, Shanghai 201210 (CN)
(72) Inventor: MA, Xiazhen, Lianyungang, Jiangsu 222047 (CN); TIAN, Chenmin, Lianyungang, Jiangsu 222047 (CN); LI, Xiaofei, Lianyungang, Jiangsu 222047 (CN)
(74) Representative: Potter Clarkson
(86) International application number: PCT/CN2022/114858
(87) International publication number: WO 2023/025249

(57) **Abstract**

The present invention relates to a pharmaceutical composition containing a fusion protein. Specifically, the present invention relates to a pharmaceutical composition comprising the fusion protein containing covalently bonded IL-2 and an anti-IL-2 antibody, and the use thereof.

## Description

### TECHNICAL FIELD

The present disclosure pertains to the field of pharmaceutical formulations. In particular, the present disclosure relates to a pharmaceutical composition comprising a fusion protein comprising IL-2 and an anti-IL-2 antibody that are covalently bound, and use thereof as a medicament.

### BACKGROUND

The statements herein merely provide background information related to the present disclosure and may not necessarily constitute the prior art.

Human interleukin-2 (IL-2), also known as T cell growth factor (TCGF), has a gene located on chromosome 4 (4q27) and comprising a sequence of 7 kb in total, and consists of 133 amino acids with a molecular weight of about 15 kD. In 1976 and 1977, Doris Morgan, Francis Ruscetti, Robert Gallo and Steven Gillis, Kendal Smith, et al., separately found that activated T cell culture medium could promote T cell proliferation. Thereafter, the stimulating factor in the culture medium was purified and identified as a single protein, i.e., IL-2.

IL-2 acts through IL-2R on the cell surface. IL-2R includes three subunits: IL-2Rα (i.e., CD25), IL-2Rβ (i.e., CD122), and IL-2Rγ (i.e., CD132). Those three subunits can form three forms of receptors: the high-binding avidity receptor comprising all of the three subunits IL-2Rα/β/γ, the medium-binding avidity receptor comprising IL-2Rβ/γ, and the low-binding avidity receptor being IL-2Rα. Among them, IL-2Rβ and IL-2Rγ are necessary for IL-2 to activate downstream signaling pathways, and when IL-2 binds to both IL-2Rβ and IL-2Rγ, those two receptor subunits form a heterodimer that phosphorylates STATS in cells, and enters cell nucleus to cause corresponding gene transcription and expression; and IL-2Rα is not necessary for signaling, but can enhance the binding of IL-2 to IL-2Rβ and IL-2Rγ. Tregs continuously express the high-binding avidity receptor IL-2Rα/β/γ, while unactivated CD8⁺ T cells, NK cells and the like only express the medium-binding avidity receptor IL-2Rβ/γ. Therefore, low-dose IL-2 preferentially binds to and activates Tregs, while high-dose IL-2 can activate immune effector cells such as CD8⁺ T cells and NK cells.

Since Tregs have immunosuppressive effects and low-dose IL-2 has the property of preferentially activating Tregs, low-dose IL-2 has been reported to be useful for the treatment of a series of immune-related diseases such as autoimmune diseases and inflammatory diseases, for example, systemic lupus erythematosus (SLE), type I diabetes (T1D), chronic graft versus host disease (cGvHD), hematopoietic stem cell transplantation (HSCT), alopecia areata, hepatitis C virus-induced vasculitis, chronic GvHD, asthma, dermatitis, acute lung injury, chronic beryllium disease, and sepsis. In many patients receiving the low-dose IL-2 treatment, there is an increase in the ratio of Tregs to effector T cells and alleviation in symptoms. However, in one aspect, IL-2 has a short half-life and requires frequent administration; in another aspect, IL-2 has a narrow administration window, and may activate immune effector cells and exacerbate autoimmune diseases if at a slightly higher dose. Those intrinsic properties or shortcomings of IL-2 make it difficult to maintain a sustained low dose of IL-2 in patients, thereby limiting the further use of IL-2.

### SUMMARY

The present disclosure provides a pharmaceutical composition comprising a fusion protein. The composition has therapeutic activity. In addition, the composition also has the advantages of good stability, being suitable for lyophilization, etc.

In some embodiments, the present disclosure provides a pharmaceutical composition, comprising a fusion protein and a buffer, wherein the fusion protein comprises IL-2 and an anti-IL-2 antibody that are covalently bound; the buffer is a histidine buffer, an acetate buffer, or a succinate buffer. In some embodiments, the buffer is a histidine buffer or an acetate buffer. In some embodiments, the buffer is a histidine-histidine hydrochloride buffer or an acetic acid-sodium acetate buffer. In some embodiments, the buffer is a histidine-histidine hydrochloride buffer.

In some embodiments, provided is the pharmaceutical composition according to any of the above, wherein the buffer has a pH of 5.0-6.5. In some embodiments, the buffer has a pH of 5.0-6.0. In some embodiments, the buffer has a pH of 5.0, 5.1, 5.2, 5.3, 5.4, 5.5, 5.6, 5.7, 5.8, 5.9, 6.0, 6.1, 6.2, 6.3, 6.4, or 6.5. In some embodiments, the buffer has a pH of 5.5-6.0. In some embodiments, the buffer has a pH of 6.0.

In some embodiments, there is a difference of no more than ±0.3 between the pH of the pharmaceutical composition and the pH of the buffer that the pharmaceutical composition comprises. In some embodiments, provided is the pharmaceutical composition according to any of the above, wherein the pharmaceutical composition has a pH of 5.0-6.5. In some embodiments, the pharmaceutical composition has a pH of 6.0-6.5. In some embodiments, the pharmaceutical composition has a pH of 6.0-6.3. In some embodiments, the pharmaceutical composition has a pH of 5.0, 5.1, 5.2, 5.3, 5.4, 5.5, 5.6, 5.7, 5.8, 5.9, 6.0, 6.1, 6.2, 6.3, 6.4, or 6.5.

In some embodiments, provided is the pharmaceutical composition according to any of the above, wherein the concentration of the fusion protein is 5 mg/mL to 50 mg/mL. In some embodiments, the concentration of the fusion protein is 5 mg/mL to 30 mg/mL. In some embodiments, the concentration of the fusion protein is 10 mg/mL to 30 mg/mL. In some embodiments, the concentration of the fusion protein is 5 mg/mL, 10 mg/mL, 15 mg/mL, 20 mg/mL, 25 mg/mL, or 30 mg/mL. In some embodiments, the concentration of the fusion protein is 30 mg/mL. In some embodiments, the concentration of the fusion protein is 10 mg/mL.

In some embodiments, provided is the pharmaceutical composition according to any of the above, wherein the pharmaceutical composition comprises a surfactant. In some embodiments, the surfactant is a nonionic surfactant. In some embodiments, the surfactant is selected from the group consisting of polysorbate, polysorbate 20, polysorbate 80, poloxamer, Triton, sodium dodecyl sulfonate, sodium lauryl sulfonate, sodium octyl glycoside, lauryl-sulfobetaine, myristyl-sulfobetaine, linoleyl-sulfobetaine, stearyl-sulfobetaine, lauryl-sarcosine, myristyl-sarcosine, linoleyl-sarcosine, stearyl-sarcosine, linoleyl-betaine, myristyl-betaine, cetyl-betaine, lauramido propyl-betaine, cocaramide propyl-betaine, linoleinamide propyl-betaine, myristylamide propyl-betaine, palmitamide propyl-betaine, isostearamide propyl-betaine, myristylamide propyl-dimethylamine, palmitamide propyl-dimethylamine, isostearamide propyl-dimethylamine, sodium methyl cocoyl, sodium methyl oleyl taurate, polyethylene glycol, polypropylene glycol, copolymers of ethylene and propylene glycol, and the like. In some embodiments, the surfactant is polysorbate or poloxamer. In some embodiments, the surfactant is polysorbate 80 (PS80), polysorbate 20 (PS20), or poloxamer 188 (PF68). In some embodiments, the surfactant is polysorbate 80 or poloxamer 188. In some embodiments, the surfactant is poloxamer 188. In some embodiments, the surfactant is polysorbate 80.

In some embodiments, provided is the pharmaceutical composition according to any of the above, wherein the concentration of the surfactant is 0.05 mg/mL to 2 mg/mL. In some embodiments, the concentration of the surfactant is 0.05 mg/mL to 1.0 mg/mL. In some embodiments, the concentration of the surfactant is 0.1 mg/mL to 0.8 mg/mL. In some embodiments, the concentration of the surfactant is 0.1 mg/mL to 0.6 mg/mL. In some embodiments, the concentration of the surfactant is 0.2 mg/mL to 0.6 mg/mL. In some embodiments, the concentration of the surfactant is 0.05 mg/mL, 0.1 mg/mL, 0.2 mg/mL, 0.3 mg/mL, 0.4 mg/mL, 0.5 mg/mL, 0.6 mg/mL, 0.7 mg/mL, 0.8 mg/mL, 0.9 mg/mL, 1.0 mg/mL, 1.1 mg/mL, 1.2 mg/mL, 1.3 mg/mL, 1.4 mg/mL, 1.5 mg/mL, 1.6 mg/mL, 1.7 mg/mL, 1.8 mg/mL, 1.9 mg/mL, or 2.0 mg/mL. In some embodiments, the concentration of the surfactant is 1.6 mg/mL. In some embodiments, the concentration of the surfactant is 1.5 mg/mL. In some embodiments, the concentration of the surfactant is 0.8 mg/mL. In some embodiments, the concentration of the surfactant is 0.6 mg/mL. In some embodiments, the concentration of the surfactant is 0.4 mg/mL.

In some embodiments, provided is the pharmaceutical composition according to any of the above, wherein the pharmaceutical composition comprises a sugar. In some embodiments, the sugar is selected from the group consisting of conventional compositions (CH₂O)ₙ and derivatives thereof, including monosaccharides, disaccharides, trisaccharides, polysaccharides, sugar alcohols, reducing sugars, non-reducing sugars, and the like. The sugar may be selected from the group consisting of glucose, sucrose, trehalose, lactose, fructose, maltose, dextran, glycerin, erythritol, glycerol, arabitol, sylitol, sorbitol, mannitol, mellibiose, melezitose, raffinose, mannotriose, stachyose, maltose, lactulose, maltulose, glucitol, maltitol, lactitol, iso-maltulose, and the like. In some embodiments, the sugar is selected from one or more of sucrose, mannitol, and trehalose. In some embodiments, the sugar is sucrose.

In some embodiments, provided is the pharmaceutical composition according to any of the above, wherein the concentration of the sugar is 20 mg/mL to 100 mg/mL. In some embodiments, the concentration of the sugar is 30 mg/mL to 80 mg/mL. In some embodiments, the concentration of the sugar is 40 mg/mL to 80 mg/mL. In some embodiments, the concentration of the sugar is 30 mg/mL to 50 mg/mL. In some embodiments, the concentration of the sugar is 20 mg/mL, 30 mg/mL, 36 mg/mL, 40 mg/mL, 45 mg/mL, 50 mg/mL, 60 mg/mL, 70 mg/mL, 80 mg/mL, 87 mg/mL, 90 mg/mL, or 100 mg/mL. In some embodiments, the concentration of the sugar is 87 mg/mL. In some embodiments, the concentration of the sugar is 80 mg/mL. In some embodiments, the concentration of the sugar is 50 mg/mL. In some embodiments, the concentration of the sugar is 45 mg/mL. In some embodiments, the concentration of the sugar is 40 mg/mL. In some embodiments, the concentration of the sugar is 36 mg/mL. In some embodiments, provided is the pharmaceutical composition according to any of the above, wherein the pharmaceutical composition further comprises an auxiliary material. In some embodiments, the auxiliary material is glycine, methionine, proline, histidine, phenylalanine, arginine hydrochloride, glutamic acid, aspartic acid, or disodium edetate. In some embodiments, the auxiliary material is glycine.

In some embodiments, provided is the pharmaceutical composition according to any of the above, wherein the concentration of the auxiliary material is 1 mg/mL to 30 mg/mL; preferably, the concentration of the auxiliary material is 5 mg/mL to 15 mg/mL. In some embodiments, provided is the pharmaceutical composition according to any of the above, wherein the concentration of the auxiliary material is 12 mg/mL. In some embodiments, provided is the pharmaceutical composition according to any of the above, wherein the concentration of the auxiliary material is 10 mg/mL. In some embodiments, provided is the pharmaceutical composition according to any of the above, wherein the concentration of the auxiliary material is 9 mg/mL.

In some embodiments, provided is the pharmaceutical composition according to any of the above, wherein the concentration of the buffer is 5 mM to 100 mM. In some embodiments, the concentration of the buffer is 5 mM, 10 mM, 20 mM, 30 mM, 40 mM, 50 mM, 60 mM, 70 mM, 80 mM, 90 mM, or 100 mM. In some embodiments, the concentration of the buffer is 5 mM to 30 mM. In some embodiments, the concentration of the buffer is 10 mM to 30 mM. In some embodiments, the concentration of the buffer is 5 mM to 10 mM. In some embodiments, the concentration of the buffer is 10 mM. In some embodiments, the concentration of the buffer is 5 mM.

In some embodiments, provided is the pharmaceutical composition according to any of the above, wherein the KD value of the affinity of the anti-IL-2 antibody for IL-2 is ≤5 nM. In some other embodiments, the KD value of the affinity of the anti-IL-2 antibody for IL-2 is >5 nM or ≥10 nM, and is ≤200 nM or ≤150 nM or ≤100 nM or ≤80 nM or ≤60 nM. In some embodiments, the KD value is >5 nM and ≤60 nM, >5 nM and ≤50 nM, ≥10 nM and ≤50 nM, ≥10 nM and ≤60 nM, >5 nM and ≤80 nM, ≥10 nM and ≤80 nM, >5 nM and ≤100 nM, ≥10 nM and ≤100 nM, >5 nM and ≤150 nM, or ≥10 nM and ≤150 nM.

In some embodiments, provided is the pharmaceutical composition according to any of the above, wherein the anti-IL-2 antibody comprises:
a HCDR1 set forth in SEQ ID NO: 13, 19, 25, 31, 37, or 43, and/or
a HCDR2 set forth in SEQ ID NO: 14, 20, 26, 32, 38, or 44, and/or
a HCDR3 set forth in SEQ ID NO: 15, 21, 27, 33, 39, or 45, and/or
a LCDR1 set forth in SEQ ID NO: 16, 22, 28, 34, 40, or 46, and/or
a LCDR2 set forth in SEQ ID NO: 17, 23, 29, 35, 41, or 47, and/or
a LCDR3 set forth in SEQ ID NO: 18, 24, 30, 36, 42, or 48.

In some embodiments, provided is the pharmaceutical composition according to any of the above, wherein the anti-IL-2 antibody comprises:
a HCDR1 set forth in SEQ ID NO: 13, 25, or 37, and/or
a HCDR2 set forth in SEQ ID NO: 14, 26, or 38, and/or
a HCDR3 set forth in SEQ ID NO: 15, 27, or 39, and/or
a LCDR1 set forth in SEQ ID NO: 16, 28, or 40, and/or
a LCDR2 set forth in SEQ ID NO: 17, 29, or 41, and/or
a LCDR3 set forth in SEQ ID NO: 18, 30, or 42.

In some embodiments, provided is the pharmaceutical composition according to any of the above, wherein the anti-IL-2 antibody comprises:
a HCDR1 set forth in SEQ ID NO: 19, 31, or 43, and/or
a HCDR2 set forth in SEQ ID NO: 20, 32, or 44, and/or
a HCDR3 set forth in SEQ ID NO: 21, 33, or 45, and/or
a LCDR1 set forth in SEQ ID NO: 22, 34, or 46, and/or
a LCDR2 set forth in SEQ ID NO: 23, 35, or 47, and/or
a LCDR3 set forth in SEQ ID NO: 24, 36, or 48.

In some embodiments, provided is the pharmaceutical composition according to any of the above, wherein the anti-IL-2 antibody comprises:
HCDR1-3 set forth in SEQ ID NOs: 13-15, respectively, and/or LCDR1-3 set forth in SEQ ID NOs: 16-18, respectively;
HCDR1-3 set forth in SEQ ID NOs: 19-21, respectively, and/or LCDR1-3 set forth in SEQ ID NOs: 22-24, respectively;
HCDR1-3 set forth in SEQ ID NOs: 25-27, respectively, and/or LCDR1-3 set forth in SEQ ID NOs: 28-30, respectively;
HCDR1-3 set forth in SEQ ID NOs: 31-33, respectively, and/or LCDR1-3 set forth in SEQ ID NOs: 34-36, respectively;
HCDR1-3 set forth in SEQ ID NOs: 37-39, respectively, and/or LCDR1-3 set forth in SEQ ID NOs: 40-42, respectively;
HCDR1-3 set forth in SEQ ID NOs: 43-45, respectively, and/or LCDR1-3 set forth in SEQ ID NOs: 46-48, respectively.

In some embodiments, provided is the pharmaceutical composition according to any of the above, wherein the anti-IL-2 antibody comprises:
a VH set forth in one of SEQ ID NOs: 1, 3, 5, 7, 9, and 11 or having at least 90%, 95%, 98%, or 99% identity thereto; and/or
a VH set forth in one of SEQ ID NOs: 2, 4, 6, 8, 10, and 12 or having at least 90%, 95%, 98%, or 99% identity thereto.

In some embodiments, provided is the pharmaceutical composition according to any of the above, wherein the anti-IL-2 antibody comprises:
a VH set forth in SEQ ID NO: 1 or having at least 90% identity thereto, and a VL set forth in SEQ ID NO: 2 or having at least 90% identity thereto;
a VH set forth in SEQ ID NO: 3 or having at least 90% identity thereto, and a VL set forth in SEQ ID NO: 4 or having at least 90% identity thereto;
a VH set forth in SEQ ID NO: 5 or having at least 90% identity thereto, and a VL set forth in SEQ ID NO: 6 or having at least 90% identity thereto;
a VH set forth in SEQ ID NO: 7 or having at least 90% identity thereto, and a VL set forth in SEQ ID NO: 8 or having at least 90% identity thereto;
a VH set forth in SEQ ID NO: 9 or having at least 90% identity thereto, and a VL set forth in SEQ ID NO: 10 or having at least 90% identity thereto; or
a VH set forth in SEQ ID NO: 11 or having at least 90% identity thereto, and a VL set forth in SEQ ID NO: 12 or having at least 90% identity thereto.

In some embodiments, provided is the pharmaceutical composition according to any of the above, wherein the variable regions and CDRs of the anti-IL-2 antibody use the Kabat numbering system.

In some embodiments, provided is the pharmaceutical composition according to any of the above, wherein the heavy chain variable region (VH) of the anti-IL-2 antibody is linked to the heavy chain constant region of human IgG1; the light chain variable region (VL) is linked to the kappa or lamda chain constant region.

In some embodiments, provided is the pharmaceutical composition according to any of the above, wherein the anti-IL-2 antibody comprises:
a HC set forth in one of SEQ ID NOs: 157, 159, 161, 163, 165, and 167 or having at least 80%, 90%, 95%, 98%, or 99% identity thereto; and/or
a LC set forth in one of SEQ ID NOs: 158, 160, 162, 164, 166, and 168 or having at least 80%, 90%, 95%, 98%, or 99% identity thereto.

In some embodiments, provided is the pharmaceutical composition according to any of the above, wherein the anti-IL-2 antibody comprises:
a full-length heavy chain (HC) set forth in SEQ ID NO: 157 or having at least 80%, 90%, 95%, 98%, or 99% identity thereto, and a full-length light chain (LC) set forth in SEQ ID NO: 158 or having at least 80%, 90%, 95%, 98%, or 99% identity thereto;
a HC set forth in SEQ ID NO: 159 or having at least 80%, 90%, 95%, 98%, or 99% identity thereto, and a LC set forth in SEQ ID NO: 160 or having at least 80%, 90%, 95%, 98%, or 99% identity thereto;
a HC set forth in SEQ ID NO: 161 or having at least 80%, 90%, 95%, 98%, or 99% identity thereto, and a LC set forth in SEQ ID NO: 162 or having at least 80%, 90%, 95%, 98%, or 99% identity thereto;
a HC set forth in SEQ ID NO: 163 or having at least 90% identity thereto, and a LC set forth in SEQ ID NO: 164 or having at least 80%, 90%, 95%, 98%, or 99% identity thereto;
a HC set forth in SEQ ID NO: 165 or having at least 90% identity thereto, and a LC set forth in SEQ ID NO: 166 or having at least 80%, 90%, 95%, 98%, or 99% identity thereto; or
a HC set forth in SEQ ID NO: 167 or having at least 80%, 90%, 95%, 98%, or 99% identity thereto, and a LC set forth in SEQ ID NO: 168 or having at least 80%, 90%, 95%, 98%, or 99% identity thereto.

In some embodiments, provided is the pharmaceutical composition according to any of the above, wherein the anti-IL-2 antibody is a Fab, an Fv, an sFv, a Fab', a F(ab')₂, a linear antibody, a single-chain antibody, an scFv, an sdAb, an sdFv, a nanobody, a peptibody, a domain antibody, or a multispecific antibody (a bispecific antibody, a diabody, a triabody, or a tetrabody, a tandem di-scFv, or a tandem tri-scFv); for example, the anti-IL-2 antibody is specifically an scFv, Fv, Fab, or Fab' fragment.

In some embodiments, provided is the pharmaceutical composition according to any of the above, wherein the IL-2 in the fusion protein is linked to the light chain variable region or the heavy chain variable region of the anti-IL-2 antibody; for example, the IL-2 is linked to the N-terminus of the light chain variable region or the heavy chain variable region of the anti-IL-2 antibody. In some embodiments, the IL-2 is linked to the N-terminus of the light chain variable region of the anti-IL-2 antibody.

In some embodiments, provided is the pharmaceutical composition according to any of the above, wherein the IL-2 in the fusion protein is linked to the anti-IL-2 antibody by a linker. In some embodiments, the linker is a flexible peptide linker. In some embodiments, the linker is 5-50, 10-40, 20-30, or 25 amino acids in length. In some embodiments, the linker is selected from the group consisting of: amino acid sequences represented by (GₘSₙ)ₓ or (GGNGT)ₓ or (YGNGT)ₓ, wherein m and n are each independently selected from the group consisting of integers from 1 to 8 (e.g., 1, 2, 3, 4, 5, 6, 7, or 8), and x is independently selected from the group consisting of integers from 1 to 20 (e.g., 1, 2, 3, 4, 5, 6, 7, 8, 9, 10, 11, 12, 13, 14, 15, 16, 17, 18, 19, or 20). In some specific embodiments, the linker has an amino acid sequence represented by (G₄S)ₓ, wherein x is independently selected from the group consisting of integers from 1 to 6 (for example, x is 4 or 5). The linker may also be another linker that is similar in flexibility and length to the amino acid sequences represented by (GₘSₙ)ₓ or (GGNGT)ₓ or (YGNGT)ₓ described above. In some embodiments, the amino acid sequence of the linker is set forth in SEQ ID No: 190.

In some embodiments, provided is the pharmaceutical composition according to any of the above, wherein the fusion protein selectively enhances the activity of or promotes the proliferation of cells that highly express IL-2Ra; the cells that highly express IL-2Ra are, for example, regulatory T (Treg) cells.

In some embodiments, provided is the pharmaceutical composition according to any of the above, wherein the IL-2 in the fusion protein has an amino acid substitution at position 3 (as an example, T3) or an N-terminal deletion. In some specific embodiments, the amino acid at position 3 of the IL-2 is substituted with Ala, Gln, or Glu (as an example, T3A, T3Q, or T3E), and the N-terminal deletion is the deletion of the first 3 or 7 amino acids from the N terminus.

In some embodiments, provided is the pharmaceutical composition according to any of the above, wherein the fusion protein comprises a HC and a LC selected from the group consisting of:
a HC set forth in SEQ ID No: 169, and a LC set forth in SEQ ID No: 158;
a HC set forth in SEQ ID No: 171, and a LC set forth in SEQ ID No: 160;
a HC set forth in SEQ ID No: 159, and a LC set forth in SEQ ID No: 172;
a HC set forth in SEQ ID No: 173, and a LC set forth in SEQ ID No: 162;
a HC set forth in SEQ ID No: 175, and a LC set forth in SEQ ID No: 164;
a HC set forth in SEQ ID No: 163, and a LC set forth in SEQ ID No: 176;
a HC set forth in SEQ ID No: 177, and a LC set forth in SEQ ID No: 166;
a HC set forth in SEQ ID No: 179, and a LC set forth in SEQ ID No: 168;
a HC set forth in SEQ ID No: 167, and a LC set forth in SEQ ID No: 180;
a HC set forth in SEQ ID No: 161, and a LC set forth in any of SEQ ID Nos: 174 and 181-186;
a HC set forth in SEQ ID No: 157, and a LC set forth in SEQ ID No: 170 or 187;
a HC set forth in SEQ ID No: 165, and a LC set forth in any of SEQ ID Nos: 178, 188, and 189; and
sequences having at least 80%, 90%, 95%, 98%, or 99% identity to the HCs and LCs described above.

In some embodiments, provided is the pharmaceutical composition according to any of the above, wherein the fusion protein selectively enhances the effect of IL-2 on the activity of Tregs (e.g., proliferation-promoting activity, STATS phosphorylation-promoting activity), and/or selectively increases the ability of IL-2 to promote the expression of one or more of FOXP3, CD25, and Icos in Treg cells.

In some embodiments, provided is the pharmaceutical composition according to any of the above, wherein the fusion protein further has one or more of (i)-(iii):
(i) activates the activity of Tregs more than it activates that of CD8+ T cells;
(ii) promotes STATS phosphorylation more in Treg cells than in CD8+ T cells; and
(iii) promotes the proliferation of Treg cells more than it promotes that of CD8+ T cells, CD4+ T cells, or NK cells.

In some embodiments, provided is the pharmaceutical composition according to any of the above, wherein the anti-IL-2 antibody comprises a heavy chain variable region and a light chain variable region; the heavy chain variable region has: a HCDR1 comprising the amino acid sequence of SEQ ID NO: 37, a HCDR2 comprising the amino acid sequence of SEQ ID NO: 38, and a HCDR3 comprising the amino acid sequence of SEQ ID NO: 39; the light chain variable region has: a LCDR1 comprising the amino acid sequence of SEQ ID NO: 40, a LCDR2 comprising the amino acid sequence of SEQ ID NO: 41, and a LCDR3 comprising the amino acid sequence of SEQ ID NO: 42. In some embodiments, the heavy chain variable region comprises the amino acid sequence of SEQ ID NO: 9, and the light chain variable region comprises the amino acid sequence of SEQ ID NO: 10. In some embodiments, the anti-IL-2 antibody comprises a heavy chain of the amino acid sequence of SEQ ID NO: 165 and a light chain of the amino acid sequence of SEQ ID NO: 166.

In some embodiments, provided is the pharmaceutical composition according to any of the above, wherein the IL-2 is fused, directly or by a linker, to the N-terminus of the light chain of the anti-IL-2 antibody. In some embodiments, the amino acid residue at position 3 of the IL-2 is E. In some embodiments, the fusion protein comprises a heavy chain of the amino acid sequence of SEQ ID NO: 165 and a light chain of the amino acid sequence of SEQ ID NO: 189.

In some embodiments, provided is the pharmaceutical composition according to any of the above, which comprises the following components:
(a) 10 mg/mL to 30 mg/mL said fusion protein, (b) 0.05 mg/mL to 2 mg/mL polysorbate or poloxamer, (c) 20 mg/mL to 100 mg/mL sugar, and (d) 10 mM to 30 mM histidine buffer, acetate buffer, or succinate buffer, the buffer having a pH of 5.5 to 6.5.

In some embodiments, provided is the pharmaceutical composition according to any of the above, which comprises the following components:
(a) 10 mg/mL to 30 mg/mL said fusion protein, (b) 0.1 mg/mL to 0.8 mg/mL polysorbate or poloxamer, (c) 30 mg/mL to 80 mg/mL sugar, and (d) 10 mM to 30 mM histidine buffer, acetate buffer, or succinate buffer, the buffer having a pH of 5.5 to 6.5. In some embodiments, provided is the pharmaceutical composition according to any of the above, which comprises the following components:
(a) 10 mg/mL to 30 mg/mL said fusion protein, (b) 0.1 mg/mL to 0.8 mg/mL polysorbate or poloxamer, (c) 40 mg/mL to 80 mg/mL sugar, and (d) 10 mM to 30 mM histidine buffer, acetate buffer, or succinate buffer, the buffer having a pH of 5.5 to 6.5. In some embodiments, provided is the pharmaceutical composition according to any of the above, which comprises the following components:
(a) 10 mg/mL to 30 mg/mL said fusion protein, (b) 0.1 mg/mL to 0.6 mg/mL polysorbate or poloxamer, (c) 40 mg/mL to 80 mg/mL sugar, and (d) 10 mM to 30 mM histidine buffer, acetate buffer, or succinate buffer, the buffer having a pH of 5.5 to 6.5. In some embodiments, provided is the pharmaceutical composition according to any of the above, which comprises the following components:
(a) 10 mg/mL to 30 mg/mL said fusion protein, (b) 0.1 mg/mL to 0.8 mg/mL polysorbate or poloxamer, (c) 40 mg/mL to 80 mg/mL sugar, and (d) 10 mM to 30 mM histidine buffer, the histidine buffer having a pH of 5.5 to 6.0.

In some embodiments, provided is the pharmaceutical composition according to any of the above, which comprises the following components:
(a) 10 mg/mL to 30 mg/mL said fusion protein, (b) 0.1 mg/mL to 0.6 mg/mL polysorbate or poloxamer, (c) 40 mg/mL to 80 mg/mL sugar, and (d) 10 mM to 30 mM histidine buffer, the histidine buffer having a pH of 5.5 to 6.0.

In some embodiments, provided is the pharmaceutical composition according to any of the above, which comprises the following components:
(a) 30 mg/mL said fusion protein, (b) 0.2 mg/mL polysorbate 80, (c) 80 mg/mL sucrose, and (d) 10 mM acetate buffer, the acetate buffer having a pH of 5.0.

In some embodiments, provided is the pharmaceutical composition according to any of the above, which comprises the following components:
(a) 30 mg/mL said fusion protein, (b) 0.2 mg/mL polysorbate 80, (c) 80 mg/mL sucrose, and (d) 10 mM histidine buffer, acetate buffer, or succinate buffer, the buffer having a pH of 5.5.

In some embodiments, provided is the pharmaceutical composition according to any of the above, which comprises the following components:
(a) 30 mg/mL said fusion protein, (b) 0.2 mg/mL polysorbate 80, (c) 80 mg/mL sucrose, and (d) 10 mM histidine buffer, the histidine buffer having a pH of 6.0.

In some embodiments, provided is the pharmaceutical composition according to any of the above, which comprises the following components:
(a) 30 mg/mL said fusion protein, (b) 0.2 mg/mL polysorbate 80, (c) 80 mg/mL sucrose, and (d) 10 mM histidine buffer, the histidine buffer having a pH of 6.5.

In some embodiments, provided is the pharmaceutical composition according to any of the above, which comprises the following components:
(a) 10 mg/mL said fusion protein, (b) 0.2 mg/mL polysorbate 80, (c) 80 mg/mL sucrose, and (d) 10 mM acetate buffer, the acetate buffer having a pH of 5.5.

In some embodiments, provided is the pharmaceutical composition according to any of the above, which comprises the following components:
(a) 30 mg/mL said fusion protein, (b) 0.2 mg/mL polysorbate 80, (c) 80 mg/mL trehalose, and (d) 10 mM acetate buffer, the acetate buffer having a pH of 5.5.

In some embodiments, provided is the pharmaceutical composition according to any of the above, which comprises the following components:
(a) 30 mg/mL said fusion protein, (b) 0.1 mg/mL to 0.6 mg/mL poloxamer 188, (c) 80 mg/mL sucrose, and (d) 10 mM acetate buffer, the acetate buffer having a pH of 5.5.

In some embodiments, provided is the pharmaceutical composition according to any of the above, which comprises the following components:
(a) 30 mg/mL said fusion protein, (b) 0.1 mg/mL to 0.6 mg/mL poloxamer 188, (c) 80 mg/mL sucrose, and (d) 10 mM histidine buffer, the histidine buffer having a pH of 5.5. In some embodiments, provided is the pharmaceutical composition according to any of the above, which comprises the following components:
(a) 10 mg/mL to 30 mg/mL said fusion protein, (b) 0.1 mg/mL to 0.6 mg/mL poloxamer 188, (c) 80 mg/mL sucrose, and (d) 10 mM histidine-histidine hydrochloride buffer, the histidine-histidine hydrochloride buffer having a pH of 5.5 to 6.0.

In some embodiments, provided is the pharmaceutical composition according to any of the above, which comprises the following components:
(a) 10 mg/mL said fusion protein, (b) 0.6 mg/mL poloxamer 188, (c) 80 mg/mL sucrose, and (d) 10 mM histidine-histidine hydrochloride buffer, the histidine-histidine hydrochloride buffer having a pH of 6.0.

In some embodiments, provided is the pharmaceutical composition according to any of the above, which comprises the following components:
(a) 30 mg/mL said fusion protein, (b) 0.6 mg/mL poloxamer 188, (c) 80 mg/mL sucrose, and (d) 10 mM histidine-histidine hydrochloride buffer, the histidine-histidine hydrochloride buffer having a pH of 6.0.

In some embodiments, provided is the pharmaceutical composition according to any of the above, which comprises the following components:
(a) 30 mg/mL said fusion protein, (b) 0.6 mg/mL polysorbate 80, (c) 80 mg/mL sucrose, and (d) 10 mM histidine-histidine hydrochloride buffer, the histidine-histidine hydrochloride buffer having a pH of 6.0.

In some embodiments, provided is the pharmaceutical composition according to any of the above, which comprises the following components:
(a) 30 mg/mL said fusion protein, (b) 0.4 mg/mL polysorbate 80, (c) 87 mg/mL sucrose, and (d) 10 mM histidine-histidine hydrochloride buffer, the histidine-histidine hydrochloride buffer having a pH of 6.0.

In some embodiments, provided is the pharmaceutical composition according to any of the above, which comprises the following components:
(a) 30 mg/mL said fusion protein, (b) 0.8 mg/mL polysorbate 80, (c) 87 mg/mL sucrose, and (d) 10 mM histidine-histidine hydrochloride buffer, the histidine-histidine hydrochloride buffer having a pH of 6.0.

In some embodiments, provided is the pharmaceutical composition according to any of the above, which comprises the following components:
(a) 30 mg/mL said fusion protein, (b) 1.5 mg/mL polysorbate 80, (c) 87 mg/mL sucrose, and (d) 10 mM histidine-histidine hydrochloride buffer, the histidine-histidine hydrochloride buffer having a pH of 6.0.

In some embodiments, provided is the pharmaceutical composition according to any of the above, which comprises the following components:
(a) 10 mg/mL to 30 mg/mL said fusion protein, (b) 0.1 mg/mL to 0.8 mg/mL polysorbate or poloxamer, (c) 30 mg/mL to 80 mg/mL sucrose, (d) 1 mg/mL to 30 mg/mL glycine, and (e) 5 mM to 10 mM histidine-histidine hydrochloride buffer, the histidine-histidine hydrochloride buffer having a pH of 6.0.

In some embodiments, provided is the pharmaceutical composition according to any of the above, which comprises the following components:
(a) 10 mg/mL to 30 mg/mL said fusion protein, (b) 0.1 mg/mL to 0.8 mg/mL polysorbate 80, (c) 30 mg/mL to 50 mg/mL sucrose, (d) 5 mg/mL to 15 mg/mL glycine, and (e) 5 mM to 10 mM histidine-histidine hydrochloride buffer, the histidine-histidine hydrochloride buffer having a pH of 6.0.

In some embodiments, provided is the pharmaceutical composition according to any of the above, which comprises the following components:
(a) 10 mg/mL said fusion protein, (b) 1.6 mg/mL polysorbate 80, (c) 36 mg/mL sucrose, (d) 12 mg/mL glycine, and (e) 10 mM histidine-histidine hydrochloride buffer, the histidine-histidine hydrochloride buffer having a pH of 6.0.

In some embodiments, provided is the pharmaceutical composition according to any of the above, which comprises the following components:
(a) 10 mg/mL said fusion protein, (b) 0.8 mg/mL polysorbate 80, (c) 36 mg/mL sucrose, and (d) 10 mM histidine-histidine hydrochloride buffer, the histidine-histidine hydrochloride buffer having a pH of 6.0.

In some embodiments, provided is the pharmaceutical composition according to any of the above, which comprises the following components:
(a) 10 mg/mL said fusion protein, (b) 0.8 mg/mL polysorbate 80, (c) 36 mg/mL sucrose, (d) 12 mg/mL glycine, and (e) 5 mM histidine-histidine hydrochloride buffer, the histidine-histidine hydrochloride buffer having a pH of 6.0.

In some embodiments, provided is the pharmaceutical composition according to any of the above, which comprises the following components:
(a) 10 mg/mL said fusion protein, (b) 0.8 mg/mL polysorbate 80, (c) 36 mg/mL sucrose, (d) 12 mg/mL glycine, and (e) 10 mM histidine-histidine hydrochloride buffer, the histidine-histidine hydrochloride buffer having a pH of 6.0.

In some embodiments, provided is the pharmaceutical composition according to any of the above, which comprises the following components:
(a) 10 mg/mL said fusion protein, (b) 1.6 mg/mL polysorbate 80, (c) 36 mg/mL sucrose, (d) 12 mg/mL glycine, and (e) 5 mM histidine-histidine hydrochloride buffer, the histidine-histidine hydrochloride buffer having a pH of 6.0.

In some embodiments, provided is the pharmaceutical composition according to any of the above, which comprises the following components:
(a) 10 mg/mL said fusion protein, (b) 0.8 mg/mL polysorbate 80, (c) 50 mg/mL sucrose, (d) 9 mg/mL glycine, and (e) 5 mM histidine-histidine hydrochloride buffer, the histidine-histidine hydrochloride buffer having a pH of 6.0.

In some embodiments, provided is the pharmaceutical composition according to any of the above, which comprises the following components:
(a) 10 mg/mL said fusion protein, (b) 0.8 mg/mL polysorbate 80, (c) 50 mg/mL sucrose, (d) 9 mg/mL glycine, and (e) 10 mM histidine-histidine hydrochloride buffer, the histidine-histidine hydrochloride buffer having a pH of 6.0.

In some embodiments, provided is the pharmaceutical composition according to any of the above, which comprises the following components:
(a) 10 mg/mL said fusion protein, (b) 0.8 mg/mL polysorbate 80, (c) 45 mg/mL sucrose, (d) 10 mg/mL glycine, and (e) 5 mM histidine-histidine hydrochloride buffer, the histidine-histidine hydrochloride buffer having a pH of 6.0.

In some embodiments, provided is the pharmaceutical composition according to any of the above, which comprises the following components:
(a) 10 mg/mL said fusion protein, (b) 0.8 mg/mL polysorbate 80, (c) 45 mg/mL sucrose, (d) 10 mg/mL glycine, and (e) 10 mM histidine-histidine hydrochloride buffer, the histidine-histidine hydrochloride buffer having a pH of 6.0.

In some embodiments, provided is the pharmaceutical composition according to any of the above, which comprises the following components:
(a) 10 mg/mL said fusion protein, (b) 0.8 mg/mL polysorbate 80, (c) 40 mg/mL sucrose, (d) 10 mg/mL glycine, and (e) 5 mM histidine-histidine hydrochloride buffer, the histidine-histidine hydrochloride buffer having a pH of 6.0.

In some embodiments, provided is the pharmaceutical composition according to any of the above, which comprises the following components:
(a) 10 mg/mL said fusion protein, (b) 0.8 mg/mL polysorbate 80, (c) 40 mg/mL sucrose, (d) 10 mg/mL glycine, and (e) 10 mM histidine-histidine hydrochloride buffer, the histidine-histidine hydrochloride buffer having a pH of 6.0.

The numerical ranges of the present disclosure are intended to include every number and subset of numbers within that range, whether specifically disclosed or not.

In some embodiments, provided is the pharmaceutical composition according to any of the above, wherein the pharmaceutical composition is a liquid formulation. In some embodiments, the solvent of the liquid formulation is water. In some embodiments, the route of administration of the composition is subcutaneous, intradermal, intravenous, or intramuscular injection. In some embodiments, the route of administration of the composition is subcutaneous injection.

The present disclosure further provides a lyophilized formulation, wherein the lyophilized formulation can be reconstituted to form the pharmaceutical composition according to any of the above. The present disclosure further provides a lyophilized formulation, which is a lyophilized form of the pharmaceutical composition according to any of the above.

The present disclosure further provides a method for preparing the lyophilized formulation, comprising the step of lyophilizing the pharmaceutical composition according to any of the above.

The present disclosure further provides a lyophilized formulation, wherein the lyophilized formulation is obtained by lyophilizing the pharmaceutical composition according to any of the above.

The present disclosure further provides a reconstituted solution, wherein the reconstituted solution is prepared by reconstituting the lyophilized formulation according to any of the above. The present disclosure further provides a lyophilized formulation, which is a reconstituted form of the lyophilized formulation according to any of the above.

The present disclosure further provides a product, comprising a container, wherein the container contains the pharmaceutical composition according to any of the above, the lyophilized formulation according to any of the above, or the reconstituted solution according to any of the above.

In some embodiments, the present disclosure further provides use of the pharmaceutical composition according to any of the above, the lyophilized formulation according to any of the above, the reconstituted solution according to any of the above, or the product according to any of the above in the preparation of a medicament for treating an autoimmune disease or delaying the progression of an autoimmune disease.

The present disclosure further provides a method for treating an autoimmune disease or delaying the progression of an autoimmune disease, comprising administering to a patient an effective amount of the pharmaceutical composition according to any of the above, the lyophilized formulation according to any of the above, the reconstituted solution according to any of the above, or the product according to any of the above.

In some embodiments, the present disclosure further provides the pharmaceutical composition according to any of the above, the lyophilized formulation according to any of the above, the reconstituted solution according to any of the above, or the product according to any of the above for use in treating an autoimmune disease or delaying the progression of an autoimmune disease.

In some embodiments, the autoimmune disease is selected from any one or a combination of the following: lupus, graft versus host disease, hepatitis C virus-induced vasculitis, type I diabetes, multiple sclerosis, inflammatory bowel disease, chronic GvHD, asthma, dermatitis, alopecia areata, acute lung injury, sepsis, reactive arthritis, and rheumatoid arthritis.

### BRIEF DESCRIPTION OF THE DRAWINGS

FIGs. 1A and 1B show the ELISA results for the anti-IL-2 antibodies blocking the binding of IL-2 to IL-2Rα and IL-2Rβ, respectively, wherein FIG. 1A shows the ELISA results for the anti-IL-2 antibodies blocking the binding of IL-2 to IL-2Rα, and FIG. 1B shows the ELISA results for the anti-IL-2 antibodies blocking the binding of IL-2 to IL-2Rβ.
FIG. 2 shows the abilities of the anti-IL-2 antibodies to bind to IL-2, as measured by ELISA.
FIGs. 3A-3H show the experimental results of stimulation of STATS phosphorylation in Tregs and CD8⁺ T cells in human peripheral blood by non-covalent complexes of anti-IL-2 antibody and IL-2.
FIGs. 4A-4D show the experimental results of activity of fusion proteins for stimulation of STATS phosphorylation in regulatory T cells (Tregs) and CD8⁺ T cells in human peripheral blood.
FIGs. 5A-5F show the experimental results of the effect of fusion proteins on the proliferation of lymphocytes in peripheral blood of Balb/c mice.
FIG. 6 shows the experimental results of the effect of fusion proteins on the proliferation of lymphocytes in the spleen of OVA-immunized mice.
FIG. 7 shows the results of the efficacy of fusion proteins in the delayed hypersensitivity mouse model.
FIGs. 8A-8B show the results of the efficacy of fusion proteins in the arthritis mouse model.
FIGs. 9A-9F show the experimental results of activity of fusion proteins formed by anti-IL-2 antibody and different IL-2 variants for stimulation of STATS phosphorylation in regulatory T cells (Tregs) and CD8⁺ T cells in human peripheral blood.
FIG. 10 shows the experimental results of the effect of D2-60-b-T3E on the protein content in the urine of MRL/lpr mice (n = 7-10).
FIGs. 11A and 11B show the experimental results of the effect of D2-60-b-T3E on the content of dsDNA IgG and dsDNA IgM in serum of MRL/lpr mice, respectively (**P < 0.01 vs. model control group).
FIGs. 12A and 12B show the experimental results of the effect of D2-60-b-T3E on renal function of MRL/lpr mice, respectively. * P < 0.05, **P < 0.01, ****P < 0.0001 vs. model control group, n = 9-10.
FIGs. 13A and 13B show the experimental results of the effect of D2-60-b-T3E on the content of IL-6 and INF-γ in serum of MRL/lpr mice, respectively. * P < 0.05, **P < 0.01, ****P < 0.0001 vs. model control group, n = 9-10.
FIG. 14 shows the experimental results of the effect of D2-60-b-T3E on the content of IL-10 in serum of MRL/lpr mice. * P < 0.05, **P < 0.01, ****P < 0.0001 vs. model control group, n = 9-10.
FIG. 15 shows the effect of D2-60-b-T3E on the pathological score of MRL/lpr mice. * P < 0.05, **P < 0.01, ****P < 0.0001 vs. model control group, n = 9-10.

### DETAILED DESCRIPTION

### Terminology

In order to facilitate the understanding of the present disclosure, some technical and scientific terms are specifically defined below. Unless otherwise explicitly defined herein, all other technical and scientific terms used herein have the meanings generally understood by those of ordinary skill in the art to which the present disclosure belongs. The three-letter and single-letter codes for amino acids used herein are as described in J. biol. chem, 243, p3558 (1968).

"Interleukin-2" or "IL-2" is intended to be interpreted broadly and includes any IL-2 related product, including but not limited to, human and non-human IL-2 homologs, fragments or truncations, fusion proteins (e.g., fused to a signal peptide or other active components (e.g., an antibody) and inactive components), modified forms (e.g., PEGylated, glycosylated, albumin conjugated/fused, Fc conjugated and/or fused, and hydroxyethylated forms), conservatively modified proteins, etc. The term encompasses any natural IL-2 of any vertebrate source, including mammals such as primates (e.g., humans) and rodents (e.g., mice and rats). The term also encompasses unprocessed IL-2 as well as any form of processed IL-2 derived from cells; naturally occurring IL-2 variants, such as splice variants or allelic variants; and conservatively modified variants of IL-2.

"II,-2Rα", "CD25" or "α subunit of IL-2 receptor" refers to any natural CD25 of any vertebrate source, including mammals such as primates (e.g., humans) and rodents (e.g., mice and rats). The term encompasses unprocessed "full-length" CD25 as well as any form of processed CD25 derived from cells; and naturally occurring CD25 variants, such as splice variants or allelic variants. In certain embodiments, CD25 is human CD25, with an illustrative sequence set forth in SEQ ID NO: 37.

"High affinity IL-2 receptor" refers to the heterotrimer form of the IL-2 receptor that consists of a receptor γ subunit (also known as a common cytokine-receptor γ subunit, γc, or CD132), a receptor β subunit (also known as CD122, p70, or IL-2Rβ), and a receptor α subunit (also known as CD25, p55, or IL-2Rα). In contrast, a "moderate affinity IL-2 receptor" refers to an IL-2 receptor that comprises only γ and β subunits but no α subunit (see, e.g., Olejniczak and Kasprzak, MedSci Monit 14, RA179-189 (2008)).

"Conservative modifications" are applicable to amino acid and nucleotide sequences. For particular nucleotide sequences, conservative modifications refer to those nucleic acids encoding identical or substantially identical amino acid sequences, or, in the case of nucleotides not encoding amino acid sequences, to substantially identical nucleotide sequences. For amino acid sequences, "conservative modifications" refer to the replacement of amino acids in a protein with other amino acids having similar characteristics (e.g., charge, side chain size, hydrophobicity/hydrophilicity, backbone conformation, and rigidity) such that changes can be made frequently without altering the biological activity of the protein. Those skilled in the art know that, generally speaking, a single amino acid replacement in a non-essential region of a polypeptide does not substantially change the biological activity (see, e.g., Watson et al. (1987) Molecular Biology of the Gene, The Benjamin/Cummings Pub. Co., p224, (4th edition)).

"Amino acid mutations" include amino acid substitutions, deletions, insertions, modifications, and any combination thereof, to obtain a final construct that possesses desired properties, such as enhanced stability and increased activity. Amino acid sequence deletions and insertions include amino-terminal and/or carboxyl-terminal deletions and amino acid insertions. Preferred amino acid mutations are amino acid substitutions. To alter the binding properties of, for example, an anti-IL-2 antibody, non-conservative amino acid substitutions may be made, i.e., one amino acid is replaced with another amino acid having different structural and/or chemical properties. Preferred amino acid substitutions include the replacement of hydrophobic amino acids with hydrophilic amino acids. Amino acid substitutions include the replacement with non-naturally occurring amino acids or with naturally occurring amino acid derivatives of the 20 standard amino acids (e.g., 4-hydroxyproline, 3-methylhistidine, ornithine, homoserine and 5-hydroxylysine). Amino acid mutations can be generated using genetic or chemical methods well known in the art, including site-directed mutagenesis, PCR, gene synthesis, chemical modification, and the like.

"Antibody" is used in the broadest sense and encompasses a variety of antibody structures, including, but not limited to monoclonal antibodies, polyclonal antibodies; monospecific antibodies, multispecific antibodies (e.g., bispecific antibodies); and full-length antibodies, and antibody fragments (or antigen-binding fragments, or antigen-binding moieties) so long as they exhibit the desired antigen-binding activity. An antibody may refer to an immunoglobulin, which is of a tetrapeptide chain structure formed by two heavy chains and two light chains linked by interchain disulfide bonds. The heavy chain constant regions of an immunoglobulin differ in their amino acid composition and arrangement, and thus in their antigenic specificity. Accordingly, immunoglobulins can be divided into five classes, or isotypes of immunoglobulins, namely IgM, IgD, IgG, IgA and IgE, with their corresponding heavy chains being µ chain, δ chain, γ chain, α chain and α chain, respectively. Ig of the same class can be divided into different subclasses according to differences in the amino acid composition of the hinge regions and the number and positions of disulfide bonds of the heavy chains; for example, IgG can be divided into IgG1, IgG2, IgG3 and IgG4. Light chains are divided into x or λ chains according to differences in the constant regions. Each of the five classes of Ig may have a x chain or λ chain. In the antibody heavy and light chains, the sequences of about 110 amino acids near the N-terminus vary considerably and thus are referred to as variable regions (V regions); the remaining amino acid sequences near the C-terminus are relatively stable and thus are referred to as constant regions (C regions). The variable regions comprise 3 hypervariable regions (CDRs) and 4 framework regions (FRs) with relatively conservative sequences. The 3 hypervariable regions determine the specificity of the antibody and thus are also known as complementarity determining regions (CDRs). Each of the light chain variable regions (VLs) and the heavy chain variable regions (VHs) consists of 3 CDR regions and 4 FR regions arranged from the amino terminus to the carboxyl terminus in the following order: FR1, CDR1, FR2, CDR2, FR3, CDR3, and FR4. The 3 CDR regions of the light chain refer to LCDR1, LCDR2, and LCDR3; and the 3 CDR regions of the heavy chain refer to HCDR1, HCDR2, and HCDR3.

For determination or definition of CDRs, the deterministic depiction of CDRs can be accomplished by resolving the structure of the antibody and/or resolving the structure of the antibody-ligand complex, and identifying residues involved in the antigen-binding site. This can be accomplished by any of a variety of techniques known to those skilled in the art, such as X-ray crystallography. A variety of analysis methods can be used to identify CDRs, including but not limited to the Kabat numbering scheme, the Chothia numbering scheme, the AbM numbering scheme, the IMGT numbering scheme, the contact definition, and the conformational definition.

The Kabat numbering scheme is a standard for numbering residues in antibodies and is generally used to identify CDR regions (see, e.g., Johnson & Wu, 2000, Nucleic Acids Res., 28:214-8).

The Chothia numbering scheme is similar to the Kabat numbering scheme, except that it takes into account the location of certain structural loop regions (see, e.g., Chothia et al., 1986, J. Mol. Biol., 196:901-17; Chothia et al., 1989, Nature, 342:877-83).

The AbM numbering scheme adopts a computer program integration suite for modeling antibody structures manufactured by Oxford Molecular Group (see, e.g., Martin et al., 1989, Proc Natl Acad Sci (USA), 86:9268-9272; "AbMTM, A Computer Program for Modeling Variable Regions of Antibodies", Oxford, UK; Oxford Molecular, Ltd.). The AbM numbering system adopts a combination of a knowledge database and the de-novo method to model the tertiary structure of antibodies from basic sequences (see those described in Samudrala et al., 1999, "Ab Initio Protein Structure Prediction Using a Combined Hierarchical Approach", PROTEINS, Structure, Function and Genetics Suppl., 3:194-198).

The contact definition is based on the analysis of the available complex crystal structures (see, e.g., MacCallum et al., 1996, J. Mol. Biol., 5:732-45).

In the conformational definition, the positions of the CDRs can be identified as residues that contribute enthalpy to the antigen binding (see, e.g., Makabe et al., 2008, Journal of Biological Chemistry, 283:1156-1166).

Other CDR boundary definitions may not strictly follow one of the above methods, but still overlap with at least a portion of the Kabat CDRs, although they may be shortened or lengthened based on predictions or experimental results that a particular residue or a particular group of residues do not significantly affect the antigen binding. As used herein, a CDR may refer to a CDR defined by any method known in the art, including combinations of methods. In the methods used herein, CDRs defined according to any of those methods may be used. For any given embodiment comprising more than one CDR, the CDRs may be defined according to any of Kabat, Chothia, extended, AbM, IMGT, contact, and/or conformational definitions.

"Monoclonal antibody" or "mAb" refers to an antibody obtained from a population of substantially homogeneous antibodies, i.e., the individual antibodies included in the population are identical except for possible naturally-occurring mutations that may be present in minor amounts. Monoclonal antibodies are highly specific, being directed against a single antigenic site. Furthermore, unlike polyclonal antibodies, each monoclonal antibody targets a single determinant on the antigen. The modifier "monoclonal" indicates the characteristics of an antibody obtained from a population of substantially homogeneous antibodies, and is not to be construed as requiring production of the antibody by any particular method. For example, monoclonal antibodies used according to the present disclosure can be prepared by the hybridoma methods first described in Kohler and Milstein, 1975, Nature 256: 495, or can be prepared by the recombinant DNA method described in U.S. Patent No. 4,816,567. For example, monoclonal antibodies can also be isolated from the generated phage library using the technique described in McCafferty et al., 1990, Nature 348:552-554.

"Human antibody" includes antibodies having variable and constant regions derived from human germline immunoglobulin sequences. The human antibody of the present disclosure may include amino acid residues not encoded by human germline immunoglobulin sequences (e.g., mutations introduced by random or site-specific mutagenesis *in vitro* or by somatic mutations *in vivo*). However, "human antibody" does not include antibodies in which CDR sequences derived from the germline of another mammalian species (such as mice) have been grafted into a human framework sequence (i.e., "humanized antibodies").

"Human antibody" or "recombinant human antibody" includes human antibodies prepared, expressed, created, or isolated by recombinant methods, involving techniques and methods well known in the art, such as:
(1) antibodies isolated from transgenic human immunoglobulin genes, transchromosomal animals (e.g., mice), or hybridomas prepared therefrom;
(2) antibodies isolated from host cells that have been transformed to express the antibodies, such as transfectomas;
(3) antibodies isolated from a recombinant combinatorial human antibody library; and
(4) antibodies prepared, expressed, created, or isolated by a method such as splicing human immunoglobulin gene sequences to other DNA sequences.

Such recombinant human antibodies comprise variable and constant regions that utilize specific human germline immunoglobulin sequences encoded by germline genes, and also include subsequent rearrangements and mutations which occur, for example, during antibody maturation.

"Antigen (Ag)" refers to a molecule used to immunize an immunologically active vertebrate to produce an antibody (Ab) that recognizes Ag; or a molecule or mimic used for screening in an expression library, e.g., especially a phage, yeast, or ribosome display library. Ag is defined more broadly herein, and is generally intended to include target molecules recognized by Abs. Therefore, Ag includes molecules, or portions or mimics thereof, used in immunization processes for producing Abs or in library screening for selecting Abs. Therefore, for the antibody of the present disclosure that binds to IL-2, full-length IL-2 from mammalian species (e.g., human, monkey, mouse, and rat IL-2), including monomers and polymers thereof (e.g., dimers, trimers, etc.), as well as clipping variants and other variants of IL-2, are all referred to as antigens.

"Epitope" refers to a site on an antigen to which an immunoglobulin (or antibody) specifically binds. An epitope may be formed from contiguous amino acids, or non-contiguous amino acids juxtaposed by tertiary folding of the protein. An epitope formed from contiguous amino acids is generally retained after exposure to a denaturing solvent, while an epitope formed by tertiary folding is generally lost after a denaturing solvent treatment. An epitope generally comprises, for example, 3 to 15 amino acids in a unique spatial conformation. Methods for determining what epitope is bound by a given antibody are well known in the art and include an immunoblotting assay, an immunoprecipitation assay, and the like. Methods for determining the spatial conformation of an epitope include techniques in the art and techniques described herein, such as X-ray crystallography and two-dimensional nuclear magnetic resonance. "Antibody fragment" includes single-chain antibodies; Fab, modified Fab, Fab', modified Fab', F(ab')2, Fv, Fab-Fv, Fab-dsFv, single-domain antibodies (e.g., VH or VL or VHH), scFv, bivalent or trivalent or tetravalent antibodies, Bis-scFv, diabodies, tribodies, triabodies, tetrabodies, and epitope-binding fragments of any of the above. Methods for producing and preparing such antibody fragments are well known in the art (see, e.g., Verma et al., 1998, Journal of Immunological Methods, 216, 165-181). Fab-Fv was first disclosed in WO2009/040562, and its disulfide-stabilized form Fab-dsFv was first disclosed in WO2010/035012. The antigen-binding fragment of the present disclosure also includes Fab and Fab' fragments described in WO2005/003169, WO2005/003170 and WO2005/003171. Multivalent antibodies may comprise multiple specificities (e.g., bispecificites) or may be monospecific (see, e.g., WO92/22583 and WO05/113605), and an example of the latter is Tri-Fab (or TFM) described in WO 92/22583.

"Binding to IL-2" refers to the ability to interact with IL-2 or an epitope thereof, wherein the IL-2 or the epitope thereof may be derived from humans.

"Antigen-binding site" refers to a continuous or discontinuous three-dimensional spatial site on an antigen that is recognized by an antibody or an antigen-binding fragment of the present disclosure.

"Regulatory T cell" or "Treg" refers to a specialized CD4⁺ T cell type that can inhibit the response of other T cells. Tregs are characterized by expression of the α subunit of IL-2 receptor (CD25) and the transcription factor forkhead box P3 (FOXP3), and play a key role in the induction and maintenance of peripheral self-tolerance to antigens, including those expressed by tumors. Treg requires IL-2 for its function and development as well as for the induction of its inhibitory characteristics.

"Effector function" refers to those biological activities attributable to the Fc region of an antibody (either the natural sequence Fc region or the amino acid sequence variant Fc region) and which vary with the antibody isotype. Examples of effector functions of an antibody include: C1q binding and complement-dependent cytotoxicity (CDC); Fc receptor binding; antibody-dependent cell-mediated cytotoxicity (ADCC); phagocytosis; down-regulation of cell surface receptors (e.g., B cell receptors); and B cell activation. "ADCC" refers to the direct killing of antibody-coated target cells by Fc receptor-expressing cells through recognition of the Fc segment of the antibody. The ADCC effector function of the antibody may be reduced or eliminated by modification of the Fc segment of the IgG. The modification refers to mutations in the heavy chain constant region of the antibody, such as mutations selected from the group consisting of N297A, L234A, L235A and P329G of IgG1, IgG2/4 chimera, and F234A/L235A of IgG4. "CDC" refers to the activation of the cytotoxic form of the complement cascade by binding the complement component C1q to the antibody Fc. Methods for detecting ADCC and CDC activity of an antibody are known in the art. For example, CDC can be evaluated by determining the binding activity between an antibody to be tested and an Fc receptor (e.g., C1q).

"Fc receptor" or "FcR" refers to a receptor that binds to the Fc region of an antibody. A preferred FcR is a human FcR. In addition, a preferred FcR is an FcR (γ receptor) binding to an IgG antibody and includes receptors of FcyRI, FcyRII, and FcyRIII subclasses, including allelic variants and alternatively spliced forms of those receptors. FcyRII receptors include FcγRIIA ("activated receptor") and FcyRIIB ("inhibitory receptor"), which have similar amino acid sequences that differ primarily in their cytoplasmic domains. The activated receptor FcγRIIA comprises an immunoreceptor tyrosine-based activation motif (ITAM) in its cytoplasmic domain. The inhibitory receptor FcyRIIB comprises an immunoreceptor tyrosine-based inhibitory motif (ITIM) in its cytoplasmic domain (see review M. Daeron, Annu. Rev. Immunol., 15:203-234 (1997)). FcRs are reviewed in Ravetch and Kinet, Annu. Rev. Immunol., 9:457-92 (1991); Capel et al., Immunomethods, 4:25-34 (1994); and de Haas et al., J. Lab. Clin. Med., 126:330-41 (1995). Other FcRs, including those to be identified in the future, are encompassed by the term "FcR" herein. The term also includes the neonatal receptor FcRn responsible for transferring maternal IgG to the fetus (Guyer et al., J. Immunol., 117:587 (1976) and Kim et al., J. Immunol. 24:249 (1994)).

"Specific binding" or "selective binding" refers to binding of an antibody to an epitope on a predetermined antigen. Typically, an antibody binds to a predetermined antigen or epitope thereof with an equilibrium dissociation constant (K_{D}) of about less than 10⁻⁷ M or even less and with an affinity that is at least twice as high as its affinity for binding to a non-specific antigen other than the predetermined antigen (or epitope thereof) or a closely related antigen (e.g., BSA, etc.), when determined by surface plasmon resonance (SPR) techniques in an instrument using recombinant human IL-2 or an epitope thereof as the analyte and an antibody as the ligand. The term "antigen-recognizing antibody" is used interchangeably herein with the term "specifically binding antibody".

"Affinity" is used herein as a measure of the strength of a non-covalent interaction between two molecules (e.g., an antibody or an antigen-binding fragment thereof and an antigen). The term "affinity" is used to describe monovalent interactions (intrinsic activity). The binding affinity between two molecules can be quantified by determining the dissociation constant (KD). KD can be determined by measuring the kinetics of complex formation and dissociation using, for example, the surface plasmon resonance (SPR) method (Biacore). The rate constants corresponding to the association and dissociation of a monovalent complex are referred to as the association rate constant ka (or kon) and the dissociation rate constant kd (or koff), respectively. K_{D} is related to ka and kd by the equation K_{D} = kd/ka. The value of the dissociation constant can be determined directly by well-known methods and can be calculated by methods such as those described by Caceci et al (1984, Byte 9:340-362) even for complex mixtures. For example, K_{D} can be determined by using a dual filtration nitrocellulose filter binding assay such as that disclosed by Wong & Lohman (1993, Proc. Natl. Acad. Sci. USA 90:5428-5432). Other standard assays for evaluating the binding ability of an antibody to a target antigen are known in the art and include, for example, ELISA, western blot, RIA and flow cytometry, as well as other assays exemplified elsewhere herein. The binding kinetics and binding affinity of the antibody can also be evaluated by standard assays known in the art, such as surface plasmon resonance (SPR), for example by using the Biacore^{™} system or KinExA. The binding avidities associated with different molecular interactions, e.g., the binding avidities of different antibodies to a given antigen, can be compared by comparing the K_{D} values of antibodies/fusion proteins. Similarly, the specificity of an interaction can be evaluated by determining and comparing the K_{D} value for the interaction of interest (e.g., a specific interaction between an antibody and an antigen) to the K_{D} value for the non-interaction of interest (e.g., a control antibody known not to bind to IL-2). In some embodiments, an anti-IL-2 antibody of the present disclosure is capable of binding to its target with an affinity at least 2 times, 10 times, 50 times, 100 times, 200 times, 500 times, 1000 times, or 10,000 times greater than its affinity for binding to another non-IL-2 molecule, without limitation herein.

"Inhibition" and "blocking" are used interchangeably and encompass both partial and complete inhibition/blocking. Inhibition and blocking are also intended to include any measurable reduction in binding affinity and cell (e.g., T cell) proliferation-promoting activity of IL-2 in contact with an anti-IL-2 antibody, as compared to IL-2 not in contact with an anti-IL-2 antibody.

"Homology" or "identity" refers to sequence similarity between two polynucleotide sequences or between two polypeptides. When positions in two compared sequences are occupied by identical bases or amino acid monomer subunits, e.g., if the position of each of two DNA molecules is occupied by adenine, the molecules are homologous at that position. The homology percentage between two sequences is a function of the number of matching or homologous positions shared by the two sequences divided by the number of positions compared × 100%. For example, if 6 out of 10 positions are matched or homologous when two sequences are optimally aligned, the two sequences are 60% homologous. Generally, when two sequences are aligned, comparison is performed to obtain the maximum homology percentage.

"Pharmaceutical composition" means that it comprises one or more fusion proteins as described herein, as well as other components such as physiologically/pharmaceutically acceptable carriers and excipients. The pharmaceutical composition is intended to promote the administration to an organism, so as to facilitate the absorption of the active ingredient, thereby exerting biological activity. As used herein, "pharmaceutical composition" and "formulation" are not mutually exclusive.

"Effective amount" comprises an amount sufficient to ameliorate or prevent a symptom or sign of a medical condition. An effective amount also refers to an amount sufficient to allow or facilitate diagnosis. The effective amount for a particular subject or veterinary subject may vary depending on factors such as the condition to be treated, the general health of the subject, the method and route and dosage of administration, and the severity of side effects. An effective amount may be the maximum dose or administration regimen to avoid significant side effects or toxic effects.

"Pharmaceutically acceptable carrier" or "pharmaceutically acceptable excipient" includes any material that, when combined with an active ingredient, allows the ingredient to retain biological activity and is non-reactive with the immune system of a subject. Examples include, but are not limited to, any standard pharmaceutical carrier, such as a phosphate buffered saline solution, water, an emulsion such as an oil/water emulsion, and various types of wetting agents. In some embodiments, the diluent for aerosol or parenteral administration is phosphate buffered saline (PBS) or normal (0.9%) saline. Compositions comprising such carriers are formulated by well-known conventional methods.

"Buffer" refers to a buffer that resists changes in pH by the action of its acid-base conjugate components. Examples of buffers that maintain the pH within an appropriate range include acetate, succinate, gluconate, histidine, oxalate, lactate, phosphate, citrate, tartrate, fumarate, glycylglycine, and other organic acid buffers.

"Histidine buffer" is a buffer comprising histidine. Examples of histidine buffers include histidine-histidine hydrochloride, histidine-histidine acetate, histidine-histidine phosphate, histidine-histidine sulfate, and the like. The histidine-histidine hydrochloride buffer is preferred. The histidine-histidine hydrochloride buffer may be prepared from histidine and hydrochloric acid, or from histidine and histidine hydrochloride.

"Citrate buffer" is a buffer comprising citrate ions. Examples of citrate buffers include citric acid-sodium citrate, citric acid-potassium citrate, citric acid-calcium citrate, citric acid-magnesium citrate, and the like. The preferred citrate buffer is citric acid-sodium citrate.

"Succinate buffer" is a buffer comprising succinate ions. Examples of succinate buffers include succinic acid-succinic acid sodium salt, succinic acid-potassium succinate, succinic acid-succinic acid calcium salt, and the like. The preferred succinate buffer is succinic acid-succinic acid sodium salt. Illustratively, the succinic acid-sodium succinate may be prepared from succinic acid and sodium hydroxide, or from succinic acid and succinic acid sodium salt.

"Phosphate buffer" is a buffer comprising phosphate ions. Examples of phosphate buffers include disodium hydrogen phosphate-sodium dihydrogen phosphate, disodium hydrogen phosphate-potassium dihydrogen phosphate, disodium hydrogen phosphate-citric acid, and the like. The preferred phosphate buffer is disodium hydrogen phosphate-sodium dihydrogen phosphate.

"Acetate buffer" is a buffer comprising acetate ions. Examples of acetate buffers include acetic acid-sodium acetate, histidine-histidine acetate, acetic acid-potassium acetate, acetic acid-calcium acetate, acetic acid-magnesium acetate, and the like. The preferred acetate buffer is acetic acid-sodium acetate.

"Lyophilized formulation" refers to a formulation or a pharmaceutical composition obtained by lyophilizing a pharmaceutical composition or a formulation in liquid or solution form in vacuum.

The pharmaceutical composition described herein can achieve a stable effect: a pharmaceutical composition in which the fusion protein substantially retains its physical and/or chemical stability and/or biological activity after storage; preferably, the pharmaceutical composition substantially retains its physical and chemical stability as well as its biological activity after storage. The storage period is generally selected based on a predetermined shelf life of the pharmaceutical composition. There are a variety of analytical techniques currently available for measuring protein stability, and the stability after storage for a selected period of time at a selected temperature can be measured.

A stable formulation is one in which no significant change is observed under the following conditions: stored at refrigeration temperature (2-8 °C) for at least 3 months, preferably 6 months, more preferably 1 year, and even more preferably up to 2 years. In addition, stable liquid formulations include liquid formulations that exhibit desirable features after storage at temperatures including 25 °C for periods including 1 month, 3 months or 6 months. Typical examples for stability are as follows: generally, no more than about 10%, preferably no more than about 5%, of fusion protein molecules aggregate or are degraded as measured by SEC-HPLC. The formulation is a pale yellow, nearly colorless and clear liquid, or a colorless and clear liquid, or is clear to slightly opalescent, by visual analysis. The concentration, pH and osmolality of the formulation have changes of no more than ±10%, preferably changes of no more than ±5%. Generally, aggregation of no more than about 10%, preferably no more than about 5% is formed in the formulation.

A fusion protein "retains its physical stability" in a pharmaceutical formulation if it shows no significant increase in aggregation, precipitation and/or denaturation upon visual inspection of color and/or clarity, or as determined by UV light scattering, size exclusion chromatography (SEC) and dynamic light scattering (DLS). Changes in protein conformation can be evaluated by fluorescence spectroscopy (which determines the protein tertiary structure) and by FTIR spectroscopy (which determines the protein secondary structure).

A fusion protein "retains its chemical stability" in a pharmaceutical formulation if it shows no significant chemical change. Chemical stability can be evaluated by detecting and quantifying chemically changed forms of the protein. Degradation processes that often change the chemical structure of proteins include hydrolysis or clipping (evaluated by methods such as size exclusion chromatography and CE-SDS), oxidation (evaluated by methods such as peptide mapping in conjunction with mass spectroscopy or MALDI/TOF/MS), deamidation (evaluated by methods such as ion-exchange chromatography, capillary isoelectric focusing, peptide mapping, and isoaspartic acid measurement), and isomerization (evaluated by measuring the isoaspartic acid content, peptide mapping, etc.).

A fusion protein "retains its biological activity" in a pharmaceutical formulation if the biological activity of the fusion protein at a given time is within a predetermined range of the biological activity exhibited during the preparation of the pharmaceutical formulation.

"Administrating", "giving", and "treating", when applied to animals, humans, experimental subjects, cells, tissues, organs or biological fluids, refer to contact of an exogenous drug, a therapeutic agent, a diagnostic agent or a composition with the animals, humans, subjects, cells, tissues, organs or biological fluids. "Administering", "giving", and "treating" can refer to, for example, therapeutic, pharmacokinetic, diagnostic, research and experimental methods. The treatment of cells comprises contacting the reagent with the cells and contacting the reagent with fluid, where the fluid is in contact with the cells. "Administering", "giving", and "treating" also refer to treating, e.g., cells by reagents, diagnosis, binding compositions or by another cell *in vitro* and *ex vivo.* "Treating", when applied to humans, veterinary, or research subjects, refers to therapeutic treatment, preventive or prophylactic measures, and research and diagnostic applications.

"Treatment" refers to administering a therapeutic agent, for example, comprising any of the pharmaceutical compositions of the present disclosure, either internally or externally to a patient with one or more symptoms of a disease on which the therapeutic agent is known to have a therapeutic effect. Generally, the therapeutic agent is administered in an amount effective to alleviate one or more symptoms of the disease in the patient or population being treated to induce regression of such symptoms or to inhibit the development of such symptoms to any clinically measurable degree. The amount of therapeutic agent effective to alleviate any particular symptom of the disease (also known as a "therapeutically effective amount") may vary depending on a variety of factors, such as the disease state, age, and weight of the patient, and the ability of the drug to produce a desired therapeutic effect in the patient. Whether a symptom of a disease has been alleviated can be evaluated by any clinical testing methods commonly used by doctors or other health care professionals to evaluate the severity or progression of the symptom. Although the embodiments of the present disclosure (for example, treatment methods or products) may not be effective in alleviating the symptoms of each disease of interest, they shall reduce the symptoms of the disease of interest in a statistically significant number of patients, as determined according to any statistical testing methods known in the art, such as Student t-test, chi-square test, Mann and Whitney's U test, Kruskal-Wallis test (H test), Jonckheere-Terpstra test and Wilcoxon test.

The details of one or more embodiments of the present disclosure are set forth in the specification above. Although any methods and materials similar or identical to those described herein can be used in the practice or testing of the present disclosure, the preferred methods and materials are described below. Other features, objects and advantages of the present disclosure will be apparent from the specification and the claims. In the specification and claims, singular forms include plural referents unless otherwise indicated clearly in the context. Unless otherwise defined, all technical and scientific terms used herein have the meanings generally understood by those of ordinary skill in the art to which the present disclosure belongs. All the patents and publications cited in the specification are incorporated by reference. The following examples are set forth in order to more fully illustrate the preferred embodiments of the present disclosure. These examples should not be construed in any way as limiting the scope of the present disclosure, which is defined by the claims.

### Examples-Fusion Proteins

The present disclosure is further described below with reference to examples, which, however, are not intended to limit the scope of the present disclosure. PCT/CN2021/076806 (priority: CN202010107662.4) is incorporated herein by reference in its entirety.

Experimental procedures without specific conditions indicated in the examples or test examples are generally conducted according to conventional conditions, or according to conditions recommended by the manufacturers of the starting materials or commercial products. See Sambrook et al., Molecular Cloning: A Laboratory Manual, Cold Spring Harbor Laboratory Press; Current Protocols in Molecular Biology, Ausubel et al., Greene Publishing Association, Wiley Interscience, NY Reagents without specific origins indicated are commercially available conventional reagents.

### Example 1. Screening for Anti-IL-2 Antibodies

### A) Screening of human natural phage Fab library

The preserved natural library phage suspension was diluted and blocked with BSA, and incubated with magnetic bead Dynabeads (M-280, Invitrogen). The phages were collected after negative screening and incubation. The beads were bound and washed according to the Kingfisher magnetic bead screening system method (Thermo Scientific), and incubated with 5% BSA. Dynabeads were coated and blocked by biotin-labeled IL-2 with a human Fc-tag (Sanyou Biopharmaceutical, GenBank Accession No: P60568-1), incubated with a phage suspension collected after negative screening, and bound and washed according to the Kingfisher magnetic bead screening system method. The phages were eluted with pancreatin. After the elution, the phage solution was mixed well with *E. coli* SS320 cells (Sanyou Biopharmaceutical) in the logarithmic growth phase, and incubated at 37 °C for 30 min. *E. coli* SS320 cells were applied to a 2YT-Car⁺-Tet⁺ plate and cultured overnight in an incubator at 37 °C. The phage input, output and the like were calculated, and the input phages were prepared by scraping plates and screened for 3 rounds.

### B) Primary screening for monoclonal phages expressing anti-IL-2 Fab capable of binding to IL-2 by ELISA

2000 clones from the 3rd round of screening were picked and cultured overnight in a 96-well deep-well plate. The supernatant was collected by centrifugation. Anti-human IgG (STAR161, Bio-rad) was diluted to 2 µg/mL with PBS and added at 30 µL/well to coat an ELISA plate, and the plate was incubated overnight at 4 °C. The plate was washed with PBST (PBS, pH 7.4 + 0.1% Tween 20) 3 times. The plate was blocked with 1% BSA for 1 h at room temperature and washed with PBST 3 times. 30 µL of the supernatant obtained by centrifugation was added, and the plate was incubated at room temperature for 2 h and washed with PBST 3 times. Biotin-labeled IL-2 with a human Fc-tag was diluted to 2 µg/mL with PBS and added to the plate at 30 µL/well. The plate was incubated at room temperature for 1 h and washed with PBST 3 times. 30 µL of secondary antibody NeutrAvidin-HRP diluted in a 1:8000 ratio was added. The plate was incubated at room temperature for 1 h and washed with PBST 9 times. 30 µL of TMB was added for color development for 5-10 min at room temperature, then 30 µL of 2 M HCl or H₂SO₄ was added to terminate the reaction. The plate was read for data by a microplate reader at OD 450. Finally, 364 clones with specific sequences were obtained. C) Primary screening for anti-IL-2 Fab capable of blocking the binding of IL-2 to IL-2Rβ by ELISA

The above 364 clones were inoculated into 50 mL of 2YT-Car⁺-Tet⁺ medium in a 1:1000 ratio, and cultured overnight at 37 °C. The bacterial solution was transferred to a 50 mL centrifuge tube, and centrifuged at room temperature. The supernatant was discarded, and 0.5 mL of protein lysis buffer (10 mM Tris-HCl, pH 9.0, 1 mM EDTA, 5 mM MgCl₂, 25 U/mL Bezonase nuclease (Merck)) pre-cooled to 4 °C was added thereto. The mixture was left to stand on ice and incubated for 1 h, and centrifuged at 4 °C. The supernatant was collected for later use.

IL-2Rβ-Fc (CJ82, Novoprotein) (GenBank Accession No: NP_000869.1) was diluted to 8 µg/mL with PBS and added at 30 µL/well to coat an ELISA plate, and the plate was incubated overnight at 4 °C. The plate was washed with PBST 3 times. The plate was blocked with 1% BSA for 1 h at room temperature and washed with PBST 3 times. The obtained Fab lysate was incubated with biotin-labeled IL-2 with a human Fc-tag (2.5 µg/mL) at room temperature for 1 h, and added to the plate at 30 µL/well. The plate was incubated at room temperature for 1 h and washed with PBST 3 times. 30 µL of secondary antibody NeutrAvidin-HRP diluted in a 1:8000 ratio was added. The plate was incubated at room temperature for 1 h and washed with PBST 9 times. 30 µL of TMB was added for color development for 5-10 min at room temperature, then 30 µL of 2 M HCl or H₂SO₄ was added to terminate the reaction. The plate was read for data by a microplate reader at OD 450.

6 Fabs with strong capability of blocking the binding of IL-2 to IL-2Rβ were obtained. For 6 fully human-derived antibodies, the sequences of the heavy chain and light chain variable regions are shown in Table 1, and the sequences of the complementarity determining regions (CDRs) according to different numbering systems are shown in Table 2.

**Table 1. Sequences of heavy chain (HCVR) and light chain (LCVR) variable regions of fully human anti-IL-2 antibodies**

| Antibody No. | Amino acid sequences of heavy chain variable region (VH) and light chain variable region (VL) | | Sequence No. |
|---|---|---|---|
| A2-22 | VH | QVQLVQSGAEVKKPGASVKVSCKASGYTFTSY YMHWVRQAPGQGLEWMGIINPSGGSTSYAQK FQGRVTMTRDTSTSTVYMELSSLRSEDTAVYY CAREDWMEYEYYYGMDVWGQGTLVTVSS | SEQ ID No: 1 |
| | VL | EIVLTQSPGTLSLSPGERATLSCRASQSVSNNYL AWYQQKPGQAPRLLIYGASSRATGIPDRFSGSG SGTDFTLTISRLEPEDFAVYSCQQYGSSPLTFGQ GTRLEIK | SEQ ID No: 2 |
| A3-21 | VH | EVQLLESGGGLAQPGGSLRLSCAGSGFTFSSYA MSWVRQAPGKGLEWVSGITGTGYRTYYADSV KGRFTVSRDNSRNTVYLQMNSLRAEDTAVYYC VRESGHYYGWFDSWGQGTLVTVSS | SEQ ID No: 3 |
| | VL | QSALTQPPSVSGAPGQRVTISCTGSSSNIGAGYN VHWYQQVPGTVPKLLIDGKGNRPSGVPDRFSG SKSGTSASLAITGLQAEDEADYYCQSYDYMLS GSVVFGRGTKLTVL | SEQ ID No: 4 |
| B2-15 | VH | EVQLLESGAEVKKPGASVKVSCKASGYTFTSY YMHWVRQAPGQGLEWMGIINPSGGSTSYAQK FQGRVTMTRDTSTSTVYMELSSLRSEDTAVYY CAREGNSSGYYNAFDIWGQGTMVTVSS | SEQ ID No: 5 |
| | VL | EIVLTQSPGTLSLSPGERATLSCRASQSVSSSYLA WYQQKPGQAPRLLIYGASNRATGIPDRFSGSGS GADFTLTISRLEPEDFAVYYCQQYGSSPLTFGGG TKVEIK | SEQ ID No: 6 |
| C2-53 | VH | EVQLVESGGGLVQPGRSLILSCAASGFTFDDYA MHWVRQAPGKGLEWVSGISWNSGSIGYADSV KGRFTISRDNAKNSLYLQMNSLRAEDTALYYC AKDQLRWGTYYYYGMDVWGQGTTVTVSS | SEQ ID No: 7 |
| | VL | DIQMTQSPSTLSASVGDRVTITCRASQSVSIWVA WYQQKPGKAPKLLIYKASSLERGVPTRFSGSGS GTEFTLTISSLQPEDFATYHCQQYDTGATFGQGT KVEMK | SEQ ID No: 8 |
| D2-60 | VH | QVQLVQSGAEVKKPGASVKVSCKASGYTFTSY GISWVRQAPGQGLEWMGWISAYNGNTNYAQK LQGRVTMTTDTSTSTAYMELRSLRSDDTAVYY CAREYSSWYSGGMDVWGQGTTVTVSS | SEQ ID No: 9 |
| | VL | DIQMTQSPSSLSASVGDRVTITCRASQGISNYLA WYQQKPGKVPKLLIYAASTLQSGVPSRFSGSGS GTDFTLTISSLQPEDVATYYCQKYDSAPFTFGQ GTRLEIK | SEQ ID No: 10 |
| D3-68 | VH | QVQLVQSGPEVKKPGASVKVSCKASGYTFTSY GISWVRRAPGQGLEWMGWISAYNGNTNYAQK LQGRVTMTTDTSTSTAYMELRSLRSDDTAVYY CARDLQSPTYYYGSGVIDYWGQGTLVTVSS | SEQ ID No: 11 |
| | VL | DIQLTQSPSTLSASVGDRVTISCRASRAVSPWLA WYQHRPGQAPKLLIYAASSLQSGVPSRFSGSGS GTDFTLTISSLQPEDFATYYCQQYRSYPFTFGPG TTVDIR | SEQ ID No: 12 |

**Table 2. Sequences of heavy chain and light chain complementarity determining regions (CDRs) of fully human anti-IL-2 antibodies**

| Antibody No. | CDR | Kabat numbering system | Chothia numbering system | IMGT numbering system | AbM numbering system |
|---|---|---|---|---|---|
| A2-22 | HCDR1 | SYYMH (SEQ ID No: 13) | GYTFTSY (SEQ ID No: 49) | GYTFTSYY (SEQ ID No: 85) | GYTFTSYYMH (SEQ ID No: 121) |
| | HCDR2 | IINPSGGSTSYAQ KFQG (SEQ ID No: 14) | NPSGGS (SEQ ID No: 50) | INPSGGST (SEQ ID No: 86) | IINPSGGSTS (SEQ ID No: 122) |
| | HCDR3 | EDWMEYEYYY GMDV (SEQ ID No: 15) | EDWMEYEYY YGMDV (SEQ ID No: 51) | ARE DWMEYE YYYGMDV (SEQ ID No: 87) | EDWMEYEYYYG MDV (SEQ ID No: 123) |
| | LCDR1 | RASQSVSNNYL A (SEQ ID No: 16) | RASQSVSNNY LA (SEQ ID No: 52) | QSVSNNY (SEQ ID No: 88) | RASQSVSNNYLA (SEQ ID No: 124) |
| | LCDR2 | GASSRAT (SEQ ID No: 17) | GASSRAT (SEQ ID No: 53) | GA (SEQ ID No: 89) | GASSRAT (SEQ ID No: 125) |
| | LCDR3 | QQYGSSPLT (SEQ ID No: 18) | QQYGSSPLT (SEQ ID No: 54) | QQYGSSPLT (SEQ ID No: 90) | QQYGSSPLT (SEQ ID No: 126) |
| A3-21 | HCDR1 | SYAMS (SEQ ID No: 19) | GFTFSSY (SEQ ID No: 55) | GFTFSSYA (SEQ ID No: 91) | GFTFSSYAMS (SEQ ID No: 127) |
| | HCDR2 | GITGTGYRTYYA DSVKG (SEQ ID No: 20) | TGTGYR (SEQ ID No: 56) | ITGTGYRT (SEQ ID No: 92) | GITGTGYRTY (SEQ ID No: 128) |
| | HCDR3 | ESGHYYGWFDS (SEQ ID No: 21) | ESGHYYGWFD S (SEQ ID No: 57) | VRESGHYYG WFDS (SEQ ID No: 93) | ESGHYYGWFDS (SEQ ID No: 129) |
| | LCDR1 | TGSSSNIGAGYN VH (SEQ ID No: 22) | TGSSSNIGAGY NVH (SEQ ID No: 58) | SSNIGAGYN (SEQ ID No: 94) | TGSSSNIGAGYN VH (SEQ ID No: 130) |
| | LCDR2 | GKGNRPS (SEQ ID No: 23) | GKGNRPS (SEQ ID No: 59) | GK (SEQ ID No: 95) | GKGNRPS (SEQ. ID No.: 131) |
| | LCDR3 | QSYDYMLSGSV V (SEQ ID No: 24) | QSYDYMLSGS VV (SEQ ID No: 60) | QSYDYMLSGS VV (SEQ ID No: 96) | QSYDYMLSGSV V (SEQ ID No: 132) |
| B2-15 | HCDR1 | SYYMH (SEQ ID No: 25) | GYTFTSY (SEQ ID No: 61) | GYTFTSYY (SEQ ID No: 97) | GYTFTSYYMH (SEQ ID No: 133) |
| | HCDR2 | IINPSGGSTSYAQ KFQG (SEQ ID No: 26) | NPSGGS (SEQ ID No: 62) | INPSGGST (SEQ ID No: 98) | IINPSGGSTS (SEQ ID No: 134) |
| | HCDR3 | EGNSSGYYNAF DI (SEQ ID No: 27) | EGNSSGYYNA FDI (SEQ ID No: 63) | AREGNSSGYY NAFDI (SEQ ID No: 99) | EGNSSGYYNAF DI (SEQ ID No: 135) |
| | LCDR1 | RASQSVSSSYLA (SEQ ID No: 28) | RASQSVSSSYL A (SEQ ID No: 64) | QSVSSSY (SEQ ID No: 100) | RASQSVSSSYLA (SEQ ID No: 136) |
| | LCDR2 | GASNRAT (SEQ ID No: 29) | GASNRAT (SEQ ID No: 65) | GA (SEQ ID No: 101) | GASNRAT (SEQ ID No: 137) |
| | LCDR3 | QQYGSSPLT (SEQ ID No: 30) | QQYGSSPLT (SEQ ID No: 66) | QQYGSSPLT (SEQ ID No: 102) | QQYGSSPLT (SEQ ID No: 138) |
| C2-53 | HCDR1 | DYAMH (SEQ ID No: 31) | GFTFDDY (SEQ ID No: 67) | GFTFDDYA (SEQ ID No: 103) | GFTFDDYAMH (SEQ ID No: 139) |
| | HCDR2 | GISWNSGSIGYA DSVKG (SEQ ID No: 32) | SWNSGS (SEQ ID No: 68) | ISWNSGSI (SEQ ID No: 104) | GISWNSGSIG (SEQ ID No: 140) |
| | HCDR3 | DQLRWGTYYYY GMDV (SEQ ID No: 33) | DQLRWGTYYY YGMDV (SEQ ID No: 69) | AKDQLRWGT YYYYGMDV (SEQ ID No: 105) | DQLRWGTYYYY GMDV (SEQ ID No: 141) |
| | LCDR1 | RASQSVSIWVA (SEQ ID No: 34) | RASQSVSIWVA (SEQ ID No: 70) | QSVSIW (SEQ ID No: 106) | RASQSVSIWVA (SEQ ID No: 142) |
| | LCDR2 | KASSLER (SEQ ID No: 35) | KASSLER (SEQ ID No: 71) | KA (SEQ ID No: 107) | KASSLER (SEQ ID No: 143) |
| | LCDR3 | QQYDTGAT (SEQ ID No: 36) | QQYDTGAT (SEQ ID No: 72) | QQYDTGAT (SEQ ID No: 108) | QQYDTGAT (SEQ ID No: 144) |
| D2-60 | HCDR1 | SYGIS (SEQ ID No: 37) | GYTFTSY (SEQ ID No: 73) | GYTFTSYG (SEQ ID No: 109) | GYTFTSYGIS (SEQ ID No: 145) |
| | HCDR2 | WISAYNGNTNY AQKLQG (SEQ ID No: 38) | SAYNGN (SEQ ID No: 74) | ISAYNGNT (SEQ ID No: 110) | WISAYNGNTN (SEQ ID No: 146) |
| | HCDR3 | EYSSWYSGGMD V (SEQ ID No: 39) | EYSSWYSGGM DV (SEQ ID No: 75) | AREYSSWYSG GMDV (SEQ ID No: 111) | EYSSWYSGGMD V (SEQ ID No: 147) |
| | LCDR1 | RASQGISNYLA (SEQ ID No: 40) | RASQGISNYLA (SEQ ID No: 76) | QGISNY (SEQ ID No: 112) | RASQGISNYLA (SEQ ID No: 148) |
| | LCDR2 | AASTLQS (SEQ ID No: 41) | AASTLQS (SEQ ID No: 77) | AA (SEQ ID No: 113) | AASTLQS (SEQ ID No: 149) |
| | LCDR3 | QKYDSAPFT (SEQ ID No: 42) | QKYDSAPFT (SEQ ID No: 78) | QKYDSAPFT (SEQ ID No: 114) | QKYDSAPFT (SEQ ID No: 150) |
| D3-68 | HCDR1 | SYGIS (SEQ ID No: 43) | GYTFTSY (SEQ ID No: 79) | GYTFTSYG (SEQ ID No: 115) | GYTFTSYGIS (SEQ ID No: 151) |
| | HCDR2 | WISAYNGNTNY AQKLQG (SEQ ID No: 44) | SAYNGN (SEQ ID No: 80) | ISAYNGNT (SEQ ID No: 116) | WISAYNGNTN (SEQ ID No: 152) |
| | HCDR3 | DLQSPTYYYGS GVIDY (SEQ ID No: 45) | DLQSPTYYYGS GVIDY (SEQ ID No: 81) | ARDLQSPTYY YGSGVIDY (SEQ ID No: 117) | DLQSPTYYYGSG VIDY (SEQ ID No: 153) |
| | LCDR1 | RASRAVSPWLA (SEQ ID No: 46) | RASRAVSPWL A (SEQ ID No: 82) | RAVSPW (SEQ ID No: 118) | RASRAVSPWLA (SEQ ID No: 153) |
| | LCDR2 | AASSLQS (SEQ ID No: 47) | AASSLQS (SEQ ID No: 83) | AA (SEQ ID No: 119) | AASSLQS (SEQ ID No: 155) |
| | LCDR3 | QQYRSYPFT (SEQ ID No: 48) | QQYRSYPFT (SEQ ID No: 84) | QQYRSYPFT (SEQ ID No: 120) | QQYRSYPFT (SEQ ID No: 156) |

### Example 2. Preparation of Full-Length Fully Human Anti-IL-2 Antibodies

The heavy chain and light chain variable regions of the 6 antibodies of Example 1 were linked to the heavy chain constant region of human IgG1 and the kappa or lamda chain constant region, respectively, to construct full-length fully human anti-IL-2 antibodies. The heavy chain constant region carries L234A and L235A mutations (LALA mutation) to remove possible ADCC function of the antibody. The full-length sequences of the 6 antibodies are shown in Table 3.

**Table 3. Full-length heavy and light chain sequences of fully human anti-IL-2 antibodies**

| Antibody No. | Full-length heavy chain (HC) and full-length light chain (LC) amino acid sequences | | Sequence No. |
|---|---|---|---|
| A2-22 | HC | QVQLVQSGAEVKKPGASVKVSCKASGYTFTSYYMHWVRQAP GQGLEWMGIINPSGGSTSYAQKFQGRVTMTRDTSTSTVYMELS SLRSEDTAVYYCAREDWMEYEYYYGMDVWGQGTLVTVSSAS TKGPSVFPLAPSSKSTSGGTAALGCLVKDYFPEPVTVSWNSGAL TSGVHTFPAVLQSSGLYSLSSVVTVPSSSLGTQTYICNVNHKPSN TKVDKKVEPKSCDKTHTCPPCPAPEAAGGPSVFLFPPKPKDTL MISRTPEVTCVVVDVSHEDPEVKFNWYVDGVEVHNAKTKPRE EQYNSTYRVVSVLTVLHQDWLNGKEYKCKVSNKALPAPIEKTI SKAKGQPREPQVYTLPPSRDELTKNQVSLTCLVKGFYPSDIAVE WESNGQPENNYKTTPPVLDSDGSFFLYSKLTVDKSRWQQGNVF SCSVMHEALHNHYTQKSLSLSPGK | SEQ ID No: 157 |
| | LC | EIVLTQSPGTLSLSPGERATLSCRASQSVSNNYLAWYQQKPGQA PRLLIYGASSRATGIPDRFSGSGSGTDFTLTISRLEPEDFAVYSCQ QYGSSPLTFGQGTRLEIKRTVAAPSVFIFPPSDEQLKSGTASVVC LLNNFYPREAKVQWKVDNALQSGNSQESVTEQDSKDSTYSLS STLTLSKADYEKHKVYACEVTHQGLSSPVTKSFNRGEC | SEQ ID No: 158 |
| A3-21 | HC | EVQLLESGGGLAQPGGSLRLSCAGSGFTFSSYAMSWVRQAPGK GLEWVSGITGTGYRTYYADSVKGRFTVSRDNSRNTVYLQMNS LRAEDTAVYYCVRESGHYYGWFDSWGQGTLVTVSSASTKGPS VFPLAPSSKSTSGGTAALGCLVKDYFPEPVTVSWNSGALTSGV HTFPAVLQSSGLYSLSSVVTVPSSSLGTQTYICNVNHKPSNTKV DKKVEPKSCDKTHTCPPCPAPEAAGGPSVFLFPPKPKDTLMISR TPEVTCVVVDVSHEDPEVKFNWYVDGVEVHNAKTKPREEQY NSTYRVVSVLTVLHQDWLNGKEYKCKVSNKALPAPIEKTISKA KGQPREPQVYTLPPSRDELTKNQVSLTCLVKGFYPSDIAVEWES NGQPENNYKTTPPVLDSDGSFFLYSKLTVDKSRWQQGNVFSCS VMHEALHNHYTQKSLSLSPGK | SEQ ID No: 159 |
| | LC | QSALTQPPSVSGAPGQRVTISCTGSSSNIGAGYNVHWYQQVPG TVPKLLIDGKGNRPSGVPDRFSGSKSGTSASLAITGLQAEDEAD YYCQSYDYMLSGSVVFGRGTKLTVLGQPKANPTVTLFPPSSEE LQANKATLVCLISDFYPGAVTVAWKADGSPVKAGVETTKPSKQ SNNKYAASSYLSLTPEQWKSHRSYSCQVTHEGSTVEKTVAPTE CS | SEQ ID No: 160 |
| B2-15 | HC | EVQLLESGAEVKKPGASVKVSCKASGYTFTSYYMHWVRQAP GQGLEWMGIINPSGGSTSYAQKFQGRVTMTRDTSTSTVYMELS SLRSEDTAVYYCAREGNSSGYYNAFDIWGQGTMVTVSSASTK GPSVFPLAPSSKSTSGGTAALGCLVKDYFPEPVTVSWNSGALTS GVHTFPAVLQSSGLYSLSSVVTVPSSSLGTQTYICNVNHKPSNT KVDKKVEPKSCDKTHTCPPCPAPEAAGGPSVFLFPPKPKDTLMI SRTPEVTCVVVDVSHEDPEVKFNWYVDGVEVHNAKTKPREEQ YNSTYRVVSVLTVLHQDWLNGKEYKCKVSNKALPAPIEKTISK AKGQPREPQVYTLPPSRDELTKNQVSLTCLVKGFYPSDIAVEWE SNGQPENNYKTTPPVLDSDGSFFLYSKLTVDKSRWQQGNVFSC SVMHEALHNHYTQKSLSLSPGK | SEQ ID No: 161 |
| | LC | EIVLTQSPGTLSLSPGERATLSCRASQSVSSSYLAWYQQKPGQA PRLLIYGASNRATGIPDRFSGSGSGADFTLTISRLEPEDFAVYYC QQYGSSPLTFGGGTKVEIKRTVAAPSVFIFPPSDEQLKSGTASVV CLLNNFYPREAKVQWKVDNALQSGNSQESVTEQDSKDSTYSL SSTLTLSKADYEKHKVYACEVTHQGLSSPVTKSFNRGEC | SEQ ID No: 162 |
| C2-53 | HC | EVQLVESGGGLVQPGRSLILSCAASGFTFDDYAMHWVRQAPGK GLEWVSGISWNSGSIGYADSVKGRFTISRDNAKNSLYLQMNSL RAEDTALYYCAKDQLRWGTYYYYGMDVWGQGTTVTVSSAST KGPSVFPLAPSSKSTSGGTAALGCLVKDYFPEPVTVSWNSGALT SGVHTFPAVLQSSGLYSLSSVVTVPSSSLGTQTYICNVNHKPSNT KVDKKVEPKSCDKTHTCPPCPAPEAAGGPSVFLFPPKPKDTLMI SRTPEVTCVVVDVSHEDPEVKFNWYVDGVEVHNAKTKPREEQ YNSTYRVVSVLTVLHQDWLNGKEYKCKVSNKALPAPIEKTISK AKGQPREPQVYTLPPSRDELTKNQVSLTCLVKGFYPSDIAVEWE SNGQPENNYKTTPPVLDSDGSFFLYSKLTVDKSRWQQGNVFSC SVMHEALHNHYTQKSLSLSPGK | SEQ ID No: 163 |
| | LC | DIQMTQSPSTLSASVGDRVTITCRASQSVSIWVAWYQQKPGKA PKLLIYKASSLERGVPTRFSGSGSGTEFTLTISSLQPEDFATYHCQ QYDTGATFGQGTKVEMKRTVAAPSVFIFPPSDEQLKSGTASVV CLLNNFYPREAKVQWKVDNALQSGNSQESVTEQDSKDSTYSL SSTLTLSKADYEKHKVYACEVTHQGLSSPVTKSFNRGEC | SEQ ID No: 164 |
| D2-60 | HC | QVQLVQSGAEVKKPGASVKVSCKASGYTFTSYGISWVRQAPG QGLEWMGWISAYNGNTNYAQKLQGRVTMTTDTSTSTAYMELR SLRSDDTAVYYCAREYSSWYSGGMDVWGQGTTVTVSSASTKG PSVFPLAPSSKSTSGGTAALGCLVKDYFPEPVTVSWNSGALTSG VHTFPAVLQSSGLYSLSSVVTVPSSSLGTQTYICNVNHKPSNTK VDKKVEPKSCDKTHTCPPCPAPEAAGGPSVFLFPPKPKDTLMIS RTPEVTCVVVDVSHEDPEVKFNWYVDGVEVHNAKTKPREEQ YNSTYRVVSVLTVLHQDWLNGKEYKCKVSNKALPAPIEKTISK AKGQPREPQVYTLPPSRDELTKNQVSLTCLVKGFYPSDIAVEWE SNGQPENNYKTTPPVLDSDGSFFLYSKLTVDKSRWQQGNVFSC SVMHEALHNHYTQKSLSLSPGK | SEQ ID No: 165 |
| | LC | DIQMTQSPSSLSASVGDRVTITCRASQGISNYLAWYQQKPGKVP KLLIYAASTLQSGVPSRFSGSGSGTDFTLTISSLQPEDVATYYCQ KYDSAPFTFGQGTRLEIKRTVAAPSVFIFPPSDEQLKSGTASVVC LLNNFYPREAKVQWKVDNALQSGNSQESVTEQDSKDSTYSLS STLTLSKADYEKHKVYACEVTHQGLSSPVTKSFNRGEC | SEQ ID No: 166 |
| D3-68 | HC | QVQLVQSGPEVKKPGASVKVSCKASGYTFTSYGISWVRRAPG QGLEWMGWISAYNGNTNYAQKLQGRVTMTTDTSTSTAYMELR SLRSDDTAVYYCARDLQSPTYYYGSGVIDYWGQGTLVTVSSAS TKGPSVFPLAPSSKSTSGGTAALGCLVKDYFPEPVTVSWNSGAL TSGVHTFPAVLQSSGLYSLSSVVTVPSSSLGTQTYICNVNHKPSN TKVDKKVEPKSCDKTHTCPPCPAPEAAGGPSVFLFPPKPKDTL MISRTPEVTCVVVDVSHEDPEVKFNWYVDGVEVHNAKTKPRE EQYNSTYRVVSVLTVLHQDWLNGKEYKCKVSNKALPAPIEKTI SKAKGQPREPQVYTLPPSRDELTKNQVSLTCLVKGFYPSDIAVE WESNGQPENNYKTTPPVLDSDGSFFLYSKLTVDKSRWQQGNVF SCSVMHEALHNHYTQKSLSLSPGK | SEQ ID No: 167 |
| | LC | DIQLTQSPSTLSASVGDRVTISCRASRAVSPWLAWYQHRPGQAP KLLIYAASSLQSGVPSRFSGSGSGTDFTLTISSLQPEDFATYYCQ QYRSYPFTFGPGTTVDIRRTVAAPSVFIFPPSDEQLKSGTASVVC LLNNFYPREAKVQWKVDNALQSGNSQESVTEQDSKDSTYSLS STLTLSKADYEKHKVYACEVTHQGLSSPVTKSFNRGEC | SEQ ID No: 168 |

| | | | |
|---|---|---|---|
| (Note: the underlined portion of HC is the heavy chain constant region of human IgG1, and the underlined portion of LC is the kappa chain constant region) | | | |

The above sequences were synthesized, digested with BamHI and XhoI, and inserted into a pcDNA3.1 expression vector (Life Technologies Cat. No. V790-20) through the BamHI/XhoI enzymatic digestion site. The expression vector and transfection reagent PEI (Polysciences, Inc. Cat. No. 23966) were transfected into HEK293 cells (Life Technologies Cat. No. 11625019) in a 1:2 ratio, and the cells were placed in a CO₂ incubator and incubated for 4-5 days. The expressed antibodies were isolated by centrifugation, purified by a conventional method, and identified to obtain the full-length fully human-derived antibodies of the present disclosure.

### Example 3. ELISA Assay on Anti-IL-2 Antibodies Capable of Blocking or Reducing the Binding of IL-2 to IL-2Rα and Blocking the Binding of IL-2 to IL-2Rβ

### A) ELISA assay on the blocking or reducing of the binding of IL-2 to IL-2Rα by antibodies

IL-2Rα-Fc (CJ78, Novoprotein) (Accession # NP_000408) was diluted to 0.5 µg/mL with PBS and added at 30 µL/well to coat an ELISA plate, and the plate was incubated overnight at 4 °C. The plate was washed with PBST 3 times. The plate was blocked with 1% BSA for 1 h at room temperature and washed with PBST 3 times. The anti-IL-2 antibody was diluted in a gradient to 10 µg/mL, 5 µg/mL, 2.5 µg/mL, 1.25 µg/mL, 0.63 µg/mL, 0.31 µg/mL, 0.16 µg/mL, and 0.08 µg/mL with PBS, pH 7.4, incubated with biotin-labeled IL-2 with a human Fc-tag (final concentration: 1 µg/mL) at room temperature for 15 min, added to the plate at 30 µL/well. The plate was incubated at room temperature for 1 h, and washed with PBST 3 times. 30 µL of secondary antibody NeutrAvidin-HRP diluted in a 1:6000 ratio was added, and the plate was incubated at room temperature for 1 h and washed with PBST 9 times. 30 µL of TMB was added for color development for 5-10 min at room temperature, then 30 µL of 2 M HCl or H₂SO₄ was added to terminate the reaction. The plate was read for data by a microplate reader at OD 450.

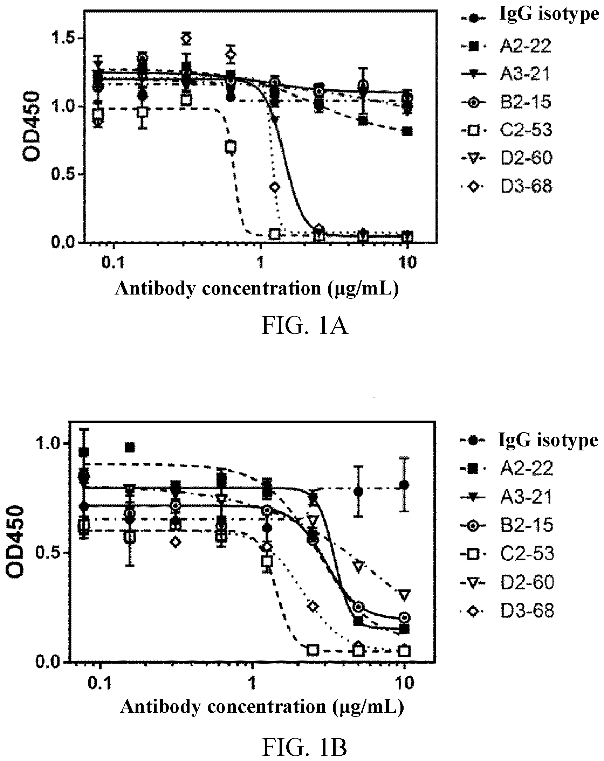

The results are shown in FIG. 1A. The results show that C2-53, D3-68 and A3-21 could completely block the binding of IL-2 to IL-2Rα, and the remaining clones might have only a weak ability to block (i.e., reduce) the binding of IL-2 to IL-2Rα at a high concentration of 10 µg/mL. IC₅₀ values for the antibodies are shown in Table 4.

**Table 4. IC₅₀ values for anti-IL-2 antibodies blocking the binding of IL-2 to IL-2Rα**

| Antibody No. | IC₅₀ (µg/mL) |
|---|---|
| A2-22 | N.A. |
| A3-21 | 1.456 |
| B2-15 | N.A. |
| C2-53 | 0.6591 |
| D2-60 | N.A. |
| D3-68 | 1.196 |

| | |
|---|---|
| (Note: N.A.: due to the weak blocking effect, no meaningful IC50 values could be obtained by fitting the curve.) | |

### B) ELISA assay on the blocking of the binding of IL-2 to IL-2Rβ by antibodies

IL-2Rβ-Fc (CJ82, Novoprotein) was diluted to 8 µg/mL with PBS and added at 30 µL/well to coat an ELISA plate, and the plate was incubated overnight at 4 °C. The plate was washed with PBST 3 times. The plate was blocked with 1% BSA for 1 h at room temperature and washed with PBST 3 times. The anti-IL-2 antibody was diluted in a gradient to 10 µg/mL, 5 µg/mL, 2.5 µg/mL, 1.25 µg/mL, 0.63 µg/mL, 0.31 µg/mL, 0.16 µg/mL, and 0.08 µg/mL with PBS, pH 7.4, incubated with biotin-labeled IL-2 with a human Fc-tag (final concentration: 5 µg/mL) at room temperature for 15 min, added to the plate at 30 µL/well. The plate was incubated at room temperature for 1 h, and washed with PBST 3 times. 30 µL of secondary antibody NeutrAvidin-HRP diluted in a 1:6000 ratio was added, and the plate was incubated at room temperature for 1 h and washed with PBST 9 times. 30 µL of TMB was added for color development for 5-10 min at room temperature, then 30 µL of 2 M HCl or H₂SO₄ was added to terminate the reaction. The plate was read for data by a microplate reader at OD 450.

The results are shown in FIG. 1B. The results show that all antibodies could completely block the binding of IL-2 to IL-2Rβ. IC₅₀ values for the antibodies are shown in Table 5.

**Table 5. IC₅₀ values for anti-IL-2 antibodies blocking the binding of IL-2 to IL-2Rβ**

| Antibody No. | IC₅₀ (µg/mL) |
|---|---|
| A2-22 | 2.728 |
| A3-21 | 3.475 |
| B2-15 | 3.045 |
| C2-53 | 1.441 |
| D2-60 | 8.272 |
| D3-68 | 2.134 |

### Example 4. Octet Assay on Anti-IL-2 Antibodies Blocking or Reducing the Binding of IL-2 to IL-2Rα and Blocking the Binding of IL-2 to IL-2Rβ

### A) Octet assay on the blocking or reducing of the binding of IL-2 to IL-2Rα by antibodies

A Streptavidin biosensor (Fortebio, #18-5020) was immersed in 200 µL of KB buffer (PBS, pH 7.4, 0.05% tween-20, 0.1% BSA) for 60 s for the wetting treatment. Then, biotinylated IL-2Rα-Fc (CJ78, Novoprotein) was diluted to 10 µg/mL with the KB buffer, and the sensor was immersed in 200 µL of the solution for 150 s. The sensor was immersed in the KB buffer for 60 s to elute excess IL-2Rα. IL-2-His (CX66, Novoprotein) was mixed with an anti-IL-2 antibody and diluted with KB buffer to final concentrations of 100 nM and 500 nM, and incubated at room temperature for 30 min. The sensor was immersed in the mixture for 300 s for association. The stronger the ability of the anti-IL-2 antibody to block or reduce the binding of IL-2 to IL-2Rα is, the lower the reading is. The values at 230 s after the beginning of the binding were read. The results are shown in Table 6.

**Table 6. The blocking or reducing of the binding of IL-2 to IL-2Rα by anti-IL-2 antibodies in Octet assay**

| Antibody No. | Values for blocking IL-2Rα at an Octet level |
|---|---|
| C2-53 | 0.410 |
| A3-21 | 0.428 |
| D3-68 | 0.473 |
| B2-15 | 0.700 |
| D2-60 | 0.754 |
| A2-22 | 0.848 |

### B) Octet assay on the blocking of the binding of IL-2 to IL-2Rβ by antibodies

A Streptavidin biosensor (Fortebio, #18-5020) was immersed in 200 µL of KB buffer (PBS, pH 7.4, 0.05% tween-20, 0.1% BSA) for 60 s for the wetting treatment. Then, biotinylated IL-2-Fc (Sanyou Biopharmaceutical) was diluted to 10 µg/mL with the KB buffer, and the sensor was immersed in 200 µL of the solution for 300 s. The sensor was immersed in the KB buffer for 60 s to elute excess IL-2. The anti-IL-2 antibody was diluted to a final concentration of 500 nM with the KB buffer, and the sensor was immersed in the antibody solution for association for 300 s. The sensor was immersed in the KB buffer for 60 s to elute excess anti-IL-2 antibody. The IL-2Rβ-Fc (CJ82, Novoprotein) was diluted to a final concentration of 8000 nM with the KB buffer, and the sensor was immersed in the antibody solution for association for 300 s. The stronger the ability of the anti-IL-2 antibody to block the binding of IL-2 to IL-2Rβ is, the lower the reading is. The values at 230 s after the beginning of the binding of IL-2Rβ-Fc were read. The results are shown in Table 7.

**Table 7. The blocking of the binding of IL-2 to IL-2Rβ by anti-IL-2 antibodies in Octet assay**

| Antibody No. | Values for blocking IL-2Rβ at an Octet level |
|---|---|
| A3-21 | 0.003 |
| C2-53 | 0.017 |
| D3-68 | 0.027 |
| A2-22 | 0.042 |
| B2-15 | 0.056 |
| D2-60 | 0.086 |

### Example 5. ELISA Assay on Binding of Anti-IL-2 Antibodies to IL-2

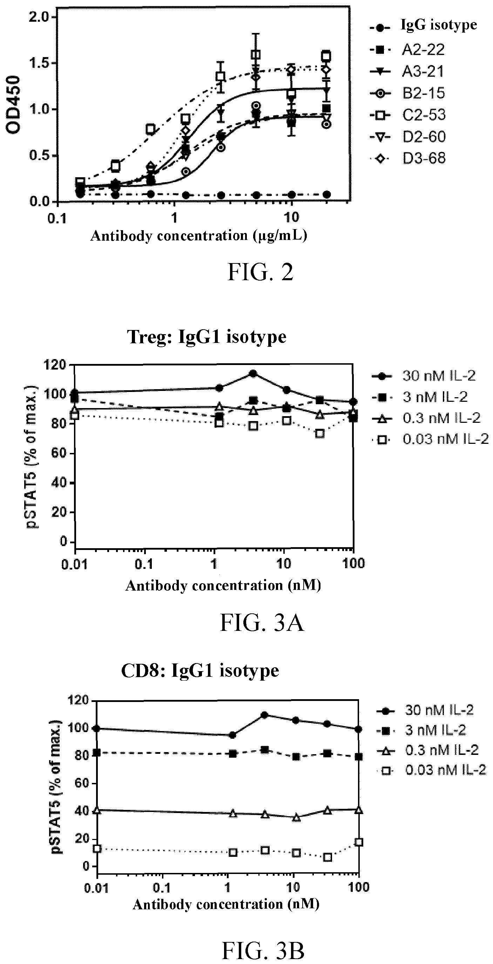

Anti-human-IgG (STAR161, Bio-rad) was diluted to 2 µg/mL with PBS and added at 30 µL/well to coat an ELISA plate, and the plate was incubated overnight at 4 °C. The plate was washed with PBST 3 times. The plate was blocked with 1% BSA for 1 h at room temperature and washed with PBST 3 times. The anti-IL-2 antibody was diluted in a gradient to 20 µg/mL, 10 µg/mL, 5 µg/mL, 2.5 µg/mL, 1.25 µg/mL, 0.625 µg/mL, 0.3125 µg/mL, and 0.15625 µg/mL with PBS. 30 µL of the antibody solution was added to an ELISA plate, and the plate was incubated at room temperature for 1 h and washed with PBST 3 times. Biotin-labeled IL-2 with a human Fc-tag (Sanyou Biopharmaceutical) was diluted to 2 µg/mL with PBS and added to the plate at 30 µL/well. The plate was incubated at room temperature for 1 h and washed with PBST 3 times. 30 µL of secondary antibody NeutrAvidin-HRP diluted in a 1:6000 ratio was added. The plate was incubated at room temperature for 1 h and washed with PBST 9 times. 30 µL of TMB was added for color development for 5-10 min at room temperature, then 30 µL of 2 M HCl or H₂SO₄ was added to terminate the reaction. The plate was read for data by a microplate reader at OD 450. The results are shown in FIG. 2 and Table 8. The results show that all antibodies could bind to IL-2 in this assay, among which C2-53 had a stronger binding ability.

**Table 8. EC₅₀ values for anti-IL-2 antibodies binding to IL-2**

| Antibody No. | EC₅₀ (µg/mL) |
|---|---|
| A2-22 | 1.289 |
| A3-21 | 1.350 |
| B2-15 | 2.117 |
| C2-53 | 0.7301 |
| D2-60 | 1.428 |
| D3-68 | 1.205 |

### Example 6. Biacore Assay on Affinity and Kinetics of Anti-IL-2 Antibodies for IL-2

Antibodies were captured with a Protein A sensor chip (GE, Cat# 29127556) of a Biacore instrument (Biacore T200, GE), in which anti-IL-2 antibodies were diluted to 1 µg/mL with 1× HBS-EP, and flowed at a flow rate of 10 µL/min for 30 s. Then, IL-2 (C013, Novoprotein) solutions at a series of concentration gradients flowed over the chip surface at a flow rate of 30 µL/min for association for 120 s. The solutions flowed at a flow rate of 30 µL/min for dissociation for 300 s. The reaction signals were detected in real time to obtain association and dissociation curves. After the dissociation was completed for each assay cycle, the chip was washed and regenerated with 10 mM Gly-HCl, pH 2.0. The data obtained from the assay were fitted with a 1:1 binding model to obtain the affinity values of the anti-IL-2 antibodies for IL-2. The results are shown in Table 9. The results show that all antibodies could bind to IL-2 with affinity values spanning from 0.267 nM for C2-53 to 39.8 nM for A2-22.

**Table 9. Affinity of anti-IL-2 antibodies for IL-2**

| Antibody No. | Kon (1/Ms) | Koff (1/s) | KD (M) |
|---|---|---|---|
| C2-53 | 1.64E+07 | 4.37E-03 | 2.67E-10 |
| D3-68 | 1. 16E+07 | 5.46E-02 | 4.72E-09 |
| D2-60 | 5.09E+05 | 6.51E-03 | 1.28E-08 |
| B2-15 | 1.46E+06 | 4.48E-02 | 3.06E-08 |
| A2-22 | 1.33E+06 | 5.27E-02 | 3.98E-08 |

### Example 7. Determination of Activity of Non-Covalent Complexes of Anti-IL-2 Antibody and IL-2 for STATS Phosphorylation in Human Peripheral Blood PBMC

The ability of the antibodies to regulate IL-2 activity was detected based on the levels of STATS phosphorylation in various cell populations (including Tregs, CD4⁺ T cells, and CD8⁺ T cells) in human peripheral blood treated with different concentrations of non-covalent complexes of anti-IL-2 antibodies and IL-2.

Basic medium: RPMI 1640 + 10% fetal bovine serum.

Antibody mixture: CD3 APC-Cy7 (BD 557832), CD4 BB515 (BD 564419), CD8 BB700 (BD 566452), CD25 BV421 (BD 564033), and pSTAT5 AF647 (BD 562076). Human PBMC STATS phosphorylation assay: freshly isolated human PBMC cells were adjusted to a density of 6.5 × 10⁶ cells/mL with a basic medium, and 80 µL of the mixture was added into a 96-well plate. The anti-IL-2 antibody at different concentrations was premixed with IL-2 at different concentrations for 30 min at room temperature, and 20 µL of the mixture was added to 90 µL of PBMC and stimulated at 37 °C for 20 min. Immediately thereafter, the cells were immobilized with a pre-warmed BD Cytofix buffer (BD, Cat No. 554655) at 37 °C for 15 min and then immobilized on ice for another 15 min. The plate was centrifuged at 400 g for 7 min at 4 °C. The supernatant was removed, and the plate was washed with 150 µL of PBS once. 150 µL of BD Phosflow Perm Buffer III (BD, Cat No. 558050) pre-cooled at -20 °C was added to perform membrane rupture overnight at -20 °C. The plate was centrifuged at 500 g for 7 min at 4 °C. The supernatant was removed, and the plate was washed with 150 µL of PBS, pH 7.4 twice. 100 µL of FcR blocking reagent diluted in a 1:200 ratio was added, and the plate was incubated at 4 °C for 20 min. The plate was centrifuged at 500 g for 7 min at 4 °C. The supernatant was removed, 50 µL of the antibody mixture was added, and the cells were stained at 4 °C for 1 h. The plate was centrifuged at 500 g for 7 min at 4 °C. The supernatant was removed, and the plate was washed with 200 µL of PBS, pH 7.4 once. The cells were resuspended in 150 µL of PBS at pH 7.4 and detected by flow cytometry (BD FACSCelesta). CD8⁺ T cells were defined as CD3⁺CD4⁻CD8⁺ cells, and Tregs were defined as CD3⁺CD4⁺CD8⁺CD25⁺ cells.

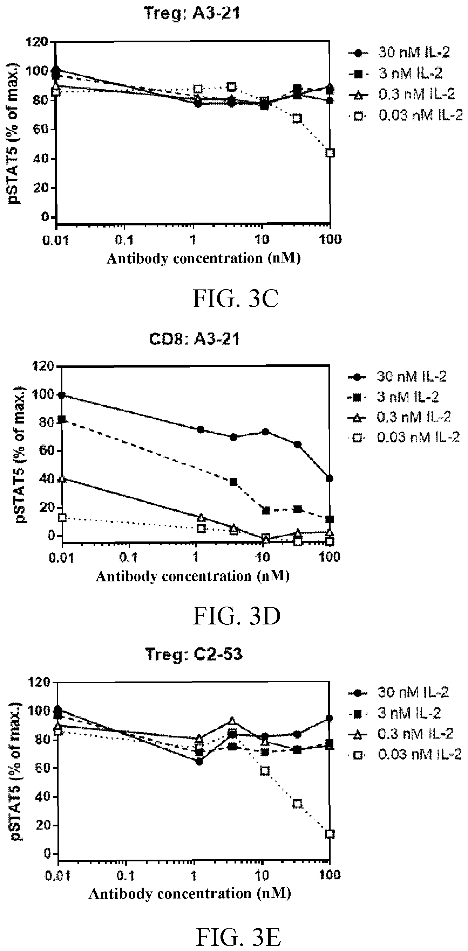

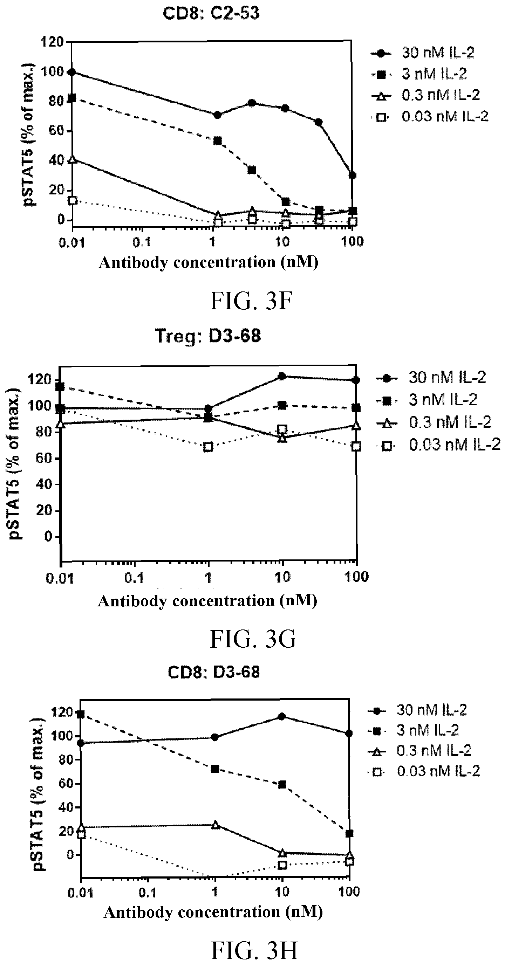

The fluorescence (MFI) values of pSTAT5 were counted for both cell populations, and the percentage of the value at each concentration to the maximum pSTAT5 MFI value in the fully activated state was calculated and fitted using a computer program or four-parameter regression calculation method. The results are shown in FIGs. 3A-3H. The results show that the IgG1 isotypes did not have any effect on the activation of pSTAT5 in Tregs and CD8⁺ T cells by IL-2 as antibody concentration increased. In contrast, A3-21 and C2-53 did not substantially affect the activity of IL-2 for Tregs, but significantly reduced the activity of IL-2 for CD8⁺ T cells over a wide concentration range. Only at high concentrations, such as 100 nM (A3-21) and 33.3 nM (C2-53), there was a certain decrease in the activity of IL-2 for Tregs, but under that condition, IL-2 completely had no activity for CD8⁺ T cells. In other words, A3-21 and C2-53 could inhibit or to a greater extent reduce, the activity of IL-2 for immune effector cells such as CD8⁺ T cells while not affecting or to a lesser extent reducing, the activity of IL-2 for Tregs. Similarly, D3-68 may also reduce the activity of IL-2 for CD8⁺ T cells to a greater extent than that for Tregs, although this ability may not be as significant as that of A3-21 and C2-53.

### Example 8. Preparation of Fusion Proteins

Non-covalent complexes of anti-IL-2 antibody and IL-2 may be easily dissociated *in vivo* to produce free IL-2, resulting in toxicity. We obtained 12 fusion proteins of anti-IL-2 antibody and IL-2 by conjugating human IL-2 to the N-terminus of the heavy or light chain of anti-IL-2 antibody via a (G₄S)₅ linker arm (i.e., GGGGSGGGGSGGGGSGGGGSGGGGS, SEQ ID No: 190).

For antibody numbering, if IL-2 is at the N-terminus of the heavy chain of the antibody, the fusion protein is suffixed a; and if IL-2 is at the N-terminus of the light chain of the antibody, the fusion protein is suffixed b.

For fused IL-2, it carries a T3A mutation (i.e., mutation of the amino acid at position 3 from Thr to Ala) to remove possible glycosylation. The mature human IL-2 protein does not contain amino acid M at position 1, so the numbering starts at amino acid A at position 2. Amino acid sequences of the 12 fusion proteins are as follows:

**Table 10. Full-length sequences of fusion proteins**

| Antibody No. | Full-length heavy chain (HC) and full-length light chain (HL) amino acid sequences | | Sequence No. |
|---|---|---|---|
| A2-22-a | HC | APASSSTKKTQLQLEHLLLDLQMILNGINNYKNPKL TRMLTFKFYMPKKATELKHLQCLEEELKPLEEVLNL AQSKNFHLRPRDLISNINVIVLELKGSETTFMCEYAD ETATIVEFLNRWITFAQSIISTLTGGGGSGGGGSGGGG SGGGGSGGGGSQVQLVQSGAEVKKPGASVKVSCK ASGYTFTSYYMHWVRQAPGQGLEWMGIINPSGGST SYAQKFQGRVTMTRDTSTSTVYMELSSLRSEDTAVY YCAREDWMEYEYYYGMDVWGQGTLVTVSSASTK GPSVFPLAPSSKSTSGGTAALGCLVKDYFPEPVTVS WNSGALTSGVHTFPAVLQSSGLYSLSSVVTVPSSSLG TQTYICNVNHKPSNTKVDKKVEPKSCDKTHTCPPCP APEAAGGPSVFLFPPKPKDTLMISRTPEVTCVVVDVS HEDPEVKFNWYVDGVEVHNAKTKPREEQYNSTYR VVSVLTVLHQDWLNGKEYKCKVSNKALPAPIEKTIS KAKGQPREPQVYTLPPSRDELTKNQVSLTCLVKGFY PSDIAVEWESNGQPENNYKTTPPVLDSDGSFFLYSKL TVDKSRWQQGNVFSCSVMHEALHNHYTQKSLSLSP GK | SEQ ID No: 169 |
| | LC | SEQ ID No: 158 | SEQ ID No: 158 |
| A2-22-b | HC | SEQ ID No: 157 | SEQ ID No: 157 |
| | LC | APASSSTKKTQLQLEHLLLDLQMILNGINNYKNPKL TRMLTFKFYMPKKATELKHLQCLEEELKPLEEVLNL AQSKNFHLRPRDLISNINVIVLELKGSETTFMCEYAD ETATIVEFLNRWITFAQSIISTLTGGGGSGGGGSGGGG SGGGGSGGGGSEIVLTQSPGTLSLSPGERATLSCRAS QSVSNNYLAWYQQKPGQAPRLLIYGASSRATGIPDR FSGSGSGTDFTLTISRLEPEDFAVYSCQQYGSSPLTFG QGTRLEIKRTVAAPSVFIFPPSDEQLKSGTASVVCLLN NFYPREAKVQWKVDNALQSGNSQESVTEQDSKDST YSLSSTLTLSKADYEKHKVYACEVTHQGLSSPVTKS FNRGEC | SEQ ID No: 170 |
| A3-21-a | HC | APASSSTKKTQLQLEHLLLDLQMILNGINNYKNPKL TRMLTFKFYMPKKATELKHLQCLEEELKPLEEVLNL AQSKNFHLRPRDLISNINVIVLELKGSETTFMCEYAD ETATIVEFLNRWITFAQSIISTLTGGGGSGGGGSGGGG SGGGGSGGGGSEVQLLESGGGLAQPGGSLRLSCAG SGFTFSSYAMSWVRQAPGKGLEWVSGITGTGYRTY YADSVKGRFTVSRDNSRNTVYLQMNSLRAEDTAVY YCVRESGHYYGWFDSWGQGTLVTVSSASTKGPSVF PLAPSSKSTSGGTAALGCLVKDYFPEPVTVSWNSGA LTSGVHTFPAVLQSSGLYSLSSVVTVPSSSLGTQTYIC NVNHKPSNTKVDKKVEPKSCDKTHTCPPCPAPEAA GGPSVFLFPPKPKDTLMISRTPEVTCVVVDVSHEDPE VKFNWYVDGVEVHNAKTKPREEQYNSTYRVVSVL TVLHQDWLNGKEYKCKVSNKALPAPIEKTISKAKG QPREPQVYTLPPSRDELTKNQVSLTCLVKGFYPSDIA VEWESNGQPENNYKTTPPVLDSDGSFFLYSKLTVDK SRWQQGNVFSCSVMHEALHNHYTQKSLSLSPGK | SEQ ID No: 171 |
| | LC | SEQ ID No: 160 | SEQ ID No: 160 |
| A3-21-b | HC | SEQ ID No: 159 | SEQ ID No: 159 |
| | LC | APASSSTKKTQLQLEHLLLDLQMILNGINNYKNPKL TRMLTFKFYMPKKATELKHLQCLEEELKPLEEVLNL AQSKNFHLRPRDLISNINVIVLELKGSETTFMCEYAD ETATIVEFLNRWITFAQSIISTLTGGGGSGGGGSGGGG SGGGGSGGGGSQSALTQPPSVSGAPGQRVTISCTGSS SNIGAGYNVHWYQQVPGTVPKLLIDGKGNRPSGVP DRFSGSKSGTSASLAITGLQAEDEADYYCQSYDYML SGSVVFGRGTKLTVLGQPKANPTVTLFPPSSEELQA NKATLVCLISDFYPGAVTVAWKADGSPVKAGVETTK PSKQSNNKYAASSYLSLTPEQWKSHRSYSCQVTHEG STVEKTVAPTECS | SEQ ID No: 172 |
| B2-15-a | HC | APASSSTKKTQLQLEHLLLDLQMILNGINNYKNPKL TRMLTFKFYMPKKATELKHLQCLEEELKPLEEVLNL AQSKNFHLRPRDLISNINVIVLELKGSETTFMCEYAD ETATIVEFLNRWITFAQSIISTLTGGGGSGGGGSGGGG SGGGGSGGGGSEVQLLESGAEVKKPGASVKVSCKA SGYTFTSYYMHWVRQAPGQGLEWMGIINPSGGSTS YAQKFQGRVTMTRDTSTSTVYMELSSLRSEDTAVYY CAREGNSSGYYNAFDIWGQGTMVTVSSASTKGPSV FPLAPSSKSTSGGTAALGCLVKDYFPEPVTVSWNSG ALTSGVHTFPAVLQSSGLYSLSSVVTVPSSSLGTQTYI CNVNHKPSNTKVDKKVEPKSCDKTHTCPPCPAPEA AGGPSVFLFPPKPKDTLMISRTPEVTCVVVDVSHEDP EVKFNWYVDGVEVHNAKTKPREEQYNSTYRVVSV LTVLHQDWLNGKEYKCKVSNKALPAPIEKTISKAKG | SEQ ID No: 173 |
| | | | |
| | LC | SEQ ID No: 162 | SEQ ID No: 162 |
| B2-15-b | HC | SEQ ID No: 161 | SEQ ID No: 161 |
| | LC | APASSSTKKTQLQLEHLLLDLQMILNGINNYKNPKL TRMLTFKFYMPKKATELKHLQCLEEELKPLEEVLNL AQSKNFHLRPRDLISNINVIVLELKGSETTFMCEYAD ETATIVEFLNRWITFAQSIISTLTGGGGSGGGGSGGGG SGGGGSGGGGSEIVLTQSPGTLSLSPGERATLSCRAS QSVSSSYLAWYQQKPGQAPRLLIYGASNRATGIPDR FSGSGSGADFTLTISRLEPEDFAVYYCQQYGSSPLTFG GGTKVEIKRTVAAPSVFIFPPSDEQLKSGTASVVCLL NNFYPREAKVQWKVDNALQSGNSQESVTEQDSKDS TYSLSSTLTLSKADYEKHKVYACEVTHQGLSSPVTK SFNRGEC | SEQ ID No: 174 |
| C2-53-a | HC | APASSSTKKTQLQLEHLLLDLQMILNGINNYKNPKL TRMLTFKFYMPKKATELKHLQCLEEELKPLEEVLNL AQSKNFHLRPRDLISNINVIVLELKGSETTFMCEYAD ETATIVEFLNRWITFAQSIISTLTGGGGSGGGGSGGGG SGGGGSGGGGSEVQLVESGGGLVQPGRSLILSCAAS GFTFDDYAMHWVRQAPGKGLEWVSGISWNSGSIGY ADSVKGRFTISRDNAKNSLYLQMNSLRAEDTALYYC AKDQLRWGTYYYYGMDVWGQGTTVTVSSASTKGP SVFPLAPSSKSTSGGTAALGCLVKDYFPEPVTVSWN SGALTSGVHTFPAVLQSSGLYSLSSVVTVPSSSLGTQ TYICNVNHKPSNTKVDKKVEPKSCDKTHTCPPCPAP EAAGGPSVFLFPPKPKDTLMISRTPEVTCVVVDVSH EDPEVKFNWYVDGVEVHNAKTKPREEQYNSTYRV VSVLTVLHQDWLNGKEYKCKVSNKALPAPIEKTISK AKGQPREPQVYTLPPSRDELTKNQVSLTCLVKGFYP SDIAVEWESNGQPENNYKTTPPVLDSDGSFFLYSKLT VDKSRWQQGNVFSCSVMHEALHNHYTQKSLSLSPG K | SEQ ID No: 175 |
| | LC | SEQ ID No: 164 | SEQ ID No: 164 |
| C2-53-b | HC | SEQ ID No: 163 | SEQ ID No: 163 |
| | LC | APASSSTKKTQLQLEHLLLDLQMILNGINNYKNPKL TRMLTFKFYMPKKATELKHLQCLEEELKPLEEVLNL AQSKNFHLRPRDLISNINVIVLELKGSETTFMCEYAD ETATIVEFLNRWITFAQSIISTLTGGGGSGGGGSGGGG SGGGGSGGGGSDIQMTQSPSTLSASVGDRVTITCRA SQSVSIWVAWYQQKPGKAPKLLIYKASSLERGVPTR FSGSGSGTEFTLTISSLQPEDFATYHCQQYDTGATFG QGTKVEMKRTVAAPSVFIFPPSDEQLKSGTASVVCLL NNFYPREAKVQWKVDNALQSGNSQESVTEQDSKDS TYSLSSTLTLSKADYEKHKVYACEVTHQGLSSPVTK | SEQ ID No: 176 |
| | | SFNRGEC | |
| D2-60-a | HC | APASSSTKKTQLQLEHLLLDLQMILNGINNYKNPKL TRMLTFKFYMPKKATELKHLQCLEEELKPLEEVLNL AQSKNFHLRPRDLISNINVIVLELKGSETTFMCEYAD ETATIVEFLNRWITFAQSIISTLTGGGGSGGGGSGGGG SGGGGSGGGGSQVQLVQSGAEVKKPGASVKVSCK ASGYTFTSYGISWVRQAPGQGLEWMGWISAYNGNT NYAQKLQGRVTMTTDTSTSTAYMELRSLRSDDTAVY YCAREYSSWYSGGMDVWGQGTTVTVSSASTKGPS VFPLAPSSKSTSGGTAALGCLVKDYFPEPVTVSWNS GALTSGVHTFPAVLQSSGLYSLSSVVTVPSSSLGTQT YICNVNHKPSNTKVDKKVEPKSCDKTHTCPPCPAPE AAGGPSVFLFPPKPKDTLMISRTPEVTCVVVDVSHE DPEVKFNWYVDGVEVHNAKTKPREEQYNSTYRVV SVLTVLHQDWLNGKEYKCKVSNKALPAPIEKTISKA KGQPREPQVYTLPPSRDELTKNQVSLTCLVKGFYPS DIAVEWESNGQPENNYKTTPPVLDSDGSFFLYSKLTV DKSRWQQGNVFSCSVMHEALHNHYTQKSLSLSPGK | SEQ ID No: 177 |
| | LC | SEQ ID No: 166 | SEQ ID No: 166 |
| D2-60-b | HC | SEQ ID No: 165 | SEQ ID No: 165 |
| | LC | APASSSTKKTQLQLEHLLLDLQMILNGINNYKNPKL TRMLTFKFYMPKKATELKHLQCLEEELKPLEEVLNL AQSKNFHLRPRDLISNINVIVLELKGSETTFMCEYAD ETATIVEFLNRWITFAQSIISTLTGGGGSGGGGSGGGG SGGGGSGGGGSDIQMTQSPSSLSASVGDRVTITCRA SQGISNYLAWYQQKPGKVPKLLIYAASTLQSGVPSR FSGSGSGTDFTLTISSLQPEDVATYYCQKYDSAPFTF GQGTRLEIKRTVAAPSVFIFPPSDEQLKSGTASVVCLL NNFYPREAKVQWKVDNALQSGNSQESVTEQDSKDS TYSLSSTLTLSKADYEKHKVYACEVTHQGLSSPVTK SFNRGEC | SEQ ID No: 178 |
| D3-68-a | HC | APASSSTKKTQLQLEHLLLDLQMILNGINNYKNPKL TRMLTFKFYMPKKATELKHLQCLEEELKPLEEVLNL AQSKNFHLRPRDLISNINVIVLELKGSETTFMCEYAD ETATIVEFLNRWITFAQSIISTLTGGGGSGGGGSGGGG SGGGGSGGGGSQVQLVQSGPEVKKPGASVKVSCKA SGYTFTSYGISWVRRAPGQGLEWMGWISAYNGNTN YAQKLQGRVTMTTDTSTSTAYMELRSLRSDDTAVYY CARDLQSPTYYYGSGVIDYWGQGTLVTVSSASTKG PSVFPLAPSSKSTSGGTAALGCLVKDYFPEPVTVSW NSGALTSGVHTFPAVLQSSGLYSLSSVVTVPSSSLGT QTYICNVNHKPSNTKVDKKVEPKSCDKTHTCPPCPA PEAAGGPSVFLFPPKPKDTLMISRTPEVTCVVVDVSH EDPEVKFNWYVDGVEVHNAKTKPREEQYNSTYRV VSVLTVLHQDWLNGKEYKCKVSNKALPAPIEKTISK AKGQPREPQVYTLPPSRDELTKNQVSLTCLVKGFYP SDIAVEWESNGQPENNYKTTPPVLDSDGSFFLYSKLT VDKSRWQQGNVFSCSVMHEALHNHYTQKSLSLSPG | SEQ ID No: 179 |
| | | K | |
| | LC | SEQ ID No: 168 | SEQ ID No: 168 |
| D3-68-b | HC | SEQ ID No: 167 | SEQ ID No: 167 |
| | LC | APASSSTKKTQLQLEHLLLDLQMILNGINNYKNPKL TRMLTFKFYMPKKATELKHLQCLEEELKPLEEVLNL AQSKNFHLRPRDLISNINVIVLELKGSETTFMCEYAD ETATIVEFLNRWITFAQSIISTLTGGGGSGGGGSGGGG SGGGGSGGGGSDIQLTQSPSTLSASVGDRVTISCRAS RAVSPWLAWYQHRPGQAPKLLIYAASSLQSGVPSRF SGSGSGTDFTLTISSLQPEDFATYYCQQYRSYPFTFGP GTTVDIRRTVAAPSVFIFPPSDEQLKSGTASVVCLLN NFYPREAKVQWKVDNALQSGNSQESVTEQDSKDST YSLSSTLTLSKADYEKHKVYACEVTHQGLSSPVTKS FNRGEC | SEQ ID No: 180 |

The above 12 fusion proteins were expressed and purified according to the method of Example 2. The expression level and purity in SEC-HPLC are shown in Table 11 below.

**Table 11. Expression level and purity of fusion proteins**

| Antibody No. | Expression level (mg/L) | Purity (%) |
|---|---|---|
| A2-22-a | 39 | 90.780 |
| A2-22-b | 104 | 96.308 |
| A3-21-a | 82 | 89.358 |
| A3-21-b | 2 | 82.095 |
| B2-15-a | 89 | 53.821 |
| B2-15-b | 70 | 99.839 |
| C2-53-a | N.A. | 54.940 |
| C2-53-b | 6 | 26.598 |
| D2-60-a | 2.4 | 40.248 |
| D2-60-b | 129 | 97.540 |
| D3-68-a | 0.3 | N.A. |
| D3-68-b | 106 | 100 |

The results show that for different anti-IL-2 antibodies, IL-2 conjugated at the N-terminus of the heavy or light chain greatly affected the expression level and purity of the fusion protein. The low expression level or purity suggests that IL-2 and antibody may not form normal intramolecular binding but intermolecular binding in the fusion protein, thereby causing aggregation of the protein. The fusion proteins with higher expression levels (at least 20 mg/L) and purity (at least 90%) were picked for subsequent identification.

### Example 9. Octet Assay on Binding of Fusion Proteins to IL-2Rα and IL-2Rβ

A Protein A biosensor (Fortebio, #18-5010) was immersed in 200 µL of KB buffer (PBS, pH 7.4, 0.02% tween-20, 0.1% BSA) for 60 s for the wetting treatment. Then, the fusion protein was diluted to 10 µg/mL with the KB buffer, and the sensor was immersed in 200 µL of the solution until the reading was 1.2 nm. The sensor was immersed in the KB buffer for 100 s to elute excess antibody-IL-2 fusion protein. IL-2Rα (ILA-H52H9, Acrobiosystem) was diluted in a 2-fold gradient to 100 nM-3.125 nM with the KB buffer. The sensor was immersed in the solution for 60 s for association. The sensor was immersed in the KB buffer for 60 s for dissociation. The data were fitted in a dynamic 1:1 binding mode. The affinity of fusion proteins for IL-2Rα is shown in Table 12. The results show that IL-2 conjugated with anti-IL-2 antibodies had reduced binding to IL-2Rα as compared to IL-2, among which A3-21-a and D3-68-b did not bind to IL-2Rα at all.

**Table 12. Affinity of fusion proteins for IL-2Rα**

| Test sample | Kon (1/Ms) | Koff (1/s) | KD (M) |
|---|---|---|---|
| IL-2 | 1.40E+06 | 2.51E-02 | 1.80E-08 |
| A2-22-a | 9.22E+05 | 3.75E-02 | 4.06E-08 |
| A2-22-b | 1.63E+06 | 4.13E-02 | 2.53E-08 |
| A3-21-a | N.A. | N.A. | No binding |
| B2-15-b | 1.23E+06 | 3.30E-02 | 2.68E-08 |
| D2-60-b | 1.24E+06 | 5.61E-02 | 4.51E-08 |
| D3-68-b | N.A. | N.A. | No binding |

| | | | |
|---|---|---|---|
| (Note: N.A.: no binding, so no numerical values can be obtained by fitting. In the assay for the affinity between IL-2 and IL-2Rα, IL-2Rα was used as a stationary phase, and IL-2 was used as a mobile phase.) | | | |

Similar to the above methods, the data were fitted in a steady-state mode by Octet. The affinity of antibody-IL-2 fusion proteins for IL-2Rβ (CD2-H5221, Acrobiosystem) determined is shown in Table 13. The antibody fusion protein was used as a stationary phase, and the IL-2Rβ was used as a mobile phase. The results show that most of IL-2 conjugated with anti-IL-2 antibodies did not bind to IL-2Rβ at all as compared to IL-2, and individual antibody-IL2 fusion proteins, such as D3-68-b, were weakened in binding to IL-2Rβ, but still had a certain degree of binding. In an assay on affinity of IL-2 for IL-2Rβ, IL-2Rβ was used as a stationary phase, and IL-2 was used as a mobile phase.

**Table 13. Affinity of fusion proteins of anti-IL-2 antibody and IL-2 for IL-2Rβ**

| Test sample | KD (nM) |
|---|---|
| IL-2 | 4.6E-07 |
| A2-22-a | No binding |
| A2-22-b | No binding |
| A3-21-a | No binding |
| B2-15-b | No binding |
| D2-60-b | No binding |
| D3-68-b | 1.1E-06 |

### Example 10. Determination of Activity of Fusion Proteins for STATS Phosphorylation in Human Peripheral Blood (PBMC)

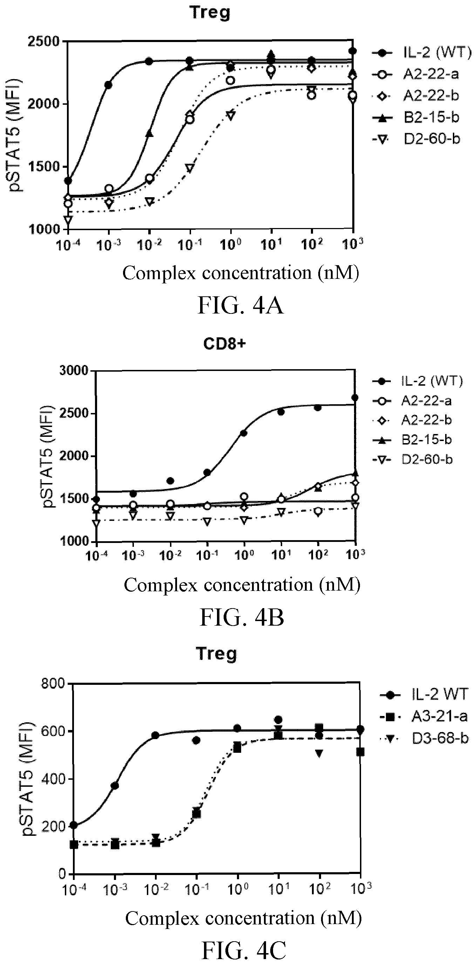

The activity of fusion proteins for STATS phosphorylation in Tregs and CD8⁺ T cells in human peripheral blood (PBMC) was determined according to the method of Example 7. As shown in FIGs. 4A-4D, the experimental results show that all fusion proteins had reduced activity for Tregs and CD8⁺ T cells as compared to IL-2. However, for A2-22-a, A2-22-b, B2-15-b, D2-60-b and A3-21-b, the activity of those fusion proteins for CD8⁺ T cells was reduced more than that for Tregs, suggesting that IL-2 in those fusion proteins may be more inclined to activate Tregs.

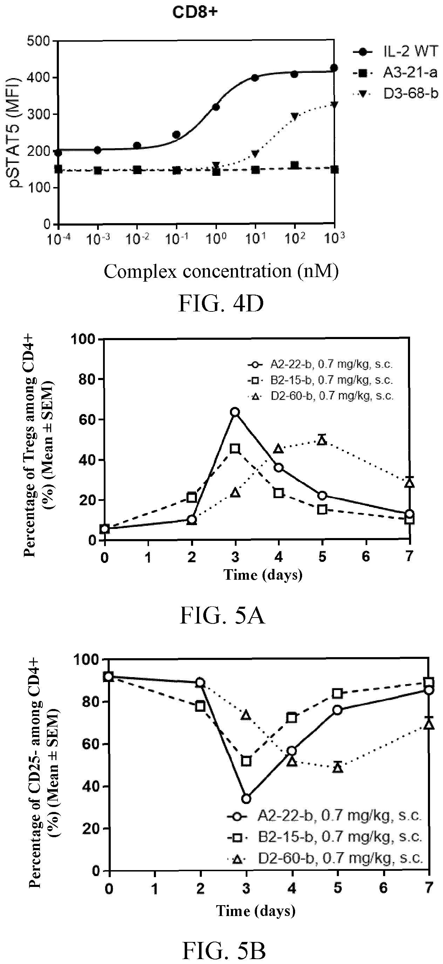

However, for D3-68-b, its activity for Tregs was reduced more than that for CD8⁺ T cells (shown in FIGs. 4C and 4D). EC50 values for fusion proteins activating pSTAT5 in Tregs and CD8⁺ T cells are shown in Tables 14 and 15.

**Table 14. EC₅₀ values for fusion proteins activating pSTAT5 in Treg cells**

| Test sample | EC₅₀ (nM) |
|---|---|
| IL-2 | 0.0003714 |
| A2-22-a | 0.04439 |
| A2-22-b | 0.05677 |
| B2-15-b | 0.01072 |
| D2-60-b | 0.1940 |
| D3-68-b | 0.1731 |
| A3-21-a | 0.1923 |

**Table 15. EC₅₀ values for fusion proteins activating pSTAT5 in CD8⁺ T cells**

| Test sample | EC₅₀ (nM) |
|---|---|
| IL-2 | 0.4343 |
| A2-22-a | N.A. |
| A2-22-b | N.A. |
| B2-15-b | N.A. |
| D2-60-b | N.A. |
| D3-68-b | 31.06 |
| A3-21-a | N.A. |

| | |
|---|---|
| (Note: N.A.: values at the highest test concentration were still low, and accurate EC₅₀ values could not be obtained by fitting). | |

### Example 11. Determination of Effect of Fusion Proteins on Immune Cells in Peripheral Blood of Balb/c Mice

Female BALB/c mice (purchased from Charles River Laboratories, Beijing) aged 6-8 weeks and weighing 18-20 g were subjected to adaptive feeding for 5 days before the experiment. All BALB/C mice were kept in an IVC constant temperature and pressure system in an SPF-grade animal room at a temperature of 20-26 °C with humidity at 40-70%, with a 12/12 hour light/dark cycle. No more than 6 BALB/c mice were kept in each cage. Mice were grouped according to body weight, and administration was performed after the grouping. The type of drug administered, the dose of administration and the route of administration are shown in Table 16. The day of model grouping was day 0.

**Table 16. Administration regimen for mice**

| Group | N | Treatment | Dose (mg/kg) | Route of administration | Administration time |
|---|---|---|---|---|---|
| 1 | 3 | A2-22-b | 0.7 | Subcutaneously | Single |
| 2 | 3 | B2-15-b | 0.7 | Subcutaneously | Single |
| 3 | 3 | D2-60-b | 0.7 | Subcutaneously | Single |

Freshly collected anticoagulated blood at each time point was lysed with red blood cell lysis buffer and washed with PBS once. A mixed staining solution was prepared with PBS containing 1% FBS. The mixed staining solution comprises CD3 APC-Cy7 (Biolegend 100329), CD8 PE (Biolegend 100708), CD4 PE-Cy7 (eBioscience 25-0042-82) and CD25 PerCP-Cy5.5 (BD 561112). 100 µL of mixed staining solution was added to each sample, and the mixture was incubated at 4 °C for 30 min. The mixture was washed with PBS containing 1% FBS twice. Fixation and membrane rupture were performed using True-Nuclear^{™} Transcription Factor Buffer Set (Biolegend 424401) for 60 min, and 100 µL of anti-mouse Foxp3 antibody (Biolegend 126405) and anti-mouse Ki67 antibody (eBioscience, 25-5698-82) were incubated at room temperature for 60 min. The mixture was washed with PBS (pH 7.4) twice, finally resuspended in 500 µL of PBS washing buffer at pH 7.4, and analyzed on an instrument. CD8⁺ T cells were defined as CD3⁺CD4⁻CD8⁺ cells, and Tregs were defined as CD3⁺CD4⁺CD25⁺Foxp3⁺ cells.

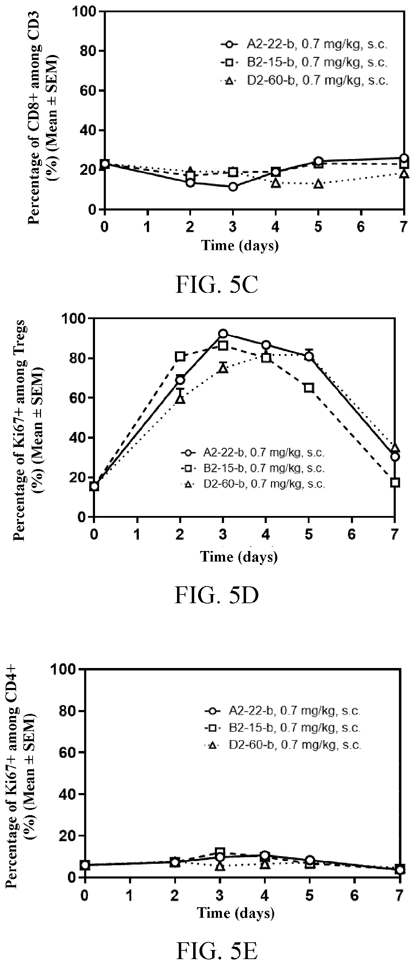

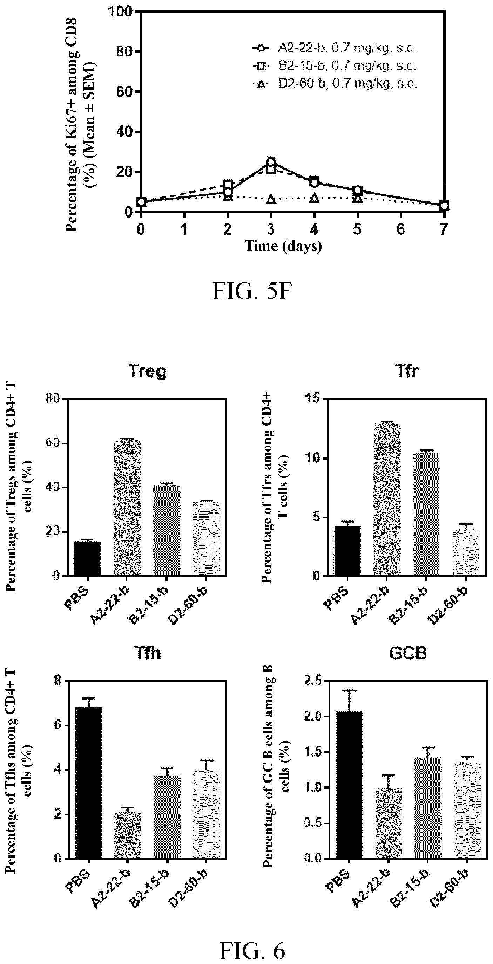

The experimental results are shown in FIGs. 5A-5F. For A2-22-b, B2-15-b, and D2-60-b, in whole blood of mice, the percentage of Tregs to CD4⁺ T cells was increased from Day 2, and the percentage of Ki67+ among Tregs was also increased significantly from Day 2 as compared to Day 0, indicating that A2-22-b, B2-15-b, and D2-60-b can strongly stimulate the proliferation of Tregs. In contrast, the percentage of Ki67⁺ to CD4⁺CD25⁻ T cells and CD8⁺ T cells showed little or no change, and the percentage of CD8⁺ T cells to CD3⁺ T cells also showed little change, indicating that A2-22-b, B2-15-b and D2-60-b hardly activate CD8⁺ T cells.

### Example 12. Determination of Effect of Fusion Proteins on Immune Cells in Spleen of Balb/c Mice Immunized with Chicken Ovalbumin (OVA)

Chicken ovalbumin (OVA, Sigma A5503) was dissolved in PBS to obtain a solution at 0.5 mg/mL. Freund's complete adjuvant (CFA, containing 1 mg/mL inactivated *Mycobacterium tuberculosis,* Sigma, F5881). Male C57B16/J mice (purchased from Shanghai Laboratory Animal Center) aged 6-8 weeks and weighing 18-20 g. Mice were grouped according to body weight. On day 1 of the experiment, 8 mL of OVA solution was taken, mixed well with 8 mL of CFA, and emulsified to form a water-in-oil solution. Each animal was immunized by intraperitoneal injection of 200 µL of the solution. The mice in all groups were each injected subcutaneously (sc) with 10 mL/kg test fusion protein or PBS control on Days 3 and 8. The type of drug administered, the dose of administration and the route of administration are shown in Table 17.

**Table 17. Immunization regimen for mice**

| Group No. | Number | Immunization | Test sample | Route of administration | Dose (mpk) | Administration time |
|---|---|---|---|---|---|---|
| 1 | 4 | OVA(50µg) | PBS | Subcutaneously | NA | Days 3 and 8 |
| 2 | 4 | OVA(50µg) | A2-22-b | Subcutaneously | 0.7 | Days 3 and 8 |
| 3 | 4 | OVA(50µg) | B2-15-b | Subcutaneously | 0.7 | Days 3 and 8 |
| 4 | 4 | OVA(50µg) | D2-60-b | Subcutaneously | 0.7 | Days 3 and 8 |

On Day 10, mice were euthanized, and spleens were taken and processed for detection of relevant immune cells by flow cytometry. The procedures were as follows: after being immersed in 75% ethanol for 5 min, the spleen of the mouse was taken out under the aseptic condition, rinsed 1-2 times with PBS, and then cut into pieces. The filter mesh was placed in a 50 mL centrifuge tube, and spleen tissue pieces were transferred to the mesh and ground while fresh PBS was added continuously. The suspension after grinding was centrifuged at 1800 rpm for 3 min, and the supernatant was discarded. 10 mL of red blood cell lysis buffer was added, the mixture was left to stand on ice for 5 min before adding PBS to terminate the lysis. The mixture was centrifuged at 1800 rpm for 3 min twice. After staining according to the BioLegend flow cytometry antibody staining process, cells were resuspended in PBS twice, sieved, and detected by flow cytometry.

The detected cells and their markers were as follows:
splenic germinal center B cells (GCBs) (Fas⁺GL-7⁺B220⁺);
follicular T helper cells (Tfhs) (PD1-high, CXCR5-high, FOXP3⁻CD4⁺);
follicular regulatory T cells (Tfrs) (PD1-high, CXCR5-high, FOXP3⁺CD4⁺);
regulatory T cells (Tregs) (FOXP3⁺CD25⁺CD4⁺CXCR5-low);

The experimental results are shown in FIG. 6. A2-22-b, B2-15-b and D2-60-b all could stimulate the proliferation of Tregs and Tfrs in the spleen and inhibit the number of Tfhs and GCBs in the spleen, indicating that A2-22-b, B2-15-b and D2-60-b have the function of inhibiting the immune system by activating Tregs.

### Example 13. Experiment on Efficacy of Fusion Proteins in Delayed Hypersensitivity Mouse Model

The experimental procedures were as follows: 2,4-dinitrofluorobenzene (DNFB, Sigma, 42085-50G) was dissolved with a mixture of acetone and olive oil (4: 1) to obtain a 1% (1 g/100 mL) solution for later use, which was then diluted to a 0.5% (0.5 g/100 mL) solution for later use. Male ICR mice (purchased from Shanghai Laboratory Animal Center) aged 6 weeks and weighing 18-20 g. Mice were grouped according to body weight. On Day 0 of the experiment, the mice were immunized by applying 50 µL of 1% DNFB solution to the abdomen; on Day 5 of the experiment, the mice were challenged by applying 10 µL of 0.5% DNFB solution (20 µL in total) to the inside and outside of the right ear; and on Day 6 of the experiment, the left and right ear tissues were separately removed by using an 8 mm puncher and weighed, and the weight difference between left and right ear tissues was calculated. The test drug was subcutaneously administered once every 5 days from 2 days before the start of the experiment (Day -2).

The type of drug administered, the dose of administration and the route of administration are shown in Table 18.

**Table 18. Immunization regimen for mice**

| Group No. | Number | Test sample | Dose (mpk) | Route of administration | Administration time |
|---|---|---|---|---|---|
| 1 | 5 | PBS | NA | Subcutaneously | Days -2 and 3 |
| 2 | 5 | A2-22-b | 1 | Subcutaneously | Days -2 and 3 |
| 3 | 5 | A2-22-b | 0.3 | Subcutaneously | Days -2 and 3 |
| 4 | 5 | A2-22-b | 0.1 | Subcutaneously | Days -2 and 3 |
| 5 | 5 | B2-15-b | 1 | Subcutaneously | Days -2 and 3 |
| 6 | 5 | B2-15-b | 0.3 | Subcutaneously | Days -2 and 3 |
| 7 | 5 | B2-15-b | 0.1 | Subcutaneously | Days -2 and 3 |
| 8 | 5 | D2-60-b | 1 | Subcutaneously | Days -2 and 3 |
| 9 | 5 | D2-60-b | 0.3 | Subcutaneously | Days -2 and 3 |
| 10 | 5 | D2-60-b | 0.1 | Subcutaneously | Days -2 and 3 |

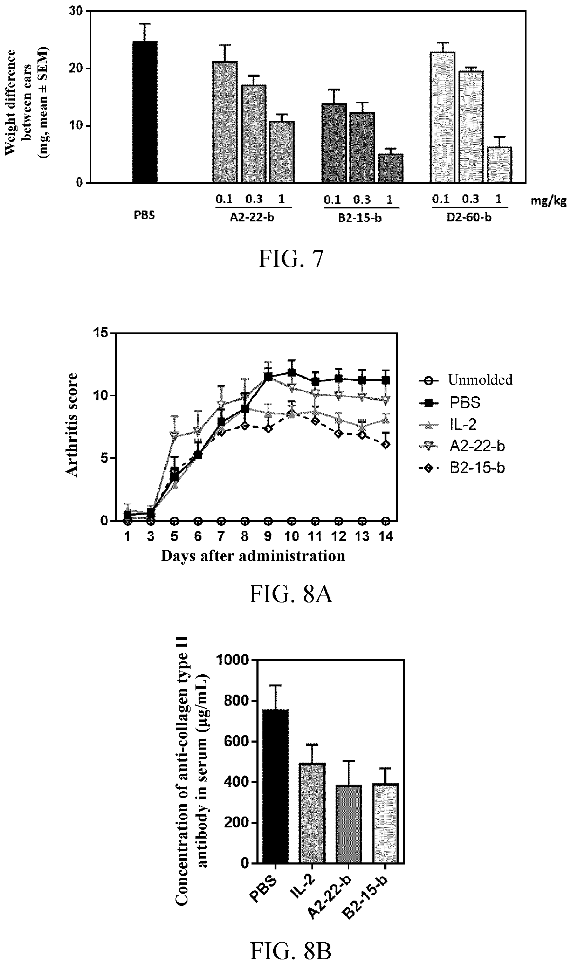

The experimental results are shown in FIG. 7. A2-22-b, B2-15-b and D2-60-b all could reduce the degree of ear swelling and show a dose-response relationship, indicating that A2-22-b, B2-15-b and D2-60-b have the function of inhibiting the immune system.

### Example 14. Experiment on Efficacy of Fusion Proteins in Arthritis Mouse Model

Male DBA/1 inbred mice were purchased from Shanghai SLAC Laboratory Animal Co., Ltd. The arthritis model was induced by immunization with bovine type II collagen (Biolead, 20022). On Day 0 of the experiment, 2.5 mL of bovine type II collagen/glacial acetic acid (2 mg/mL) was taken and mixed well with 2.5 mL of Freund's complete adjuvant to form a water-in-oil emulsion. 0.1 mL of the emulsion was injected intradermally into the tail root of each mouse under light ether anesthesia, followed by one booster injection 3 weeks later. The molded animals were randomly divided into 6 groups: a normal control group, a model control group, an IL-2 (C013, novoprotein) group, an A2-22-b group, and a B2-15-b group, with 8 animals in each group. Meanwhile, normal control animals were included in the normal control group. The groups are shown in Table 19.

**Table 19. Immunization regimen for mice**

| Group No. | Group | Number of animals | Dose (mg/kg) | Frequency of administration | Route of administration | Volume (mL/kg) |
|---|---|---|---|---|---|---|
| 1 | Normal control group | 8 | - | Q5D × 14 days | i.p. | - |
| 2 | Model control group | 8 | - | Q5D × 14 days | i.p. | - |
| 3 | IL-2 group | 8 | 100000IU | Q2D × 14 days | S. C. | 5 |
| 4 | A2-22-b group | 8 | 0.7 | Q5D × 14 days | i.p. | 5 |
| 5 | B2-15-b group | 8 | 0.7 | Q5D × 14 days | i.p. | 5 |

| | | | | | | |
|---|---|---|---|---|---|---|
| (Note: Q5D, once every five days; Q2D, once every two days; i.p., intraperitoneal administration; s.c., subcutaneous administration.) | | | | | | |

Evaluation of joint inflammation: mice were weighed and scored semi-quantitatively (clinical scores) for the severity of joint inflammation every 3 days starting from secondary immunization:
0 points: no redness and swelling;
1 point: inflammation or redness and swelling at one place on the foot;
2 points: mild inflammation on the foot or redness and swelling at two or more places on the whole foot;
3 points: moderate redness and swelling on the whole foot; and
4 points: severe redness and swelling, even joint stiffness, deformity and mobility disorder on the whole foot.

The maximum score is 4 points per foot and 16 points per mouse. The observation of the severity of joint inflammation continued until the end of the experiment.

Measurement of concentration of anti-bovine type II collagen antibody in serum: at the end of the experiment, blood was collected, and serum was separated and stored at -80 °C. The level of the anti-bovine type II collagen antibody in serum was measured using an ELISA detection kit (Chondrex, 20322T). The specific steps were carried out according to the instructions.

The experimental results are shown in FIGs. 8A and 8B. The weight of the mice in the model control group was significantly reduced and the arthritis score was significantly increased (p < 0.001) as compared to that in the normal control group; and B2-15-b could significantly inhibit the weight loss of arthritis model mice and significantly reduce the arthritis score (p < 0.05) as compared to the model control group. The concentration of the anti-bovine type II collagen IgG2a antibody in serum of mice in the model control group was significantly increased (p < 0.001) compared with that in the normal control group; and the administration of B2-15-b administration could significantly reduce the secretion of the anti-bovine type II collagen IgG2a antibody in serum of arthritis mice (p < 0.01) as compared to the model control group.

### Example 15: Effect of Different Lengths of Linker Arms on Expression Level and Purity of Fusion Protein

The (G₄S)₅ linker arm between IL-2 and the light chain of anti-IL-2 antibody B2-15 in B2-15-b was shortened to 4 repeats of G₄S while the heavy chain remained unchanged, so as to obtain a variant B2-15-b-(G₄S)₄ of B2-15-b. The light chain sequence of the variant is shown in Table 20.

**Table 20. Full-length light chain sequence of the antibody in the fusion protein**

| Fusion protein No. | Full-length light chain amino acid sequence | | Sequence No. |
|---|---|---|---|
| B2-15-b-(G ₄S)₄ | LC | APASSSTKKTQLQLEHLLLDLQMILNGINNYKNP KLTRMLTFKFYMPKKATELKHLQCLEEELKPLEE VLNLAQSKNFHLRPRDLISNINVIVLELKGSETTF MCEYADETATIVEFLNRWITFAQSIISTLTGGGGS GGGGSGGGGSGGGGSEIVLTQSPGTLSLSPGERA TLSCRASQSVSSSYLAWYQQKPGQAPRLLIYGA SNRATGIPDRFSGSGSGADFTLTISRLEPEDFAVY YCQQYGSSPLTFGGGTKVEIKRTVAAPSVFIFPPS DEQLKSGTASVVCLLNNFYPREAKVQWKVDNA LQSGNSQESVTEQDSKDSTYSLSSTLTLSKADYE KHKVYACEVTHQGLSSPVTKSFNRGEC | SEQ ID No: 181 |

After the variant of B2-15-b described above was expressed and purified according to the method of Example 2, the expression level could still reach 47 mg/L, and the purity was 99% by SEC-HPLC, indicating that different lengths of linker arms all can achieve the expression of the fusion protein.

### Example 16. Determination of Activity of Fusion Proteins of Anti-IL-2 Antibody and Different IL-2 Variants for STATS Phosphorylation in Human Peripheral Blood (PBMC)

IL-2 was mutated to introduce T3E or T3Q, or to remove the first 3 amino acids from the N-terminus of IL-2 (B2-15-b-Del3), or to remove the first 7 amino acids from the N-terminus of IL-2 (B2-15-b-Del7). Alternatively, wild-type IL-2 was directly conjugated with anti-IL-2 antibodies. The following fusion proteins were produced (the heavy chain sequence of each antibody was kept constant), shown in Table 21.

**Table 21. Full-length light chain sequences of antibodies in fusion proteins**

| Fusion protein No. | Full-length light chain amino acid sequence | | Sequence No. |
|---|---|---|---|
| B2-15-b-T3E | LC | APESSSTKKTQLQLEHLLLDLQMILNGINNYKNPKLT RMLTFKFYMPKKATELKHLQCLEEELKPLEEVLNLA QSKNFHLRPRDLISNINVIVLELKGSETTFMCEYADET ATIVEFLNRWITFAQSIISTLTGGGGSGGGGSGGGGSG GGGSGGGGSEIVLTQSPGTLSLSPGERATLSCRASQS VSSSYLAWYQQKPGQAPRLLIYGASNRATGIPDRFSG SGSGADFTLTISRLEPEDFAVYYCQQYGSSPLTFGGGT KVEIKRTVAAPSVFIFPPSDEQLKSGTASVVCLLNNFY PREAKVQWKVDNALQSGNSQESVTEQDSKDSTYSL | SEQ ID No: 182 |
| | | SSTLTLSKADYEKHKVYACEVTHQGLSSPVTKSFNR GEC | |
| B2-15-b-WT (wild-ty pe IL-2) | LC | APTSSSTKKTQLQLEHLLLDLQMILNGINNYKNPKLT RMLTFKFYMPKKATELKHLQCLEEELKPLEEVLNLA QSKNFHLRPRDLISNINVIVLELKGSETTFMCEYADET ATIVEFLNRWITFAQSIISTLTGGGGSGGGGSGGGGSG GGGSGGGGSEIVLTQSPGTLSLSPGERATLSCRASQS VSSSYLAWYQQKPGQAPRLLIYGASNRATGIPDRFSG SGSGADFTLTISRLEPEDFAVYYCQQYGSSPLTFGGGT KVEIKRTVAAPSVFIFPPSDEQLKSGTASVVCLLNNFY PREAKVQWKVDNALQSGNSQESVTEQDSKDSTYSL SSTLTLSKADYEKHKVYACEVTHQGLSSPVTKSFNR GEC | SEQ ID No: 183 |
| B2-15-b-T3Q | LC | APQSSSTKKTQLQLEHLLLDLQMILNGINNYKNPKLT RMLTFKFYMPKKATELKHLQCLEEELKPLEEVLNLA QSKNFHLRPRDLISNINVIVLELKGSETTFMCEYADET ATIVEFLNRWITFAQSIISTLTGGGGSGGGGSGGGGSG GGGSGGGGSEIVLTQSPGTLSLSPGERATLSCRASQS VSSSYLAWYQQKPGQAPRLLIYGASNRATGIPDRFSG SGSGADFTLTISRLEPEDFAVYYCQQYGSSPLTFGGGT KVEIKRTVAAPSVFIFPPSDEQLKSGTASVVCLLNNFY PREAKVQWKVDNALQSGNSQESVTEQDSKDSTYSL SSTLTLSKADYEKHKVYACEVTHQGLSSPVTKSFNR GEC | SEQ ID No: 184 |
| B2-15-b-Del3 | LC | SSSTKKTQLQLEHLLLDLQMILNGINNYKNPKLTRM LTFKFYMPKKATELKHLQCLEEELKPLEEVLNLAQS KNFHLRPRDLISNINVIVLELKGSETTFMCEYADETAT IVEFLNRWITFAQSIISTLTGGGGSGGGGSGGGGSGG GGSGGGGSEIVLTQSPGTLSLSPGERATLSCRASQSVS SSYLAWYQQKPGQAPRLLIYGASNRATGIPDRFSGSG SGADFTLTISRLEPEDFAVYYCQQYGSSPLTFGGGTK VEIKRTVAAPSVFIFPPSDEQLKSGTASVVCLLNNFYP REAKVQWKVDNALQSGNSQESVTEQDSKDSTYSLS STLTLSKADYEKHKVYACEVTHQGLSSPVTKSFNRG EC | SEQ ID No: 185 |
| B2-15-b-Del7 | LC | KKTQLQLEHLLLDLQMILNGINNYKNPKLTRMLTFK FYMPKKATELKHLQCLEEELKPLEEVLNLAQSKNFH LRPRDLISNINVIVLELKGSETTFMCEYADETATIVEF LNRWITFAQSIISTLTGGGGSGGGGSGGGGSGGGGSG GGGSEIVLTQSPGTLSLSPGERATLSCRASQSVSSSYL AWYQQKPGQAPRLLIYGASNRATGIPDRFSGSGSGA DFTLTISRLEPEDFAVYYCQQYGSSPLTFGGGTKVEIK RTVAAPSVFIFPPSDEQLKSGTASVVCLLNNFYPREA KVQWKVDNALQSGNSQESVTEQDSKDSTYSLSSTLT LSKADYEKHKVYACEVTHQGLSSPVTKSFNRGEC | SEQ ID No: 186 |
| A2-22-b -Del3 | LC | SSSTKKTQLQLEHLLLDLQMILNGINNYKNPKLTRM LTFKFYMPKKATELKHLQCLEEELKPLEEVLNLAQS KNFHLRPRDLISNINVIVLELKGSETTFMCEYADETAT IVEFLNRWITFAQSIISTLTGGGGSGGGGSGGGGSGG GGSGGGGSEIVLTQSPGTLSLSPGERATLSCRASQSVS | SEQ ID No: 187 |
| | | NNYLAWYQQKPGQAPRLLIYGASSRATGIPDRFSGS GSGTDFTLTISRLEPEDFAVYSCQQYGSSPLTFGQGTR LEIKRTVAAPSVFIFPPSDEQLKSGTASVVCLLNNFYP REAKVQWKVDNALQSGNSQESVTEQDSKDSTYSLS STLTLSKADYEKHKVYACEVTHQGLSSPVTKSFNRG EC | |
| D2-60-b -Del3 | LC | SSSTKKTQLQLEHLLLDLQMILNGINNYKNPKLTRM LTFKFYMPKKATELKHLQCLEEELKPLEEVLNLAQS KNFHLRPRDLISNINVIVLELKGSETTFMCEYADETAT IVEFLNRWITFAQSIISTLTGGGGSGGGGSGGGGSGG GGSGGGGSDIQMTQSPSSLSASVGDRVTITCRASQGI SNYLAWYQQKPGKVPKLLIYAASTLQSGVPSRFSGS GSGTDFTLTISSLQPEDVATYYCQKYDSAPFTFGQGT RLEIKRTVAAPSVFIFPPSDEQLKSGTASVVCLLNNFY PREAKVQWKVDNALQSGNSQESVTEQDSKDSTYSL SSTLTLSKADYEKHKVYACEVTHQGLSSPVTKSFNR GEC | SEQ ID No: 188 |
| D2-60-b -T3E | LC | APESSSTKKTQLQLEHLLLDLQMILNGINNYKNPKLT RMLTFKFYMPKKATELKHLQCLEEELKPLEEVLNLA QSKNFHLRPRDLISNINVIVLELKGSETTFMCEYADET ATIVEFLNRWITFAQSIISTLTGGGGSGGGGSGGGGSG GGGSGGGGSDIQMTQSPSSLSASVGDRVTITCRASQ GISNYLAWYQQKPGKVPKLLIYAASTLQSGVPSRFSG SGSGTDFTLTISSLQPEDVATYYCQKYDSAPFTFGQG TRLEIKRTVAAPSVFIFPPSDEQLKSGTASVVCLLNNF YPREAKVQWKVDNALQSGNSQESVTEQDSKDSTYS LSSTLTLSKADYEKHKVYACEVTHQGLSSPVTKSFN RGEC | SEQ ID NO: 189 |

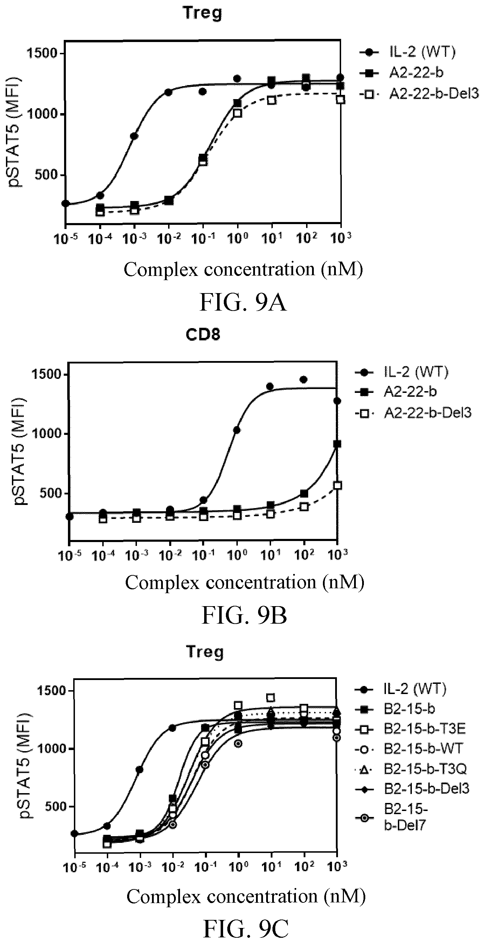

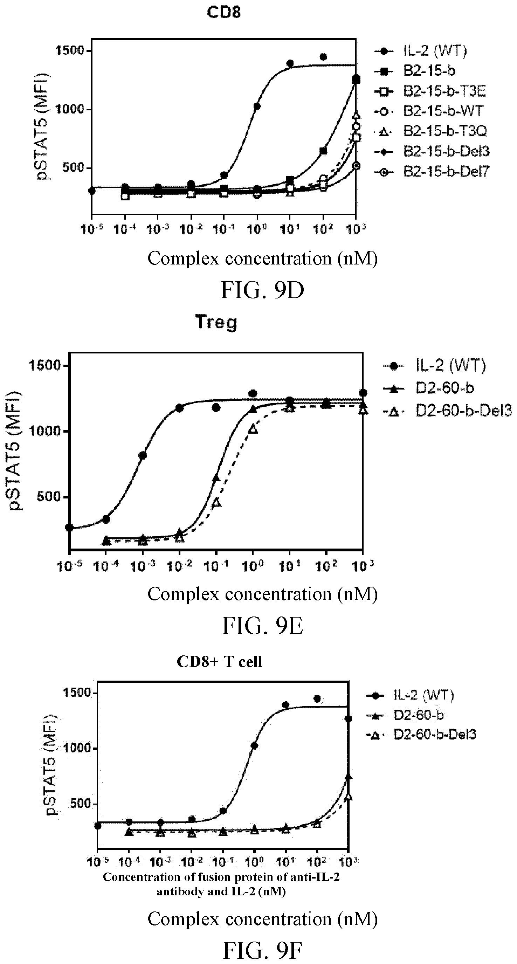

The activity of the fusion proteins of anti-IL-2 antibody and IL-2 variants described above for STATS phosphorylation in Tregs and CD8⁺ T cells in human peripheral blood (PBMC) was determined according to the method of Example 7. As shown in FIGs. 9A-9F and Table 22, the experimental results show that those IL-2 variants had little effect on the activity of the antibody fusion proteins for Tregs and CD8⁺ T cells. Those fusion proteins were still more inclined to activate Tregs instead of CD8⁺ T cells.

**Table 22. EC₅₀ values for fusion proteins of anti-IL-2 antibody and different IL-2 variants activating pSTAT5 in Treg cells**

| Fusion protein No. | EC₅₀ (nM) |
|---|---|
| A2-22-b | 0.168 |
| A2-22-b-Del3 | 0.144 |
| B2-15-b | 0.016 |
| B2-15-b-T3E | 0.031 |
| B2-15-b-WT | 0.041 |
| B2-15-b-T3Q | 0.029 |
| B2-15-b-Del3 | 0.038 |
| B2-15-b-Del7 | 0.055 |
| D2-60-b | 0.114 |
| D2-60-b-Del3 | 0.229 |
| D2-60-b-T3E | 0.168 |

### Example 17. Experiment on Efficacy of Fusion Proteins in MRL/lpr Spontaneous Lupus Erythematosus Mouse Model

Female BALB/c mice and female MRL/lpr mice were purchased from Shanghai SLAC Laboratory Animal Co., Ltd. Female BALB/c mice were included in a normal control group, and female MRL/lpr mice were randomly divided into the following 3 groups according to proteinuria level and body weight: an MRL/lpr control group (model control group), a 1 mg/kg administration group and a 3 mg/kg administration group. The mice in the two administration groups, when aged at 8 weeks, were each subcutaneously injected with D2-60-b-T3E at doses of 1 mg/kg and 3 mg/kg, respectively, once every 3 days for consecutive 8 weeks.

**Table 23. Experimental design and grouping**

| Mice | Group | Dose (mg/kg) | Frequency of administration | Route of administration | Number of animals |
|---|---|---|---|---|---|
| Normal Balb/c | Normal control group | - | Once every 3 days | SC. | 10 |
| MRL/lpr | Model control group | - | Once every 3 days | sc. | 10 |
| | D2-60-b-T3E administration group | 1 | Once every 3 days | sc. | 10 |
| | | 3 | Once every 3 days | sc. | 10 |

During the treatment period, the urine protein content of mice in each group was determined every two weeks; at the end of 8 consecutive weeks of treatment, the following tests were performed: (1) determination of the content of anti-double-stranded DNA IgM and IgG antibodies in the serum of mice in each group; (2) determination of the content of serum creatinine (CRE) and blood urea nitrogen (BUN) in the serum of mice in each group; (3) determination of the content of IL-6, IL-10 and IFN-γ in the serum of mice in each group; and (4) pathological analysis and scoring of renal tissue of mice in each group.

### 1) Determination of the urine protein content of mice

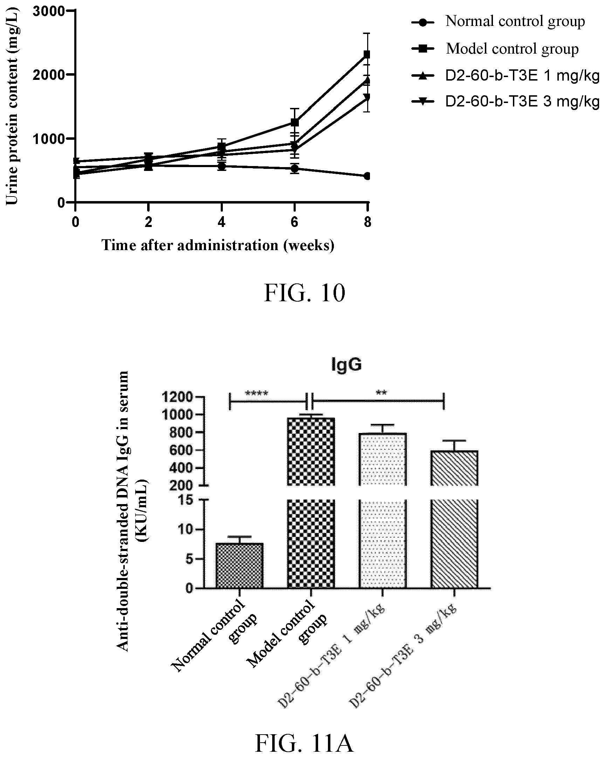

In the experiment, the urine of mice was collected by the tail-lift reflex method, and the urine protein concentration of mice was determined using a urine protein detection kit (CBB method, NanJing JianCheng Bioengineering Institute, #C035-2-1). During the pathogenesis of the lupus erythematosus in MRL/lpr mice, kidney damage occurred due to the deposition of dsDNA antibodies, which in turn led to decreased glomerular filtration function and reabsorption capacity and increased urine protein concentration. As lupus nephritis in MRL/lpr mice progressed, the urine protein level of mice would gradually increase. The determination of the urine protein level of the mice performed every two weeks showed that initially there was no significant difference in the urine protein level among the groups, but as the experiment progressed, the urine protein level of the mice in the MRL/lpr control group (model control group) was gradually increased and significantly higher than that in the normal group, indicating that the MRL/lpr mice suffer from severe spontaneous renal injury due to the deposition of autoantibodies. The average urine protein content of mice in the 1 mg/kg and 3 mg/kg D2-60-b-T3E administration groups was lower than that in the model control group and showed a dose-dependent relationship, but there was no statistical difference between the administration groups and the model control group due to the large individual differences in the data. The data are shown in FIG. 10.

### 2) Determination of the content of anti-double-stranded DNA IgM and IgG antibodies in serum

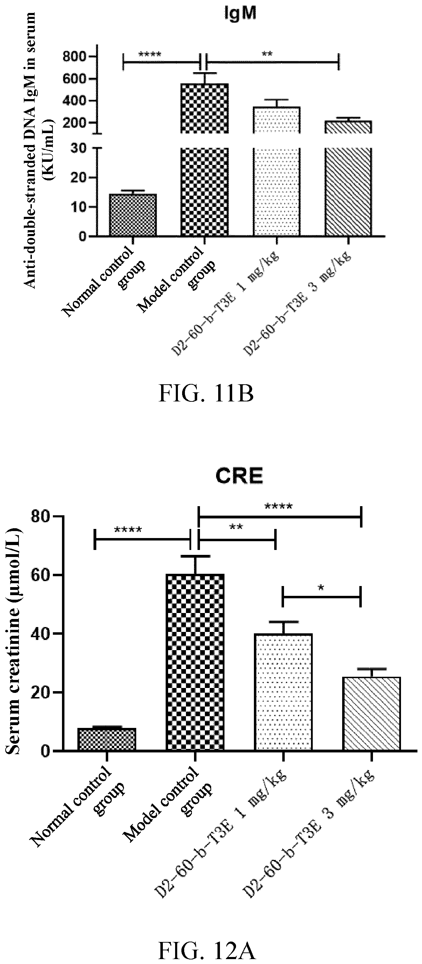

The content of anti-double-stranded DNA (anti-dsDNA) IgG and IgM antibodies in serum was determined by the ELISA method. Refer to the instructions of ELISA kit (Alpha Diagnostic, #5120 and #5130) for specific procedures. The average content of anti-dsDNA IgG and IgM antibodies in the serum of mice in the 3 mg/kg D2-60-b-T3E administration group was significantly lower than that in the model control group (P < 0.01), and there was no statistically significant difference between the 1 mg/kg group and the model control group although the content of IgG and IgM in the 1 mg/kg group tended to be lower than that in the model control group. The experimental results are shown in FIGs. 11A and 11B.

### 3) Effect of D2-60-b-T3E on renal function of MRL/lpr mice

The content of serum creatinine and blood urea nitrogen in the serum of mice at the end of the experiment was detected using a creatinine (CRE) determination kit (NanJing JianCheng Bioengineering Institute, #C011-2-1) and a blood urea nitrogen (BUN) determination kit (NanJing JianCheng Bioengineering Institute, #C013-2-1).

Blood urea nitrogen and creatinine are metabolic products of human proteins and muscles, respectively, and the kidney is the final excretory organ. After the renal function is damaged, blood urea nitrogen and creatinine in the serum cannot be effectively excreted, and the concentration of blood urea nitrogen (BUN) and serum creatinine (CRE) is gradually increased due to retention. Therefore, the levels of BUN and CRE in serum are the major indicators of renal function clinically. The content of serum creatinine (CRE) and blood urea nitrogen (BUN) in the serum of mice in the MRL/lpr control group (model control group) was significantly increased as compared to that of normal mice (P < 0.0001), suggesting that the mice in the MRL/lpr model group have functional renal injury.

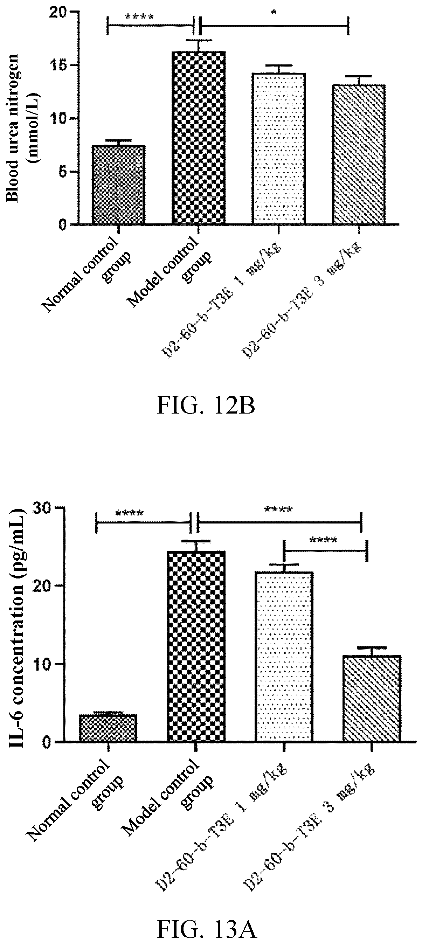

The content of creatinine in the serum of mice in the 1 mg/kg and 3 mg/kg administration groups was significantly lower than that in the model control group (P < 0.01 and P < 0.0001), indicating that the renal function of MRL/lpr mice can be improved to a certain extent. In addition, the content of creatinine in the serum of mice in the 3 mg/kg administration group was significantly lower than that in the 1 mg/kg administration group. The content of blood urea nitrogen in the serum of mice in the 3 mg/kg administration group was also significantly lower than that in the model control group (P < 0.05), indicating that the D2-60-b-T3E at 3 mg/kg can reverse the spontaneous renal injury of MRL/lpr mice. The results are shown in FIGs. 12A and 12B.

### 4) Effect of D2-60-b-T3E on the content of IL-6, IL10 and INF-γ in serum of MRL/lpr mice

The content of the cytokines IFN-γ, IL-6 and IL-10 in the serum was determined by the ELISA method.

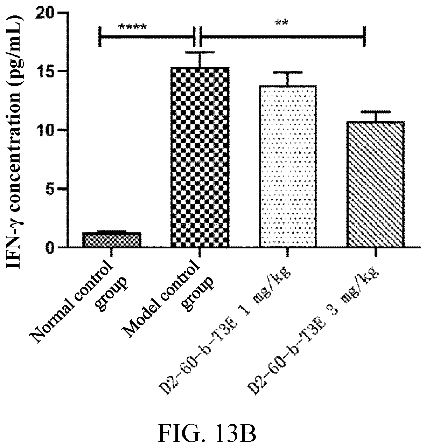

The content of IL-6 and IFN-γ in the serum of mice in the MRL/lpr control group (model group) was significantly increased as compared to that of normal mice (P < 0.0001). The content of IL-6 in the serum of mice in the 3 mg/kg administration group was significantly lower than that in the model control group and the 1 mg/kg administration group (P < 0.0001), and showed a dose-dependent relationship. The content of IFN-γ in the serum of mice in the 3 mg/kg administration group was significantly lower than that in the model control group (P < 0.01). The results are shown in FIGs. 13A and 13B.

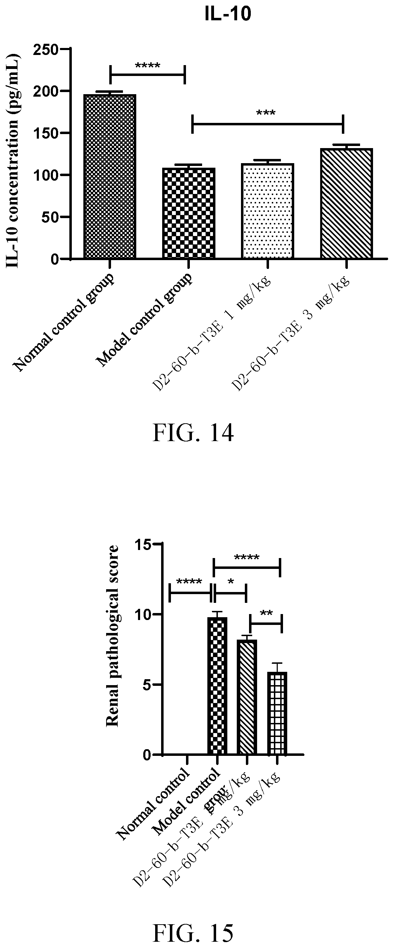

The content of IL-10 in the serum of mice in the MRL/lpr control group (model control group) was significantly decreased as compared to that of normal mice (P < 0.0001). The content of IL-10 in the serum of mice in the 3 mg/kg D2-60-b-T3E administration group was significantly higher than that in a model control group (P < 0.001). The results are shown in FIG. 14.

### 5) Effect of D2-60-b-T3E on renal lesions of MRL/lpr mice

At the end of the experiment, the left kidney of the mouse was fixed in 4% paraformaldehyde, embedded in paraffin, sectioned and then stained by H&E. The section was observed under a microscope and subjected to pathological scoring to grade and score pathological injuries of glomerulus, renal interstitium, blood vessel and the like. The scoring criteria are shown in Table 24.

**Table 24. Renal pathological scoring criteria**

| Score | Glomerulonephritis | Interstitial nephritis | Inflammation of blood vessels |
|---|---|---|---|
| 1+ | Focal, mild or early hyperplasia | 1-3 foci (5-10 cells) monocytes | Monocytes around the renal pelvis/blood vessels |
| 2+ | Multifocal hyperplasia with increased stromal and inflammatory cells | Mild monocyte infiltration around the single tubule; isolated atrophic tubules | Monocyte infiltration around large arteries; monocyte foci of 10-20 cells around interlobular arteries |
| 3+ | Diffuse hyperplasia | More extensive infiltration with large tubular atrophic lesions | More extensive monocyte lesions around small arterial branches |
| 4+ | Extensive sclerosis/crescent shape; proteinuria | Extensive monocyte infiltration between tubules; extensive tubular atrophy necrosis | Monocyte infiltration into the surrounding parenchyma/involvement of most of blood vessels/vasculitis |

The mice in the MRL/lpr control group (model control group) showed severe pathological injuries of glomerulus, renal interstitium and blood vessel in kidney, with glomerular sclerosis and partial inward depression, massive cell infiltration in the perivascular and interstitial regions, and severe glomerular swelling and hyperplasia. The renal pathological scores of the mice in the MRL/lpr control group (model control group) were significantly increased as compared to those of normal mice (P < 0.001), suggesting that the mice in the model control group have significant renal lesions.

The pathological injuries of glomerulus, renal interstitium and blood vessel of the mice in the administration groups were relieved to a certain extent. The renal pathological scores of the mice in the 1 mg/kg and 3 mg/kg administration groups were significantly lower than those of the model control group (P < 0.05 and P < 0.0001), indicating that D2-60-b-T3E can significantly reduce spontaneous renal lesions in MRL/lpr mice. The results are shown in FIG. 15.

### Examples-Formulations

### SEC molecular exclusion chromatography:

This is a method for analyzing the separation of a solute by the relative relationship between the pore size of the gel pores and the size of the polymer sample molecule coil. SEC% (SEC monomer content percentage) = A monomer / A total × 100% (A monomer represents the peak area of the main peak monomer in the sample, and A total represents the sum of all peak areas). ΔSEC% = SEC% of formulation before forced degradation - SEC% of formulation after forced degradation.

Instrument for SEC measurement: Agilent 1260; column: waters, XBrige BEH200Å SEC (300 × 7.8 mm 3.5 µm).

### NR-CE capillary gel electrophoresis:

This is a method of moving the gel into a capillary as a supporting medium for electrophoresis and performing separation according to the molecular weight of the sample under a certain voltage.

NR-CE% = A main peak/A total × 100% (A main peak represents the light chain main peak area + the heavy chain main peak area, and A total represents the sum of all peak areas). ΔNR-CE% = NR-CE% of formulation before forced degradation - NR-CE% of formulation after forced degradation.

Instrument for CE determination: Beckman model plus800

### iCIEF imaged capillary isoelectric focusing electrophoresis:

This is a technique for separation according to the difference of isoelectric points (pI) of proteins.

iCIEF% = neutral peak area/total area × 100% (total area represents the sum of areas of acidic, neutral and basic peaks). ΔiCIEF% = iCIEF% of formulation before forced degradation - iCIEF% of formulation after forced degradation.

Manufacturer of instrument for the iCIEF determination: simple protein, model: muarice.

### Osmotic pressure determination:

The freezing point method is used for determining the osmotic pressure. The freezing point of a solution is determined by using a high-sensitivity temperature-sensing element on the basis of the proportional relation between the freezing point depression value and the molar concentration of the solution, and then converted into the osmotic pressure through electric quantity. Manufacturer of instrument: Loser, model: OM815.

### Fusion protein

The fusion protein used in the following examples was D2-60-b-T3E. The concentration of the fusion protein was calculated based on the protein concentration. Instrument for protein concentration measurement: ultraviolet-visible spectrophotometer; model: Nano Drop oneC; optical path length: 1 mm.

### Formulation Example 1. pH and Buffer System Screening

Formulations containing the buffer systems shown in Table 25, 30 mg/mL fusion protein, 80 mg/mL sucrose, and 0.2 mg/mL polysorbate 80 (PS80) were prepared. A forced degradation study (standing at 40 °C for 1 month and shaking at 25 °C at 300 rpm for 6 days) was performed on samples, and the effect of different buffer systems on the stability of the fusion protein was evaluated using SEC and iCIEF.

The results are shown in Table 25. The SEC data show that after 6 days of shaking, the monomer purities of the formulations containing His-HCl were better than those of the formulations containing AA or SA. The iCIEF data show no significant difference between these groups after shaking. After one month of standing at 40 °C, the formulation containing AA (pH 5.5) and the formulations containing His-HCl (pH 6.0-6.5) were superior to the other groups. In conclusion, the buffer system is preferably AA and His-HCl, and the pH range is preferably 5.5-6.5.

**Table 25. The results of the pH and buffer system screening**

| Group | Buffer system | Conditions | ΔSEC% | ΔiCIEF% |
|---|---|---|---|---|
| 1 | 10mMAApH5.0 | Shaking D6 | 2.3 | 1.6 |
| | | 40°C M1 | 8.9 | 19.6 |
| 2 | 10mMAApH5.5 | Shaking D6 | 3.7 | -0.5 |
| | | 40°C M1 | 7.3 | 15.4 |
| 3 | 10mM SA pH5.5 | Shaking D6 | 5.6 | 0.9 |
| | | 40°C M1 | 8.8 | 21.1 |
| 4 | 10mM His-HCl pH5.5 | Shaking D6 | 1.8 | 1.0 |
| | | 40°C M1 | 8.8 | 17.3 |
| 5 | 10mM His-HCl pH6.0 | Shaking D6 | 1.3 | 0.4 |
| | | 40°C M1 | 7.6 | 14.7 |
| 6 | 10mM His-HCl pH6.5 | Shaking D6 | 1.6 | 0.4 |
| | | 40°C M1 | 6.6 | 15.7 |

| | | | | |
|---|---|---|---|---|
| AA stands for acetic acid-sodium acetate; SA stands for succinic acid-sodium succinate; His-HCl stands for histidine-histidine hydrochloride; Shaking D6: shaking at 25 °C at 300 rpm for 6 days; 40 °C M1: standing at 40 °C for 1 month. The same applies hereinafter. | | | | |

### Formulation Example 2. Protein Concentration Screening

Formulations containing 10 mM AA buffer (pH 5.5), the fusion protein at the protein concentrations shown in Table 26, 80 mg/mL sucrose, and 0.2 mg/mL PS80 were prepared. A forced degradation study (standing at 40 °C for 1 month) was performed on samples, and the effect of different protein concentrations on the stability of the fusion protein was evaluated using SEC, NR-CE, and iCIEF.

The results are shown in Table 26. When the protein concentration was 10-30 mg/mL, there was no significant difference in appearance and purity during standing at high temperature, and the changes in purity were all within an acceptable range. This indicates that within that concentration range, the fusion protein samples have good stability.

**Table 26. The results of the protein concentration screening**

| Group | Concentration (mg/mL) | Conditions | ΔSEC% | ΔNR-CE% | ΔiCIEF% |
|---|---|---|---|---|---|
| 1 | 30 | 40°C M1 | 7.3 | 3.2 | 15.4 |
| 2 | 10 | 40°C M1 | 6.7 | 3.5 | 14.9 |

Formulation Example 3. Formulations Containing Different Sugars

Anti-IL-2 protein formulations containing different sugar species, 10 mg/mL protein, and 0.2 mg/mL PS80 were prepared in 10 mM AA pH 5.5 buffer. The specific sugar species and their concentrations were as follows:
1) 80 mg/mL sucrose
2) 80 mg/mL trehalose

Each of the formulations was filtered and bottled, and the bottles were stoppered and capped. Samples were shaken at 25 °C at 300 rpm, and their stability was evaluated using SEC. The results are shown in Table 27: the formulation containing sucrose was more stable than the formulation containing trehalose.

**Table 27. The results of the sugar screening**

| Sugar | Conditions | ΔSEC% |
|---|---|---|
| | | Monomer |
| 80 mg/mL sucrose | Shaking D9 | 2.1 |
| 80 mg/mL trehalose | Shaking D9 | 4.1 |

### Formulation Example 4. Surfactant Concentration Screening

Formulations containing 30 mg/mL fusion protein, 80 mg/mL sucrose, the surfactants shown in Table 28, and 10 mM AA buffer (pH 5.5) were prepared. Samples were subjected to a forced degradation experiment (standing at 40 °C for 1 month), and the stability of the samples was evaluated using SEC and iCIEF. The results are shown in Table 28. The results show that there was no significant change in the protein monomer purity of the formulations containing poloxamer 188 (PF68).

**Table 28. The results of the surfactant species and concentration screening for the fusion protein**

| Group | Surfactant | Conditions | ΔSEC% | ΔiCIEF% |
|---|---|---|---|---|
| 1 | 0.1 mg/mL PF68 | 40°C M1 | 7.0 | 17.1 |
| 2 | 0.2 mg/mL PF68 | 40°C M1 | 7.4 | 20.9 |
| 3 | 0.4 mg/mL PF68 | 40°C M1 | 6.9 | 19.9 |
| 4 | 0.6 mg/mL PF68 | 40°C M1 | 7.0 | 18.9 |

### Formulation Example 5. Surfactant Screening

Anti-IL-2 protein formulations containing 80 mg/mL sucrose, different surfactant species, and 30 mg/mL protein were prepared with 10 mM pH 6.0 His-HCl as a buffer system. Each of the formulations was filtered and bottled, and the bottles were stoppered and capped. Samples were taken and subjected to a 4 °C long-term study, and the stability of the samples was evaluated according to their appearances and using SEC. The results of the study are shown in Table 29. The specific surfactants used were as follows:
1) 0.6 mg/mL PS80
2) 0.6 mg/mL PF68

The experimental results show that after being stored at 4 °C for 13 months with the same surfactant concentration, there was no significant difference in SEC monomer purity between the PF68 group and the PS80 group. With the same surfactant concentration, the PS80 group was better than the PF68 group in appearance. According to the above data, PS80 is preferred as a surfactant for anti-IL-2 protein formulations.

**Table 29. The results of the surfactant species screening for the anti-IL-2 protein**

| Group | Conditions | Appearance | SEC% | ΔSEC% |
|---|---|---|---|---|
| | | | Monomer | |
| 1 | D0 | Clear and transparent | 97.1 | N/A |
| | 4°C M13 | Clear and transparent | 96.3 | -0.9 |
| 2 | D0 | Clear and transparent | 97.2 | N/A |
| | 4°C M13 | Fine particles | 96.2 | -1.0 |

### Formulation Example 6. Surfactant Concentration Screening

Anti-IL-2 protein formulations containing 87 mg/mL sucrose, PS80 at different concentrations, and 30 mg/mL protein were prepared with 10 mM pH 6.0 His as a buffer system. The specific PS80 concentrations used were as follows:
1) 0.4 mg/mL PS80
2) 0.8 mg/mL PS80
3) 1.5 mg/mL PS80

Each of the formulations was filtered and bottled, and the bottles were stoppered and capped. A freeze-thaw test was performed, and the stability of samples was evaluated according to their appearances and using SEC. The results of the test are shown in Table 30. The MFI results of the freeze-thaw test show that as the concentration of PS80 increases, the number of ≥2 µm particles decreases.

**Table 30. The results of the surfactant concentration screening**

| Group | Conditions | Appearance | MFI(≥2µm) |
|---|---|---|---|
| 1 | FT5C | Clear and transparent | 180 |
| 2 | FT5C | Clear and transparent | 129 |
| 3 | FT5C | Clear and transparent | 100 |

| | | | |
|---|---|---|---|
| FT5C: 5 freeze-thaw cycles, and so forth. | | | |

### Formulation Example 7. Auxiliary Material Screening

Anti-IL-2 protein formulations containing different auxiliary materials, 10 mg/mL protein, and 0.8 mg/mL PS80 were prepared in 10 mM pH 6.0 His-HCl buffer. The specific auxiliary materials were as follows:
1) 36 mg/mL sucrose
2) 36 mg/mL sucrose and 12 mg/mL glycine

Each of the formulations was filtered and bottled, and the bottles were stoppered and capped. Samples were subjected to a freeze-thaw test, and an evaluation was performed according to their appearances and using DLS and MFI. The results are shown in Table 31. The appearance, DLS, and MFI data show no significant difference between these groups. Given subcutaneous formulations, it is best to control the osmotic pressure to make the formulations isotonic so that they are less irritating during injection. Therefore, auxiliary material concentrations were screened to determine the isotonic concentration. Only with 36 mg/mL sucrose was the osmotic pressure relatively low. 36 mg/mL sucrose plus 12 mg/mL glycine or 40 mg/mL sucrose plus 10 mg/mL glycine or 45 mg/mL sucrose plus 10 mg/mL glycine or 50 mg/mL sucrose plus 9 mg/mL glycine can maintain the osmotic pressure within an isotonic range.

**Table 31. The results of the auxiliary material screening**

| Group | Conditions | Appearance | DLS (nm) | MFI (≥2µm) |
|---|---|---|---|---|
| 1 | D0 | Clear and transparent | 13.2 | 124 |
| | FT1C | Clear and transparent | 14.8 | 301 |
| 2 | D0 | Clear and transparent | 13.0 | 126 |
| | FT1C | Clear and transparent | 13.1 | 369 |

### Formulation Example 8. Screening of Different Ionic Strengths

Anti-IL-2 protein formulations containing 36 mg/mL sucrose, 12 mg/mL glycine, 0.8 mg/mL PS80, and 10 mg/mL protein were prepared with pH 6.0 His-HCl as a buffer system. The specific different ionic strengths used were as follows:
1) 10 mM
2) 5 mM

Each of the formulations was filtered and bottled, and the bottles were stoppered and capped. A freeze-thaw test was performed, and the stability of samples was evaluated according to their appearances and using SEC. The results of the test are shown in Table 32. The results show no significant difference in appearance and SEC between these groups.

**Table 32. The results of the screening of different ionic strengths**

| Group | Conditions | Appearance | ΔSEC% |
|---|---|---|---|
| 1 | D0 | Clear and transparent | 96.4 |
| | FT1C | Clear and transparent | 96.5 |
| 2 | D0 | Clear and transparent | 96.4 |
| | FT1C | Clear and transparent | 96.5 |

### Formulation Example 9. Lyophilized Formulation

An anti-IL-2 protein formulation containing 50 mg/mL sucrose plus 9 mg/mL glycine (group 1) or 40 mg/mL sucrose plus 10 mg/mL glycine (group 2), 0.8 mg/mL PS80, and 10 mg/mL protein was prepared with 10 mM pH 6.0 His-HCl as a buffer system.

The formulation was filtered and bottled, and the bottles were stoppered and capped. A sample of the formulation was lyophilized through a lyophilization program including pre-freezing, primary drying, and secondary drying (the parameters are shown in Table 33). After the end of the lyophilization program, stoppering was performed under vacuum. Then the lyophilized formulation was reconstituted with water for injection to form an anti-IL-2 antibody reconstituted solution with a protein concentration of 10 mg/mL. Differences in appearance, NR-CE purity, SEC purity, etc. were examined between the formulation before the lyophilization and the reconstituted lyophilized formulation. The experimental results are shown in Table 34, and the results show that the anti-IL-2 antibody formulation maintained good stability both before and after the lyophilization.

**Table 33. The lyophilization program for the anti-IL-2 antibody formulation**

| Lyophilization process parameters | Set temperature (°C) | Set time (min) | Holding time (min) | Degree of vacuum (Pa) |
|---|---|---|---|---|
| Pre-freezing | 5 | 10 | 60 | N/A |
| Pre-freezing | -45 | 50 | 60 | N/A |
| Primary drying | -25 | 120 | 3000 | 10 |
| Secondary drying | 25 | 60 | 1 | 10 |
| | 25 | 1 | 480 | 10 |

**Table 34. A comparison of the experimental results for the anti-IL-2 antibody formulations before and after lyophilization**

| Group | Standing conditions | Appearance | SEC% | NR-CE% |
|---|---|---|---|---|
| | | | Monomer | Monomer |
| 1 | Formulation before lyophilization | Clear and transparent | 96.8 | 97.2 |
| | Reconstituted lyophilized formulation | Clear and transparent | 96.7 | 97.2 |
| 2 | Formulation before lyophilization | Clear and transparent | N/A | N/A |
| | Reconstituted lyophilized formulation | Clear and transparent | N/A | N/A |

### Formulation Example 10. Lyophilized Formulation

An anti-IL-2 protein formulation containing 45 mg/mL sucrose, 10 mg/mL glycine, 0.8 mg/mL PS80, and 10 mg/mL protein was prepared with 10 mM pH 6.0 His-HCl or 5 mM pH 6.0 His-HCl as a buffer system.

The formulation was filtered and bottled, and the bottles were stoppered and capped. A sample of the formulation was lyophilized through a lyophilization program including pre-freezing, primary drying, and secondary drying (the parameters are shown in Table 35). After the end of the lyophilization program, stoppering was performed under vacuum. Then the lyophilized formulation was reconstituted with water for injection to form an anti-IL-2 antibody reconstituted solution with a protein concentration of 10 mg/mL. Differences in appearance, SEC purity, etc. were examined between the formulation before the lyophilization and the reconstituted lyophilized formulation. The experimental results are shown in Table 36, and the results show that the anti-IL-2 antibody formulation maintained good stability both before and after the lyophilization.

**Table 35. The lyophilization program for the anti-IL-2 antibody formulation**

| Lyophilization process parameters | Set temperature (°C) | Set time (min) | Holding time (min) | Degree of vacuum (Pa) |
|---|---|---|---|---|
| Pre-freezing | 5 | 10 | 60 | N/A |
| Pre-freezing | -45 | 50 | 60 | N/A |
| Primary drying | -25 | 120 | 3000 | 10 |
| Secondary drying | 25 | 60 | 1 | 10 |
| | 25 | 1 | 480 | 10 |

**Table 36. A comparison of the experimental results for the anti-IL-2 antibody formulations before and after lyophilization**

| Group | Standing conditions | Appearance | SEC% |
|---|---|---|---|
| | | | Monomer |
| 1 (10mM pH6.0) | Formulation before lyophilization | Clear and transparent | 96.7 |
| | Reconstituted lyophilized formulation | Clear and transparent | 96.6 |
| 2 (5mM pH6.0) | Formulation before lyophilization | Clear and transparent | 96.4 |
| | Reconstituted lyophilized formulation | Clear and transparent | 96.5 |

## Claims

1. A pharmaceutical composition, comprising a fusion protein and a buffer, wherein the fusion protein comprises IL-2 and an anti-IL-2 antibody that are covalently bound; the buffer is a histidine buffer, an acetate buffer, or a succinate buffer;
preferably, the buffer is a histidine buffer or an acetate buffer;
more preferably, the buffer is a histidine-histidine hydrochloride buffer or an acetic acid-sodium acetate buffer.

2. The pharmaceutical composition according to claim 1, wherein the concentration of the fusion protein is 5 mg/mL to 50 mg/mL;
preferably, the concentration of the fusion protein is 10 mg/mL to 30 mg/mL.

3. The pharmaceutical composition according to claim 1 or 2, wherein the pharmaceutical composition comprises a surfactant;
preferably, the surfactant is polysorbate or poloxamer;
more preferably, the surfactant is polysorbate 80, polysorbate 20, or poloxamer 188.

4. The pharmaceutical composition according to claim 3, wherein the concentration of the surfactant is 0.05 mg/mL to 2 mg/mL;
preferably, the concentration of the surfactant is 0.1 mg/mL to 0.8 mg/mL.

5. The pharmaceutical composition according to any of claims 1 to 4, wherein the pharmaceutical composition comprises a sugar;
preferably, the sugar is one or more of sucrose, mannitol, and trehalose;
more preferably, the sugar is sucrose.

6. The pharmaceutical composition according to claim 5, wherein the concentration of the sugar is 20 mg/mL to 100 mg/mL;
preferably, the concentration of the sugar is 30 mg/mL to 80 mg/mL;
more preferably, the concentration of the sugar is 30 mg/mL to 50 mg/mL.

7. The pharmaceutical composition according to claim 6, wherein the pharmaceutical composition further comprises an auxiliary material; preferably, the auxiliary material is glycine, methionine, proline, histidine, phenylalanine, arginine hydrochloride, glutamic acid, aspartic acid, or disodium edetate; more preferably, the auxiliary material is glycine.

8. The pharmaceutical composition according to claim 7, wherein the concentration of the auxiliary material is 1 mg/mL to 30 mg/mL; preferably, the concentration of the auxiliary material is 5 mg/mL to 15 mg/mL.

9. The pharmaceutical composition according to any of claims 1 to 8, wherein the concentration of the buffer is 5 mM to 100 mM;
preferably, the concentration of the buffer is 5 mM to 30 mM;
more preferably, the concentration of the buffer is 5 mM to 10 mM.

10. The pharmaceutical composition according to any of claims 1 to 9, wherein the buffer has a pH of 5.0 to 6.5;
preferably, the buffer has a pH of 5.5 to 6.0;
more preferably, the buffer has a pH of 6.0.

11. The pharmaceutical composition according to any of claims 1 to 10, wherein the anti-IL-2 antibody comprises a heavy chain variable region and a light chain variable region; the heavy chain variable region has: a HCDR1 comprising the amino acid sequence of SEQ ID NO: 37, a HCDR2 comprising the amino acid sequence of SEQ ID NO: 38, and a HCDR3 comprising the amino acid sequence of SEQ ID NO: 39; the light chain variable region has: a LCDR1 comprising the amino acid sequence of SEQ ID NO: 40, a LCDR2 comprising the amino acid sequence of SEQ ID NO: 41, and a LCDR3 comprising the amino acid sequence of SEQ ID NO: 42;
preferably, the heavy chain variable region comprises the amino acid sequence of SEQ ID NO: 9, and the light chain variable region comprises the amino acid sequence of SEQ ID NO: 10;
more preferably, the anti-IL-2 antibody comprises a heavy chain of the amino acid sequence of SEQ ID NO: 165 and a light chain of the amino acid sequence of SEQ ID NO: 166.

12. The pharmaceutical composition according to any of claims 1 to 11, wherein the IL-2 is fused, directly or by a linker, to the N-terminus of the light chain of the anti-IL-2 antibody;
preferably, the amino acid residue at position 3 of the IL-2 is E;
more preferably, the fusion protein comprises a heavy chain of the amino acid sequence of SEQ ID NO: 165 and a light chain of the amino acid sequence of SEQ ID NO: 189.

13. The pharmaceutical composition according to any of claims 1 to 12, comprising the following components:
a) 10 mg/mL to 30 mg/mL said fusion protein,
b) 0.1 mg/mL to 0.8 mg/mL polysorbate or poloxamer,
c) 30 mg/mL to 80 mg/mL sucrose,
d) 1 mg/mL to 30 mg/mL glycine, and
e) 5 mM to 10 mM histidine-histidine hydrochloride buffer, the histidine-histidine hydrochloride buffer having a pH of 6.0;
preferably, the pharmaceutical composition comprises the following components:
a) 10 mg/mL to 30 mg/mL said fusion protein,
b) 0.1 mg/mL to 0.8 mg/mL polysorbate 80,
c) 30 mg/mL to 50 mg/mL sucrose,
d) 5 mg/mL to 15 mg/mL glycine, and
e) 5 mM to 10 mM histidine-histidine hydrochloride buffer, the histidine-histidine hydrochloride buffer having a pH of 6.0.

14. The pharmaceutical composition according to any of claims 1 to 13, wherein the composition is a liquid formulation;
preferably, the route of administration of the composition is subcutaneous, intradermal, intravenous, or intramuscular injection;
more preferably, the route of administration of the composition is subcutaneous injection.

15. A lyophilized formulation, wherein the lyophilized formulation can be reconstituted to form the pharmaceutical composition according to any of claims 1 to 14.

16. A method for preparing a lyophilized formulation, comprising the step of lyophilizing the pharmaceutical composition according to any of claims 1 to 14.

17. A lyophilized formulation, wherein the formulation is obtained by lyophilizing the pharmaceutical composition according to any of claims 1 to 14.

18. A reconstituted solution, wherein the reconstituted solution is prepared by reconstituting the lyophilized formulation according to claim 15 or 17.

19. A product, comprising a container, wherein the container contains the pharmaceutical composition according to any of claims 1 to 14, the lyophilized formulation according to claim 15 or 17, or the reconstituted solution according to claim 18.

20. A method for treating an autoimmune disease or delaying the progression of an autoimmune disease, wherein the method comprises administering to a subject in need thereof a therapeutically effective amount of the pharmaceutical composition according to any of claims 1 to 14, the lyophilized formulation according to claim 15 or 17, the reconstituted solution according to claim 18, or the product according to claim 19; preferably, the autoimmune disease is selected from any one or a combination of the following: lupus, graft versus host disease, hepatitis C virus-induced vasculitis, type I diabetes, multiple sclerosis, inflammatory bowel disease, chronic GvHD, asthma, dermatitis, alopecia areata, acute lung injury, sepsis, reactive arthritis, and rheumatoid arthritis.
